# EUROPEAN PATENT SPECIFICATION

(11) **EP 3 802 549 B1**
(45) Date of publication and mention of the grant of the patent: **01.03.2023**
(21) Application number: 19731155.8
(22) Date of filing: 27.05.2019
(51) Int. Cl.: C07D 519/00, A61P 31/04, A61K 31/437, A61K 31/4375

(54) **NOVEL OXOQUINOLIZINE COMPOUNDS FOR THE TREATMENT AND PROPHYLAXIS OF BACTERIAL INFECTION**
NEUE OXOCHINOLIZINVERBINDUNGEN ZUR BEHANDLUNG UND PROPHYLAXE EINER BAKTERIELLEN INFEKTION
NOUVEAUX COMPOSÉS D'OXOQUINOLIZINE POUR LE TRAITEMENT ET LA PROPHYLAXIE D'UNE INFECTION BACTÉRIENNE

(30) Priority: 28.05.2018 WO PCT/CN2018/088728
(43) Date of publication of application: 14.04.2021
(73) Proprietor: F. Hoffmann-La Roche AG, 4070 Basel (CH)
(72) Inventor: DEY, Fabian, 4070 Basel (CH); DING, Xiao, Shanghai, 201203 (CN); HU, Yimin, Shanghai, 201203 (CN); LIU, Yongqiang, Shanghai, 201203 (CN); SHEN, Hong, Shanghai, 201203 (CN); SHI, Houguang, Shanghai, 201203 (CN); TAN, Xuefei, Shanghai, 201203 (CN); ZHOU, Chengang, Shanghai, 201203 (CN); ZHOU, Mingwei, Shanghai, 201203 (CN)
(74) Representative: Jochnowitz, Evan
(86) International application number: PCT/EP2019/063562
(87) International publication number: WO 2019/228940

(56) References cited:
- WO-A1-2012/125746
- WO-A1-2018/178041
- JP-A- 2003 012 670

## Description

The present invention relates to organic compounds useful for therapy and/or prophylaxis in a mammal, and in particular to inhibitors of DNA gyrase and/or topoisomerase IV useful for treatment and/or prophylaxis of bacterial infection.

### FIELD OF THE INVENTION

Bacterial infections pose a continuing medical problem because anti-bacterial drugs eventually engender resistance in the bacteria on which they are used. Bacterial resistance against virtually all current antibiotic drugs are increasing. Many forms of antibiotic resistance can even cross international boundaries and spread with remarkable speed. Thus novel classes of antibacterial compounds are urgently needed.

One target for development of anti-bacterial drugs has been DNA gyrase and topoisomerase IV (bacterial type IIA topoisomerases), which are essential to cell life, that solve DNA topological problems resulting from the replication, transcription, and recombination of DNA. DNA Gyrase controls DNA supercoiling and relieves topological stress that occurs when the DNA strands are untwisted such as during replication. Topoisomerase IV primarily resolves linked chromosome dimers at the conclusion of DNA replication. Both enzymes can introduce double stranded DNA breaks; pass a second DNA strand through the break and rejoining the broken strands. The activity of both enzymes is driven by the binding and hydrolysis of ATP. Bacterial DNA gyrase consists of two A (GyrA) and two B (GyrB) subunits. Binding and cleavage of the DNA is associated with GyrA, whereas ATP is bound and hydrolyzed by GyrB. Bacterial Topoisomerase IV is also a hetero-tetramer that consists of two C (ParC) and two E (ParE) subunits. The latter subunits bind ATP like GyrB in order to supply energy necessary for catalytic turnover of the enzymes.

Inhibition of DNA gyrase and topoisomerase IV has potential for the development of broad-spectrum antibiotics. The enzymes are highly conserved across a broad range of gram-positive and gram-negative pathogens. There are two classes of antibiotics that demonstrated such mechanism of action. The first, well-represented by the quinolones, inhibits GyrA and ParC subunits by stabilizing the cleaved DNA-enzyme complex, thus inhibiting overall gyrase function, leading to cell death. Novobiocin, the only marketed drug in the second class, exerts its effect by blocking the ATPase activity of the enzymes. Novobiocin was identified in 1950s. But its use declined rapidly and it was eventually withdrawn from the market, mainly due to its low permeability in many bacteria strains, rise of spontaneous resistance development, and the development of more effective drugs, such as penicillinase-stable penicillins and the first cephalosporins in 1960s and 1970s.

Recently, strong inhibition of DNA gyrase and/or topoisomerase IV has been recognized to be important for low resistance development in bacterial strains treated by inhibitors of the enzymes. Inhibitors of bacterial DNA gyrase and/or topoisomerase IV with different mechanism of action compare to the widely used quinolones will exhibit minimal cross resistance, and will be potentially useful in combating quinolone resistance that has increased significantly in the past few years JP2003012670 discloses substituted quinolizine derivatives as anti-bacterials.

### SUMMARY OF THE INVENTION

The present invention relates to novel compounds of formula (I), 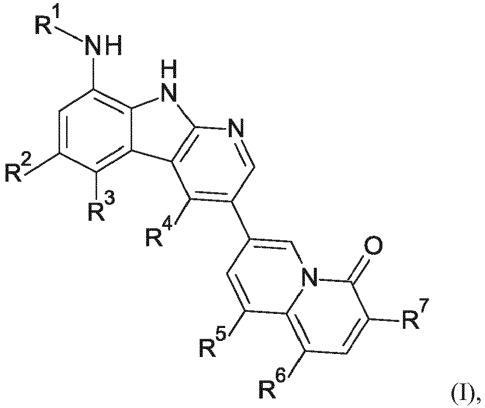 wherein
R¹ is C₁₋₆alkyl;
R² is halogen;
R³ is H, halogen or cyano;
R⁴ is (C₁₋₆alkyl)₂amino or heterocyclyl;
R⁵ is H, halogen or C₁₋₆alkyl;
R⁶ is H, ((C₁₋₆alkyl)₂amino)C₁₋₆alkyl, C₁₋₆alkyl, C₃₋₇cycloalkyl, haloC₁₋₆alkyl, halogen, morpholinylC₁₋₆alkyl or pyrrolidinylC₁₋₆alkyl;
R⁷ is carboxy;
or a pharmaceutically acceptable salt, or enantiomer or diastereomer thereof.

Further objects of the present invention are novel compounds of formula (I), their manufacture, medicaments based on a compound in accordance with the invention and their production as well as the use of compounds of formula (I) for the treatment or prophylaxis of bacterial infection. The use of compounds of formula (I) as DNA gyrase and/or topoisomerase IV inhibitors is also one of the objections of present invention. The compounds of formula (I) showed superior anti-bacterial activity, good solubility, good CC₅₀ profiles, improved microsomal stability and/or improved PK profile.

### DETAILED DESCRIPTION OF THE INVENTION

Unless otherwise defined, all technical and scientific terms used herein have the same meaning as commonly understood by one of ordinary skill in the art to which this invention belongs. Furthermore, the following definitions are set forth to illustrate and define the meaning and scope of the various terms used to describe the invention. Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

### DEFINITIONS

The term "C₁₋₆alkyl" denotes a saturated, linear or branched chain alkyl group containing 1 to 6, particularly 1 to 4 carbon atoms, for example methyl, ethyl, propyl, isopropyl, n-butyl, isobutyl, tert-butyl and the like. Particular "C₁₋₆alkyl" groups are methyl, ethyl and propyl.

The term "halogen" and "halo" are used interchangeably herein and denote fluoro, chloro, bromo, or iodo.

The term "haloC₁₋₆alkyl" denotes an alkyl group wherein at least one of the hydrogen atoms of the alkyl group has been replaced by same or different halogen atoms, particularly fluoro atom. Examples of haloC₁₋₆alkyl include monofluoro-, difluoro-or trifluoro-methyl, -ethyl or -propyl, for example 3,3,3-trifluoropropyl, 2-fluoroethyl, 2,2,2-trifluoroethyl, fluoromethyl, difluoromethyl, trifluoromethyl and trifluoroethyl.

The term "C₃₋₇cycloalkyl" denotes a saturated monocyclic or bicyclic carbon ring containing from 3 to 7 carbon atoms, particularly from 3 to 6 carbon atoms, for example, cyclopropyl, cyclobutyl, cyclopentyl, cyclohexyl, cycloheptyl, bicyclo[1.1.1]pentanyl and the like. Particular "C₃₋₇cycloalkyl" group is cyclopropyl.

The term "heterocyclyl" denotes a monovalent saturated or partly unsaturated mono-, bicyclic or tricyclic ring system of 3 to 12 ring atoms, comprising 1, 2, or 3 ring heteroatoms selected from N, O and S, the remaining ring atoms being carbon. In particular embodiments, heterocyclyl is a monovalent saturated or partly unsaturated monocyclic or bicyclic ring system of 4 to 10 ring atoms, comprising 1, 2, or 3 ring heteroatoms selected from N, O and S, the remaining ring atoms being carbon. Examples for monocyclic saturated heterocyclyl are aziridinyl, oxiranyl, azetidinyl, oxetanyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Bicyclic heterocyclyl can be spiro ring, fused ring or bridged ring. Examples for bicyclic heterocyclyl are decahydronaphthyridine; azabicyclo[3.2.0]heptanyl; octahydrocyclopenta[b]pyrrolyl; octahydrocyclopenta[c]pyrrolyl; 1,3,3a,4,6,6a-hexahydrofuro[3,4-c]pyrrolyl; 1,3,3a,4,6,6a-hexahydropyrrolo[3,4-c]pyrrolyl; 1,7-diazaspiro[3.4]octanyl; 1,7-diazaspiro[4.4]nonanyl; 2,3,3a,4,6,6a-hexahydrofuro[2,3-c]pyrrolyl; 2,3,3a,4,6,6a-hexahydropyrrolo[2,3-c]pyrrolyl; 2,3,3a,5,6,6a-hexahydropyrrolo[3,2-b]pyrrolyl; 2,3,3a,4,6,6a-hexahydropyrrolo[3,4-b]pyrrolyl; 2,3,3a,5,6,6a-hexahydropyrrolo[3,2-b]pyrrolyl; 2,3,4a,5,7,7a-hexahydropyrrolo[3,4-b][1,4]oxazinyl; 2,6-diazabicyclo[3.2.0]heptanyl; 2,6-diazaspiro[3.4]octanyl; 2,7-diazaspiro[4.4]nonanyl; 2,8-diazaspiro[4.5]decanyl; 2,9-diazaspiro[4.5]decanyl; 3,3a,4,5,6,6a-hexahydro-1H-cyclopenta[c]pyrrolyl; 3,3a,4,5,7,7a-hexahydro-2H-pyrrolo[2,3-c]pyridinyl; 3,3a,4,6,7,7a-hexahydro-2H-pyrrolo[3,2-c]pyridinyl; 3,3a,5,6,7,7a-hexahydro-2H-pyrrolo[3,2-b]pyridinyl; 3,4,4a,5,7,7a-hexahydro-2H-pyrrolo[3,4-b]pyridinyl; 3,4,6,6a-tetrahydro-2H-pyrrolo[2,3-c]pyrrolyl; 3,4,6,6a-tetrahydro-2H-pyrrolo[3,4-b]pyrrolyl; 3,4,6,7,8,8a-hexahydro-1H-pyrrolo[1,2-a]pyrazinyl; 3,6-diazabicyclo[3.2.0]heptanyl; 3,8-diazabicyclo[4.2.0]octanyl; 3,7-diazabicyclo[4.2.0]octanyl; 2,7-diazabicyclo[4.2.0]octanyl; 3-azabicyclo[3.1.0]hexanyl; 5-azaspiro[2.4]heptanyl; 5-oxa-2,8-diazaspiro[3.5]nonanyl; 6-azaspiro[3.4]octanyl; 6-oxa-2,9-diazaspiro[4.5]decanyl; hexahydro-1H-isoindol-2(3H)-yl; hexahydro-1H-pyrrolo[3,4-b]pyridin-6(2H)-yl; hexahydropyrazino[2,1-c][1,4]oxazin-8(1H)-yl; hexahydropyrrolo[3,4-b][1,4]oxazin-6(2H)-yl; hexahydropyrrolo[3,4-b]pyrrol-1(2H)-yl; oxaazabicyclo[2.2.1]heptanyl; and oxaazabicyclo[3.1.1]heptanyl.. Examples for partly unsaturated heterocyclyl are dihydrofuryl, imidazolinyl, dihydro-oxazolyl, tetrahydro-pyridinyl, or dihydropyranyl. Monocyclic or bicyclic heterocyclyl can be further substituted once, twice or three times by hydroxyC₁₋₆alkyl, (C₁₋₆alkoxyC₁₋₆alkyl(C₁₋₆alkyl)amino)C₁₋₆alkyl, (C₁₋₆alkyl)₂amino, (C₁₋₆alkyl)₂aminoC₁₋₆alkyl, (C₃₋₇cycloalkyl(C₁₋₆alkyl)amino)C₁₋₆alkyl, (C₃₋₇cycloalkylamino)C₁₋₆alkyl, amino, aminoC₁₋₆alkyl, C₁₋₆alkoxy, C₁₋₆alkoxyC₁₋₆alkyl, C₁₋₆alkoxyC₁₋₆alkyl(C₁₋₆alkyl)amino, C₁₋₆alkyl, (C₁₋₆alkyl)₂amino, C₃₋₇cycloalkyl, C₃₋₇cycloalkyl(C₁₋₆alkyl)amino, C₃₋₇cycloalkylamino, haloC₁₋₆alkyl, halogen, hydroxy, hydroxyC₁₋₆alkyl, hydroxyC₁₋₆alkyl(C₁₋₆alkyl)amino, morpholinoC₁₋₆alkyl, pyrrolidinyl or pyrrolidinylC₁₋₆alkyl.

The term "cis-isomers" and "trans-isomers" denote the relative stereochemistry of the molecule or moiety. For example: Intermediate C2 ( 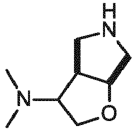 ) as the "cis-isomers" refers to a mixture of 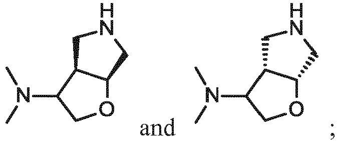 similarly, Intermediate in Example 5.01A ( 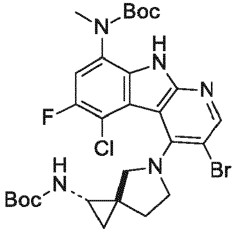 ) as the "*trans*-isomers" refers to a mixture of 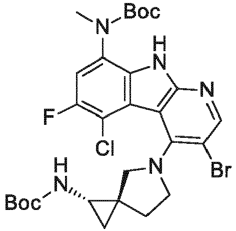 and 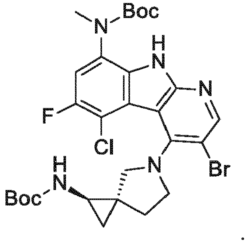 The way of showing relative stereochemistry also applies to the final compounds.

The term "enantiomer" denotes two stereoisomers of a compound which are non-superimposable mirror images of one another.

The term "diastereomer" denotes a stereoisomer with two or more centers of chirality and whose molecules are not mirror images of one another. Diastereomers have different physical properties, e.g. melting points, boiling points, spectral properties, and reactivities.

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, *p*-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and *tert*iary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, *N*-ethylpiperidine, and polyamine resins.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, e.g., a human in need thereof.

### INHIBITORS OF DNA GYRASE AND/OR TOPOISOMERASE IV

The present invention relates to a compound of formula (I), 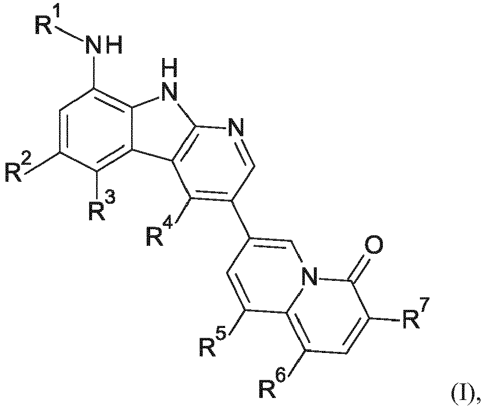 wherein
R¹ is C₁₋₆alkyl;
R² is halogen;
R³ is H, halogen or cyano;
R⁴ is (C₁₋₆alkyl)₂amino or heterocyclyl;
R⁵ is H, halogen or C₁₋₆alkyl;
R⁶ is H, ((C₁₋₆alkyl)₂amino)C₁₋₆alkyl, C₁₋₆alkyl, C₃₋₇cycloalkyl, haloC₁₋₆alkyl, halogen, morpholinylC₁₋₆alkyl or pyrrolidinylC₁₋₆alkyl;
R⁷ is carboxy;
or a pharmaceutically acceptable salt, or enantiomer or diastereomer thereof.

A further embodiment of present invention is (ii) a compound of formula (I) according to (i), wherein
R¹ is C₁₋₆alkyl;
R² is halogen;
R³ is H, halogen or cyano;
R⁴ is (C₁₋₆alkyl)₂amino;
   2,3,3a,4,6,6a-hexahydrofuro[2,3-c]pyrrolyl substituted by (C₁₋₆alkyl)₂amino;
   2,3,3a,4,6,6a-hexahydropyrrolo[2,3-c]pyrrolyl substituted by C₁₋₆alkyl;
   2,3,3a,4,6,6a-hexahydropyrrolo[3,4-b]pyrrolyl substituted by C₁₋₆alkyl;
   2,3,4a,5,7,7a-hexahydropyrrolo[3,4-b][1,4]oxazinyl substituted by C₁₋₆alkyl;
   3,3a,4,5,6,6a-hexahydro-1H-cyclopenta[c]pyrrolyl substituted by (C₁₋₆alkyl)₂amino;
   3,3a,4,5,7,7a-hexahydro-2H-pyrrolo[2,3-c]pyridinyl substituted by C₁₋₆alkyl;
   3,4,6,6a-tetrahydro-2H-pyrrolo[2,3-c]pyrrolyl substituted twice by C₁₋₆alkyl;
   3-azabicyclo[3.1.0]hexanyl substituted by (C₁₋₆alkyl)₂amino;
   5-azaspiro[2.4]heptanyl substituted by amino;
   aminoC₃₋₇cycloalkylpyrrolidinyl;
   morpholinyl, said morpholinyl being unsubstituted or substituted by ((C₁₋₆alkyl)₂amino)C₁₋₆alkyl;
R⁵ is H, halogen or C₁₋₆alkyl;
R⁶ is H, ((C₁₋₆alkyl)₂amino)C₁₋₆alkyl, C₁₋₆alkyl, C₃₋₇cycloalkyl, haloC₁₋₆alkyl, halogen, morpholinylC₁₋₆alkyl or pyrrolidinylC₁₋₆alkyl;
R⁷ is carboxy;
or a pharmaceutically acceptable salt, or enantiomer or diastereomer thereof.

A further embodiment of present invention is (iii) a compound of formula (I) according to (ii), wherein
R¹ is methyl;
R² is chloro or fluoro;
R³ is H, chloro, fluoro or cyano;
R⁴ is dimethylamino; dimethylamino-2,3,3a,4,6,6a-hexahydrofuro[2,3-c]pyrrolyl; methyl-2,3,3a,4,6,6a-hexahydropyrrolo[2,3-c]pyrrolyl; methyl-2,3,3a,4,6,6a-hexahydropyrrolo[3,4-b]pyrrolyl; methyl-2,3,4a,5,7,7a-hexahydropyrrolo[3,4-b][1,4]oxazinyl; dimethylamino-3,3a,4,5,6,6a-hexahydro-1H-cyclopenta[c]pyrrolyl; methyl-3,3a,4,5,7,7a-hexahydro-2H-pyrrolo[2,3-c]pyridinyl; dimethyl-3,4,6,6a-tetrahydro-2H-pyrrolo[2,3-c]pyrrolyl; dimethylamino-3-azabicyclo[3.1.0]hexanyl; amino-5-azaspiro[2.4]heptanyl; aminocyclopropylpyrrolidinyl; morpholinyl or ((dimethylamino)methyl)morpholinyl;
R⁵ is H, fluoro or methyl;
R⁶ is H, (dimethylamino)methyl, methyl, cyclopropyl, trifluoromethyl, chloro, morpholinylmethyl or pyrrolidinylmethyl;
R⁷ is carboxy;
or a pharmaceutically acceptable salt, or enantiomer or diastereomer thereof.

A further embodiment of present invention is (iv) a compound of formula (I) according to (ii), wherein R⁴ is 2,3,3a,4,6,6a-hexahydropyrrolo[2,3-c]pyrrolyl substituted by C₁₋₆alkyl; 2,3,3a,4,6,6a-hexahydropyrrolo[3,4-b]pyrrolyl substituted by C₁₋₆alkyl; or 3,3a,4,5,6,6a-hexahydro-1H-cyclopenta[c]pyrrolyl substituted by (C₁₋₆alkyl)₂amino.

A further embodiment of present invention is (v) a compound of formula (I) according to (iv),wherein R⁴ is methyl-2,3,3a,4,6,6a-hexahydropyrrolo[2,3-c]pyrrolyl; methyl-2,3,3a,4,6,6a-hexahydropyrrolo[3,4-b]pyrrolyl; or dimethylamino-3,3a,4,5,6,6a-hexahydro-1H-cyclopenta[c]pyrrolyl.

A further embodiment of present invention is (vi) a compound of formula (I) according to (iv), wherein R⁵ is H.

A further embodiment of present invention is (vii) a compound of formula (I) according to (vi),wherein R⁶ is H.

A further embodiment of present invention is (viii) a compound of formula (I) according to (ii), wherein
R¹ is C₁₋₆alkyl;
R² is halogen;
R³ is H, halogen or cyano;
R⁴ is 2,3,3a,4,6,6a-hexahydropyrrolo[2,3-c]pyrrolyl substituted by C₁₋₆alkyl;
   2,3,3a,4,6,6a-hexahydropyrrolo[3,4-b]pyrrolyl substituted by C₁₋₆alkyl; or
   3,3a,4,5,6,6a-hexahydro-1H-cyclopenta[c]pyrrolyl substituted by (C₁₋₆alkyl)₂amino;
R⁵ is H;
R⁶ is H;
R⁷ is carboxy;
or a pharmaceutically acceptable salt, or enantiomer or diastereomer thereof.

A further embodiment of present invention is (ix) a compound of formula (I) according to (viii), wherein
R¹ is methyl;
R² is chloro or fluoro;
R³ is H, chloro, fluoro or cyano;
R⁴ is methyl-2,3,3a,4,6,6a-hexahydropyrrolo[2,3-c]pyrrolyl;
   methyl-2,3,3a,4,6,6a-hexahydropyrrolo[3,4-b]pyrrolyl; or
   dimethylamino-3,3a,4,5,6,6a-hexahydro-1H-cyclopenta[c]pyrrolyl;
R⁵ is H;
R⁶ is H;
R⁷ is carboxy;
or a pharmaceutically acceptable salt, or enantiomer or diastereomer thereof.

Another embodiment of present invention is that compounds of formula (I) are selected from:
7-(4-(dimethylamino)-5,6-difluoro-8-(methylamino)-9H-pyrido[2,3-b]indol-3-yl)-4-oxo-4H-quinolizine-3-carboxylic acid;
7-(6-fluoro-8-(methylamino)-4-morpholino-9H-pyrido[2,3-b]indol-3-yl)-4-oxo-4H-quinolizine-3-carboxylic acid;
7-[5,6-difluoro-4-(*cis*-5-methyl-2,3,3a,4,6,6a-hexahydropyrrolo[2,3-c]pyrrol-1-yl)-8-(methylamino)-9H-pyrido[2,3-b]indol-3-yl]-4-oxo-quinolizine-3-carboxylic acid;
7-[4-[(3a*S*,6a*S*)-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-4-oxo- quinolizine-3-carboxylic acid;
7-[4-[*cis*-(4*R*)-4-(dimethylamino)-3,3a,4,5,6,6a-hexahydro-1H-cyclopenta[c]pyrrol-2-yl]-5,6-difluoro-8-(methylamino)-9H-pyrido[2,3-b]indol-3-yl]-4-oxo-quinolizine-3-carboxylic acid;
7-[4-[*cis*-3-(dimethylamino)-2,3,3a,4,6,6a-hexahydrofuro[2,3-c]pyrrol-5-yl]-5,6-difluoro-8-(methylamino)-9H-pyrido[2,3-b]indol-3-yl]-4-oxo-quinolizine-3-carboxylic acid;
7-(5-chloro-6-fluoro-8-(methylamino)-4-((3a*S*,6a*S*)-5-methyl-2,3,3a,4,6,6a-hexahydropyrrolo[2,3-c]pyrrol-1-yl)-9H-pyrido[2,3-b]indol-3-yl)-4-oxo-4H-quinolizine-3-carboxylic acid;
7-[4-[*cis*-(4*R*)-4-(dimethylamino)-3,3a,4,5,6,6a-hexahydro-1H-cyclopenta[c]pyrrol-2-yl]-5-chloro-6-fluoro-8-(methylamino)-9H-pyrido[2,3-b]indol-3-yl]-4-oxo-quinolizine-3-carboxylic acid;
7-[4-[*cis*-(4*S*)-4-(dimethylamino)-3,3a,4,5,6,6a-hexahydro-1H-cyclopenta[c]pyrrol-2-yl]-5,6-difluoro-8-(methylamino)-9H-pyrido[2,3-b]indol-3-yl]-4-oxo-quinolizine-3-carboxylic acid;
7-[4-(3a,5-dimethyl-3,4,6,6a-tetrahydro-2H-pyrrolo[2,3-c]pyrrol-1-yl)-6-fluoro-8-(methylamino)-9H-pyrido[2,3-b]indol-3-yl]-4-oxo-quinolizine-3-carboxylic acid;
7-[5-cyano-6-fluoro-4-(*cis*-1-methyl-2,3,3a,4,6,6a-hexahydropyrrolo[3,4-b]pyrrol-5-yl)-8-(methylamino)-9H-pyrido[2,3-b]indol-3-yl]-4-oxo-quinolizine-3-carboxylic acid;
7-(5-cyano-4-(*cis*-(4*R*)-4 (dimethylamino)-3,3a,4,5,6,6a-hexahydro-1H-cyclopenta[c]pyrrol-2-yl)-6-fluoro-8-(methylamino)-9H-pyrido[2,3-b]indol-3-yl)-4-oxo-4H-quinolizine-3-carboxylic acid;
7-(4-((1*R*,5*S*,6*S*)-6-(dimethylamino)-3-azabicyclo[3.1.0]hexan-3-yl)-5,6-difluoro-8-(methylamino)-9H-pyrido[2,3-b]indol-3-yl)-4-oxo-4H-quinolizine-3-carboxylic acid;
7-[4-[(3a*S*,6a*S*)-5-methyl-2,3,3a,4,6,6a-hexahydropyrrolo[2,3-c]pyrrol-1-yl]-5-cyano-6-fluoro-8-(methylamino)-9H-pyrido[2,3-b]indol-3-yl]-4-oxo-quinolizine-3-carboxylic acid;
7-[5-cyano-4-[cis-3-(dimethylamino)-2,3,3a,4,6,6a-hexahydrofuro[2,3-c]pyrrol-5-yl]-6-fluoro-8-(methylamino)-9H-pyrido[2,3-b]indol-3-yl]-4-oxo-quinolizine-3-carboxylic acid;
7-[4-[*cis*-1-methyl-2,3,3a,4,6,6a-hexahydropyrrolo[3,4-b]pyrrol-5-yl]-5-cyano-6-fluoro-8-(methylamino)-9H-pyrido[2,3-b]indol-3-yl]-4-oxo-quinolizine-3-carboxylic acid;
7-[4-[*cis*-1-methyl-3,3a,4,5,7,7a-hexahydro-2H-pyrrolo[2,3-c]pyridin-6-yl]-5-cyano-6-fluoro-8-(methylamino)-9H-pyrido[2,3-b]indol-3-yl]-4-oxo-quinolizine-3-carboxylic acid;
7-[5-cyano-4-(dimethylamino)-6-fluoro-8-(methylamino)-9H-pyrido[2,3-b]indol-3-yl]-4-oxo-quinolizine-3-carboxylic acid;
7-[6-chloro-4-(*cis*-5-methyl-2,3,3a,4,6,6a-hexahydropyrrolo[2,3-c]pyrrol-1-yl)-8-(methylamino)-9H-pyrido[2,3-b]indol-3-yl]-4-oxo-quinolizine-3-carboxylic acid;
7-[4-(dimethylamino)-5,6-difluoro-8-(methylamino)-9H-pyrido[2,3-b]indol-3-yl]-9-methyl-4-oxo-quinolizine-3-carboxylic acid;
7-[4-[3-(1-aminocyclopropyl)pyrrolidin-1-yl]-5,6-difluoro-8-(methylamino)-9H-pyrido[2,3-b]indol-3-yl]-4-oxo-quinolizine-3-carboxylic acid;
7-[4-[(4a*S*,7a*R*)-4-methyl-2,3,4a,5,7,7a-hexahydropyrrolo[3,4-b][1,4]oxazin-6-yl]-5,6-difluoro-8-(methylamino)-9H-pyrido[2,3-b]indol-3-yl]-4-oxo-quinolizine-3-carboxylic acid;
7-[4-[(3aS,6aS)-5-methyl-2,3,3a,4,6,6a-hexahydropyrrolo[2,3-c]pyrrol-1-yl]-6-cyano-5-fluoro-8-(methylamino)-9H-pyrido[2,3-b]indol-3-yl]-4-oxo-quinolizine-3-carboxylic acid;
7-(5,6-difluoro-8-(methylamino)-4-(*cis*-5-methyl-2,3,3a,4,6,6a-hexahydropyrrolo[2,3-c]pyrrol-1-yl)-9H-pyrido[2,3-b]indol-3-yl)-9-methyl-4-oxo-4H-quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-9-fluoro-4-oxo-quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-1-[(dimethylamino)methyl]-4-oxo-quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-1-methyl-4-oxo-quinofizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido [2,3-b]indol-3-yl]-1-cyclopropyl-4-oxo-quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-4-oxo-1-(trifluoromethyl)quinolizine-3-carboxylic acid;
(*R*)-7-(4-(2-((dimethylamino)methyl)morpholino)-6-fluoro-8-(methylamino)-9H-pyrido[2,3-b]indol-3-yl)-4-oxo-4H-quinolizine-3-carboxylic acid;
(*S*)-7-(4-(2-((dimethylamino)methyl)morpholino)-6-fluoro-8-(methylamino)-9H-pyrido[2,3-b]indol-3-yl)-4-oxo-4H-quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-1-(morpholinomethyl)-4-oxo-quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-4-oxo-1-(pyrrolidin-1-ylmethyl)quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-1-chloro-4-oxo-quinolizine-3-carboxylic acid;
7-[4-[(3a*R*,6a*R*)-1-methyl-2,3,3a,4,6,6a-hexahydropyrrolo[3,4-b]pyrrol-5-yl]-6-fluoro-8-(methylamino)-9H-pyrido[2,3-b]indol-3-yl]-4-oxo-quinolizine-3-carboxylic acid;
7-[4-[(3a*S*,6a*S*)-1-methyl-2,3,3a,4,6,6a-hexahydropyrrolo[3,4-b]pyrrol-5-yl]-6-fluoro-8-(methylamino)-9H-pyrido[2,3-b]indol-3-yl]-4-oxo-quinolizine-3-carboxylic acid;
7-[4-[(3a*S*,6a*S*)-5-methyl-2,3,3a,4,6,6a-hexahydropyrrolo[2,3-c]pyrrol-1-yl]-6-fluoro-8-(methylamino)-9H-pyrido[2,3-b]indol-3-yl]-4-oxo-quinolizine-3-carboxylic acid;
7-[4-[(3a*R*,6a*R*)-5-methyl-2,3,3a,4,6,6a-hexahydropyrrolo[2,3-c]pyrrol-1-yl]-6-fluoro-8-(methylamino)-9H-pyrido[2,3-b]indol-3-yl]-4-oxo-quinolizine-3-carboxylic acid;
7-[4-[(3a*R*,6a*R*)-1-methyl-2,3,3a,4,6,6a-hexahydropyrrolo[3,4-b]pyrrol-5-yl]-5,6-difluoro-8-(methylamino)-9H-pyrido[2,3-b]indol-3-yl]-4-oxo-quinolizine-3-carboxylic acid;
7-[4-[(3a*S*,6a*S*)-1-methyl-2,3,3a,4,6,6a-hexahydropyrrolo[3,4-b]pyrrol-5-yl]-5,6-difluoro-8-(methylamino)-9H-pyrido[2,3-b]indol-3-yl]-4-oxo-quinolizine-3-carboxylic acid;
7-[4-[(3a*R*,7a*R*)-1-methyl-3,3a,4,5,7,7a-hexahydro-2H-pyrrolo[2,3-c]pyridin-6-yl]-5,6-difluoro-8-(methylamino)-9H-pyrido[2,3-b]indol-3-yl]-4-oxo-quinolizine-3-carboxylic acid;
7-[4-[(3a*S*,7a*S*)-1-methyl-3,3a,4,5,7,7a-hexahydro-2H-pyrrolo[2,3-c]pyridin-6-yl]-5,6-difluoro-8-(methylamino)-9H-pyrido[2,3-b]indol-3-yl]-4-oxo-quinolizine-3-carboxylic acid;
7-[4-[(1*S*,3*R*)-1-amino-5-azaspiro[2.4]heptan-5-yl]-5,6-difluoro-8-(methylamino)-9H-pyrido[2,3-b]indol-3-yl]-4-oxo-quinolizine-3-carboxylic acid;
7-(4-((1*R*,3*R*)-1-amino-5-azaspiro[2.4]heptan-5-yl)-5,6-difluoro-8-(methylamino)-9H-pyrido[2,3-b]indol-3-yl)-4-oxo-4H-quinolizine-3-carboxylic acid;
7-(4-((1*R*,3*S*)-1-amino-5-azaspiro[2.4]heptan-5-yl)-5,6-difluoro-8-(methylamino)-9H-pyrido[2,3-b]indol-3-yl)-4-oxo-4H-quinolizine-3-carboxylic acid;
7-(4-((1*S*,3*S*)-1-amino-5-azaspiro[2.4]heptan-5-yl)-5,6-difluoro-8-(methylamino)-9H-pyrido[2,3-b]indol-3-yl)-4-oxo-4H-quinolizine-3-carboxylic acid;
7-(4-((1*S*,3*R*)-1-amino-5-azaspiro[2.4]heptan-5-yl)-5-chloro-6-fluoro-8-(methylamino)-9H-pyrido[2,3-b]indol-3-yl)-4-oxo-4H-quinolizine-3-carboxylic acid;
7-(4-((1*R*,3*S*)-1-amino-5-azaspiro[2.4]heptan-5-yl)-5-chloro-6-fluoro-8-(methylamino)-9H-pyrido[2,3-b]indol-3-yl)-4-oxo-4H-quinolizine-3-carboxylic acid;
7-(4-((1*R*,3*R*)-1-amino-5-azaspiro[2.4]heptan-5-yl)-5-chloro-6-fluoro-8-(methylamino)-9H-pyrido[2,3-b]indol-3-yl)-4-oxo-4H-quinolizine-3-carboxylic acid; and
7-(4-((1*S*,3*S*)-1-amino-5-azaspiro[2.4]heptan-5-yl)-5-chloro-6-fluoro-8-(methylamino)-9H-pyrido[2,3-b]indol-3-yl)-4-oxo-4H-quinolizine-3-carboxylic acid;
or a pharmaceutically acceptable salt, or enantiomer or diastereomer thereof.

### SYNTHESIS

The compounds of the present invention can be prepared by any conventional means. Suitable processes for synthesizing these compounds as well as their starting materials are provided in the schemes below and in the examples. All substituents, in particular, R¹ to R⁷ are as defined above unless otherwise indicated. Furthermore, and unless explicitly otherwise stated, all reactions, reaction conditions, abbreviations and symbols have the meanings well known to a person of ordinary skill in organic chemistry. 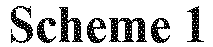 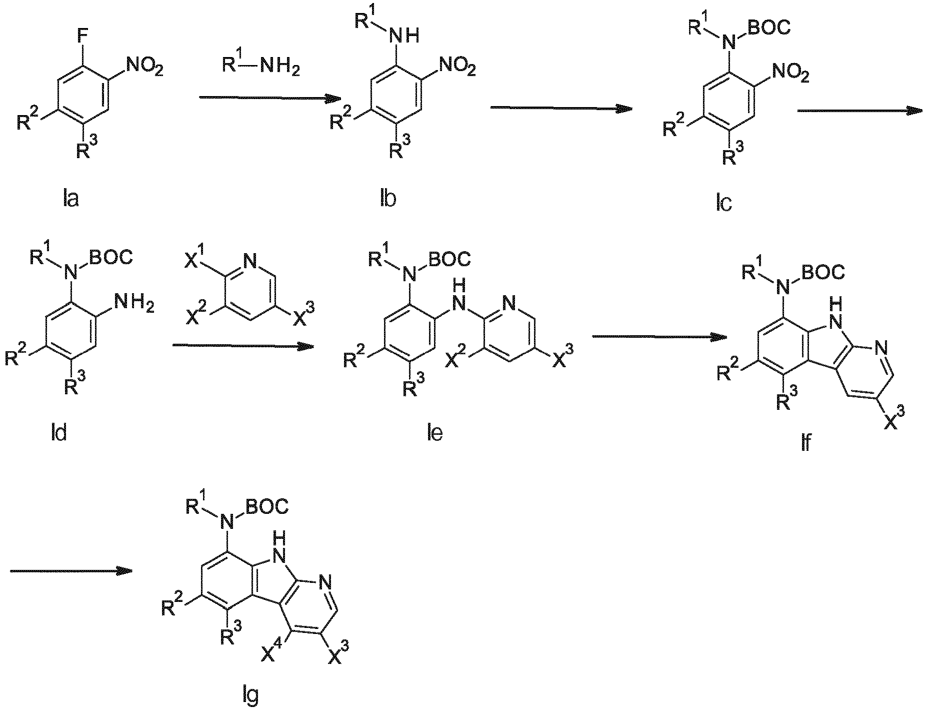 wherein X¹, X², X³ and X⁴ are halogen.

Compound of formula (Ig) can be prepared according to Scheme 1. Nucleophilic substitution of ortho-fluoro nitrobenzene (Ia) with amine R¹-NH₂ affords aniline (Ib). The aniline (Ib) can be protected with di-tert-butyl carbonate to give the protected aniline (Ic). The nitro group in aniline (Ic) can be reduced by a reducing agent, such as H₂ with palladium catalysts, to give the compound of formula (Id). Coupling of the compound of formula (Id) with tri-halogenated pyridine can be achieved using palladium catalysts and phosphine ligands to give compound of formula (Ie). Cyclization of compound of formula (Ie) using palladium catalysts and phosphine ligands gives compound of formula (If). Compound of formula (Ig) can be obtained through oxidation of the pyridine of compound of formula (If) followed by halogenation, such as treatment of POCl₃ or POBr₃. Compound of formula (Ig) can also be obtained by additional halogenation of compound of formula (Ig) (when R³ is H) with halogenating reagent, such as NCS, followed by di-*tert*-butyl carbonate re-protection. 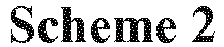 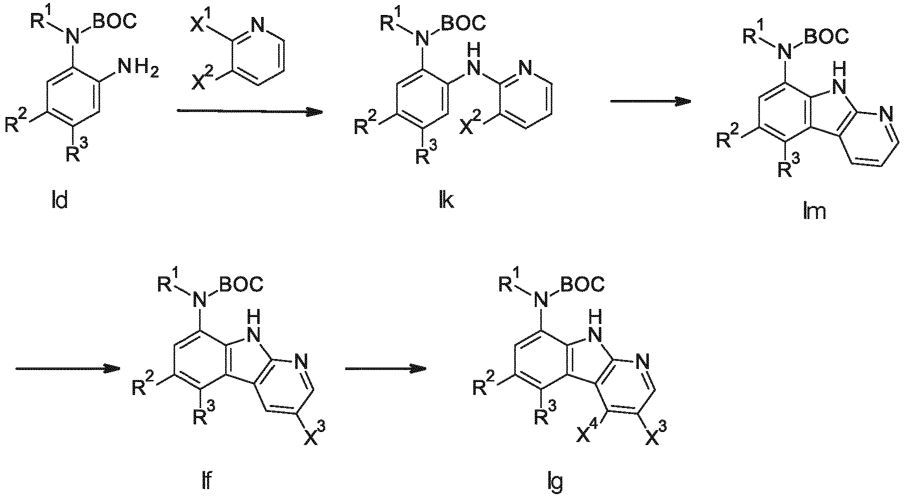 wherein X¹, X², X³ and X⁴ are halogen.

Alternatively, compound of formula (Ig) can be prepared according to Scheme 2. Coupling of the compound of formula (Id) with di-halogenated pyridine can be achieved using palladium catalyst and phosphine ligands to give compound of formula (Ik). Cyclization of compound of formula (Ik) using palladium catalyst and phosphine ligands gives compound of formula (Im). Compound of formula (Im) can be subject to halogenation using halogenating reagent, such as NCS, NBS or NIS, to give compound of formula (If), which subsequently undergoes oxidation of the pyridine followed by halogenation, such as treatment of POCl₃ or POBr₃. Alternatively, compound of formula (If) can be obtained by treatment of compound of formula (If) (when R³ is H) with halogenating reagent, such as NBS, to give compound of formula (If) (when R³ is halogen, in particular bromo). Compound of formula (If) can also be obtained by converting compound of formula (If) (when R³ is halogen, in particular bromo) to compound of formula (If) (when R³ is CN) via palladium mediated substitution or nucleophilic substitution. Compound of formula (Ig) can then be obtained through oxidation of the pyridine of compound of formula (If) followed by halogenation, such as treatment of POCl₃ or POBr₃. 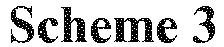 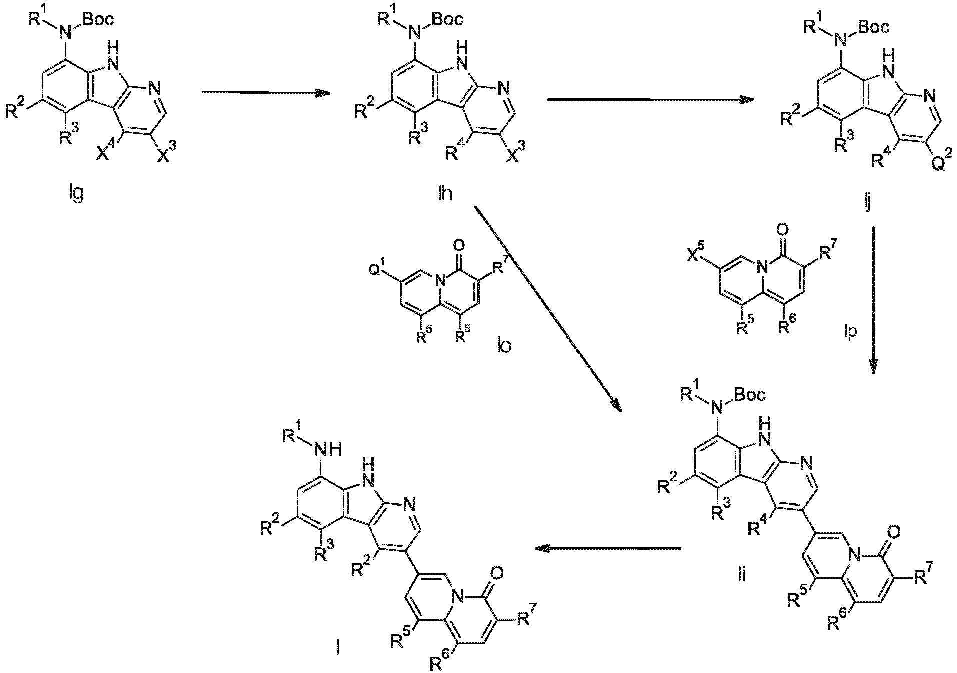 wherein X³ and X⁴ are halogen, X⁵ are halogen or OTf; Q¹ and Q² are boronic acids or esters.

Compound of formula (I) can be prepared according to Scheme 3. Introducing R⁴ to compound of formula (Ig) can be achieved either through nucleophilic substitution with amine and a base for certain C-N bond formation (with R⁴ bearing a nucleophilic N), or a Buchwald-Hartwig Cross Coupling Reaction for certain C-N bond formation (with R⁴ bearing a basic N), to give compound of formula (Ih). Further coupling of compound of formula (Ih) with compound of formula (Io) to give compound of formula (Ii) can be achieved using a palladium catalyzed Suzuki coupling. Chiral separation can be achieved on compound of formula (Ih) or compound of formula (Ii). Some special compound of formula (Ii) need to reverse the Suzuki coupling for C-C bond formation by converting compound of formula (Ih) to compound of formula (Ij) as a boronic ester or boronic acid then coupling with compound of formula (Ip). Ester hydrolysis such as NaOH in ethanol followed by deprotection of compound of formula (Ii) in the presence of an acid, such as trifluoroacetic acid, then affords compound of formula (I).

This invention also relates to a process for the preparation of a compound of formula (I) comprising the reaction of compound of formula (Ii), 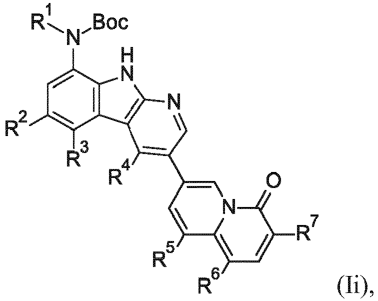
with an acid, which can be for example trifluoroacetic acid;
wherein R¹ to R⁷ are defined above. A compound of formula (I) when manufactured according to the above process is also an object of the invention.

### PHARMACEUTICAL COMPOSITIONS AND ADMINISTRATION

Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula (I) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compounds of formula (I) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to reduced bacterial load or improve host survival through the inhibition of bacterial DNA gyrase and/or Topoisomerase IV. For example, such amount may be below the amount that is toxic to normal cells, or the mammal as a whole.

In one example, the pharmaceutically effective amount of the compound of the invention administered parenterally per dose will be in the range of about 0.1 to 1000 mg/kg, alternatively about 1 to 100 mg/kg of patient body weight per day. In another embodiment, oral unit dosage forms, such as tablets and capsules, preferably contain from about 5 to about 5000 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 10 to 500 mg of the compound of the invention compounded with about 40 to 400mg anhydrous lactose, about 5 to 50 mg sodium croscarmellose, about 5 to 50 mg polyvinylpyrrolidone (PVP) K30, and about 1 to 10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 5 to 1000 mg) of the invention in a suitable buffer solution, e.g. a phosphate buffer, adding a tonicifier, e.g. a salt such sodium chloride, if desired. The solution may be filtered, e.g., using a 0.2 micron filter, to remove impurities and contaminants.

An embodiment, therefore, includes a pharmaceutical composition comprising a compound of formula (I), or a stereoisomer or pharmaceutically acceptable salt thereof. In a further embodiment includes a pharmaceutical composition comprising a compound of formula (I), or a stereoisomer or pharmaceutically acceptable salt thereof, together with a pharmaceutically acceptable carrier or excipient.

Another embodiment includes a pharmaceutical composition comprising a compound of formula (I) for use in the treatment and/or prophylaxis of bacterial infections.

In some embodiments, the compounds of this invention may be administered, as part of a single or multiple dosage regimen, orally, parenterally, by inhalation spray, topically, rectally, nasally, buccally, vaginally, or via an implanted reservoir. The term parenteral as used includes subcutaneous, intracutaneous, intravenous, intramuscular, intra-articular, intrasynovial, intrasternal, intrathecal, intralesional and intracranial injection or infusion techniques. Typically, the pharmaceutical compositions of the invention will be administered from about 1 to 5 times per day or alternatively, as a continuous infusion upon improvement of a patient's condition.

### INDICATIONS AND METHODS OF TREATMENT

The compounds of the invention are useful for treatment and/or prophylaxis of bacterial infection in humans or other animals by administering to the subject in need of a therapeutically effective amount of compound of formula (I), or a pharmaceutically acceptable salt, or enantiomer or diastereomer thereof. The compounds and methods of the invention are particularly well suited for human patients infected by pathogens that include *Staphylococcus aureus, Escherichia coli, Klebsiella pneumoniae, Acinetobacter baumannii* and *Pseudomonas aeruginosa.* Examples of bacterial organisms that may also be controlled by the compounds of the invention include, but not limited to, the following Gram-Positive and Gram-Negative organisms: *Streptococcus pneumoniae, Streptococcus pyogenes, Enterococcus faecalis, Enterococcus faecium, Enterobacter spp.* species, *Proteus spp.* species, *Serratia marcescens, Staphylococcus aureus, Coag. Neg. Staphylococci, Haemophilus influenzae, Bacillus anthraces, Mycoplasma pneumoniae, Moraxella catarrhalis, Chlamydophila pneumoniae, Chlamydia trachomatis, Legionella pneumophila, Mycobacterium tuberculosis, Helicobacter pylori, Staphylococcus saprophyticus, Staphylococcus epidermidis, Francisella tularensis, Yersinia pestis, Clostridium difficile, Bacteroides spp.* species *Neisseria gonorrhoeae, Neisseria meningitidis, Burkholderia pseudomallei, Burkholderia mallei, Borrelia burgdorferi, Mycobacterium avium complex, Mycobacterium abscessus, Mycobacterium kansasii, E. coli and Mycobacterium ulcerans.*

Examples of bacterial infections may include, but not limited to, upper respiratory infections, lower respiratory infections, ear infections, pleuropulmonary and bronchial infections, complicated urinary tract infections, uncomplicated urinary tract infections, intra-abdominal infections, cardiovascular infections, a blood stream infection, sepsis, bacteremia, CNS infections, skin and soft tissue infections, GI infections, bone and joint infections, genital infections, eye infections, or granulomatous infections. Examples of specific bacterial infections include, but not limited to, uncomplicated skin and skin structure infections (uSSSI), complicated skin and skin structure infections (cSSSI), catheter infections, pharyngitis, sinusitis, otitis extema, otitis media, bronchitis, empyema, pneumonia, community-acquired bacterial pneumoniae (CABP), hospital-acquired pneumonia (HAP), hospital-acquired bacterial pneumonia, ventilator-associated pneumonia (VAP), diabetic foot infections, vancomycin resistant enterococci infections, cystitis and pyelonephritis, renal calculi, prostatitis, peritonitis, complicated intra-abdominal infections (cIAI) and other inter-abdominal infections, dialysis-associated peritonitis, visceral abscesses, endocarditis, myocarditis, pericarditis, transfusion-associated sepsis, meningitis, encephalitis, brain abscess, osteomyelitis, arthritis, genital ulcers, urethritis, vaginitis, cervicitis, gingivitis, conjunctivitis, keratitis, endophthalmitisa, an infection in cystic fibrosis patients or an infection of febrile neutropenic patients.

Furthermore, the invention relates to the use of a compound of formula (I) for the treatment and/or prophylaxis of bacterial infection. The invention relates to the use of a compound of formula (I) for the preparation of a medicament for the treatment and/or prophylaxis of bacterial infection. Another embodiment includes a method for the treatment or prophylaxis of bacterial infection which method comprises administering an effective amount of a compound of formula (I), or pharmaceutically acceptable salt, or enantiomer or diastereomer thereof.

### EXAMPLES

The invention will be more fully understood by reference to the following examples. They should not, however, be construed as limiting the scope of the invention.

### ABBREVIATIONS

Abbreviations used herein are as follows:
- ACN or MeCN: acetonitrile
- AcOK: potassium acetate
- [α]D₂₀: optical rotation at 20 degrees Celsius
- B₂Pin₂: Bis(pinacolato)diboron
- BINAP: 2,2'-Bis(diphenylphosphino)-1,1'-binaphthalene
- BOMCl: benzylchloromethyl ester
- CAMHB: Caution-Adjusted Mueller Hinton Broth
- calc'd: calculated
- CC₅₀: concentration results in the death of 50 percent of the cells
- CL: clearance
- Ct: cycle threshold
- d: day
- DBU: 1,8-Diazabicyclo[5.4.0]undec-7-ene
- DCM: dichloromethane
- DHP: dihydropyran
- DIAD: diisopropyl azodicarboxylate
- DIPEA: N,N-diisopropylethylamine
- EDTA-k2: edathamil
- EtOAc or EA: ethyl acetate
- FA: formic acid
- h(s) or hr(s): hour
- HATU:: 1-[Bis(dimethylamino)methylene]-1H-1,2,3-triazolo[4,5-b]pyridinium 3-oxid hexafluorophosphate
- HPLC:: high performance liquid chromatography
- HPLC-UV:: high performance liquid chromatography with ultraviolet detector
- IV: intravenous
- LAH: lithium aluminum hydride
- LDA: lithium diisopropylamide
- NADPH: nicotinamide adenine dinucleotide phosphate
- MIC: minimum inhibitory concentration
- OD: optical density
- Pd-Ad₂nBuP Biphenyl: Chloro[(di(1-adamantyl)-N-butylphosphine)-2-(2-aminobiphenyl)]palladium(II)
- PE: petroleum ether
- PBS: phosphate buffered saline
- Precat: precatalyst
- prep-HPLC: preparative high performance liquid chromatography
- qPCR: quantitative polymerase chain reaction
- qRT-PCR: quantitative real-time polymerase chain reaction
- rel: relative
- rt: room temperature
- Rt: retention time
- rpm: revolutions per minute
- SEM: 2-methoxyethyl(trimethyl)silane
- SFC: supercritical fluid chromatography
- SM: starting material
- TLC: Thin Layer Chromatography
- UV: ultraviolet detector
- wt: weight

### GENERAL EXPERIMENTAL CONDITIONS

Intermediates and final compounds were purified by flash chromatography using one of the following instruments: i) Biotage SP1 system and the Quad 12/25 Cartridge module, ii) ISCO combi-flash chromatography instrument. Silica gel brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using X Bridge^{™} Perp C₁₈ (5 µm, OBD^{™} 30 × 100 mm) column or SunFire^{™} Perp C₁₈ (5 µm, OBD^{™} 30 × 100 mm) column.

Chiral Separation was conducted on Thar 350 preparative SFC using ChiralPak AD-10µ (200 × 50 mm I.D.) with mobile phase A for CO₂ and B for ethanol. LC/MS spectra were obtained using a Waters UPLC-SQD Mass. Standard LC/MS conditions were as follows (running time: 3 minutes):
Acidic condition: A: 0.1% formic acid and 1% acetonitrile in H₂O; B: 0.1% formic acid in acetonitrile;
Basic condition: A: 0.05% NH₃H₂O in H₂O; B: acetonitrile.
Mass spectra (MS): generally only ions which indicate the parent mass are reported, and unless otherwise stated the mass ion quoted is the positive mass ion (M+H)⁺.
NMR Spectra were obtained using Bruker Avance 400MHz.

All reactions involving air-sensitive reagents were performed under an nitrogen/or argon atmosphere. Reagents were used as received from commercial suppliers without further purification unless otherwise noted.

### PREPARATIVE EXAMPLES

### Intermediate A1

### tert-Butyl N-(3,4-dichloro-6-fluoro-9H-pyrido[2,3-b]indol-8-yl)-N-methyl-carbamate

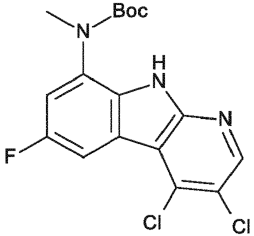

The titled compound was synthesized according to the following scheme:

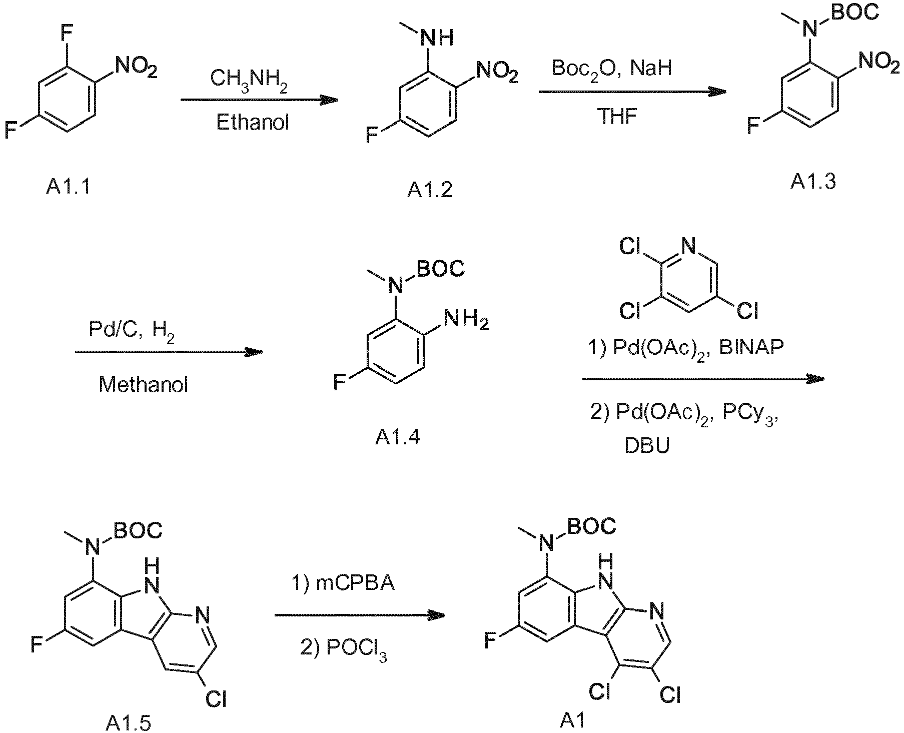

### Step (a) Preparation of 5-Fluoro-N-methyl-2-nitro-aniline (compound A1.2)

Methylamine solution (355 g, 2.866 mol, 25% in ethanol) was added dropwise to 2,4-difluoronitrobenzene (147 g, 0.924 mol) at 0 °C over 15 min. After completion of the addition, the reaction mixture was stirred at 0 °C for 2 h. The solution was diluted with ethanol (500 mL) and poured into 2 L of ice-water. The resulting precipitates were collected by filtration and dried *in vacuo* to give 5-fluoro-N-methyl-2-nitro-aniline (143 g, 63% yield) as a yellow solid.

### Step (b) Preparation of tert-Butyl N-(5-fluoro-2-nitro-phenyl)-N-methyl-carbamate (compound A1.3)

To the suspension of sodium hydride (141 g, 3.5 mol, 60 % dispersion in mineral oil) in dry tetrahydrofuran (2.0 L) was added 5-fluoro-N-methyl-2-nitro-aniline (60 g, 0.35 mol, compound A1.2) portion wise at 0 °C. The solution was stirred at 0 °C for 1 h. Then the solution of di-*tert*-butyl dicarbonate (115 g, 0.53 mol) in tetrahydrofuran (0.5 L) was added dropwise and the reaction continued for another 15 h at 15 °C. The reaction mixture was poured into 1.6 L of ice-water and extracted with EtOAc (1.6 L) two times. The combined organic layer was dried over anhy. sodium sulfate and concentrated *in vacuo.* The crude product was purified by flash chromatography (silica gel, 1-5% ethyl acetate in petroleum ether) to give *tert*-butyl N-(5-fluoro-2-nitro-phenyl)-N-methyl-carbamate (70 g, 73% yield) as a yellow solid. MS (ESI): 293.0 ([M+Na]⁺), 171.0 ([M-C₄H₈-CO₂+H]⁺).

### Step (c) Preparation of tert-Butyl N-(2-amino-5-fluoro-phenyl)-N-methyl-carbamate (compound A1.4)

To the solution of *tert*-butyl N-(5-fluoro-2-nitro-phenyl)-N-methyl-carbamate (70.0 g, 259 mmol, compound A1.3) in methanol (1.0 L) was added palladium on carbon (5.0 g, 10 wt. % loading). The reaction mixture was stirred at 16 °C for 18 h under H₂ atmosphere (50 psi). The remaining palladium catalyst was removed by filtration. The filtrate was concentrated *in vacuo* to give *tert*-butyl N-(2-amino- 5-fluoro-phenyl)-N-methyl-carbamate (60 g, 96% yield) as a white solid. MS (ESI): 263.1 ([M+Na]⁺), 185.0 ([M-C₄H₈ +H]⁺), 141.0 ([M-C₄H₈-CO₂+H]⁺).

### Step (d) Preparation of tert-Butyl N-(3-chloro-6-fluoro-9H-pyrido[2,3-b]indol-8-yl)-N-methylcarbamate (compound A 1.5)

To the solution of *tert*-butyl N-(2-amino-5-fluoro-phenyl)-N-methyl-carbamate (80.0 g, 333 mmol, compound A1.4) and 2,3,5-trichloropyridine (66.8 g, 366 mmol, CAS: 16063-70-0) in dioxane (2.0 L) were added cesium carbonate (217 g, 666 mmol), palladium(II) acetate (3.74 g, 16.7 mmol), and BINAP ( 20.7 g, 33.3 mmol, CAS: 98327-87-8). Reaction continued at 120 °C for 16 h under nitrogen atmosphere. Then the reaction mixture was cooled to room temperature and diluted with EtOAc (800 mL). The precipitate was removed by filtration. The filtrate was concentrated *in vacuo* and the residue was purified by flash chromatography (silica gel, 0.2% to 5% EtOAc in petroleum ether) to give *tert*-butyl N-[2-[(3,5-dichloro-2-pyridyl)amino]-5-fluorophenyl]-N-methyl-carbamate (75 g, 58% yield). MS (ESI): 390.1 ([{³⁷Cl}M+H]⁺), 388.1 ([{³⁷Cl+³⁵Cl }M+H]⁺), 386.1 ([{³⁵Cl}M+H]⁺).

To the solution of *tert*-butyl N-[2-[(3,5-dichloro-2-pyridyl)amino]-5-fluoro-phenyl]-N-methyl-carbamate (5.0 g, 12.95 mmol) and DBU (3.94 g, 25.9 mmol, CAS: 6674-22-2) in the mixture of o-xylene (7.5 mL) and N,N-dimethylacetamide (7.5 mL) were added palladium(II) acetate (727 mg, 3.24 mmol) and tricyclohexylphosphine tetrafluoroborate (2.38 g, 6.48 mmol) under nitrogen atmosphere. The reaction mixture was stirred at 160 °C for 6 h before it was cooled to room temperature and poured into water (100 mL). Then the mixture was extracted with EtOAc (200 mL) and the organic layer was collected and washed with water (50 mL) two times and brine (30 mL) two times, dried over anhy. sodium sulfate. The separated organic layer was concentrated *in vacuo* and the residue was purified by flash chromatography (silica gel, 0.5% to 20% EtOAc in dichloromethane) to give *tert*-butyl N-(3-chloro-6-fluoro-9H-pyrido[2,3-b]indol-8-yl)-N-methyl-carbamate (873 mg, 19.3% yield) as a yellow solid. MS (ESI): 352.1 ([{³⁷Cl}M+H]⁺), 350.1 ([{³⁵Cl}M+H]⁺).

### Step (e) Preparation of tert-Butyl N-(3,4-dichloro-6-fluoro-9H-pyrido[2,3-b] indol-8-yl)-N-methyl-carbamate (Intermediate A1)

To the solution of *tert*-butyl N-(3-chloro-6-fluoro-9H-pyrido[2,3-b]indol-8-yl)-N-methylcarbamate (4.8 g, 13.7 mmol, compound A1.5) in dichloromethane (200 mL) was added 3-chloroperbenzoic acid (9.47 g, 54.9 mmol) at 0 °C under nitrogen atmosphere. Afterwards, the reaction mixture was allowed to warm up to 30°C and stirred for 12 h. Then the reaction mixture was poured into sodium sulfite aqueous solution (10%, 150 mL) and stirred for 1 h followed by extraction with EtOAc (750 mL) three times. The combined organic layer was washed by sodium bicarbonate aqueous solution (5N, 200 mL) and brine (250 mL), then dried over anhy. sodium sulfate and concentrated *in vacuo* to give *tert*-butyl N-(3-chloro-6-fluoro-1-oxido-9H-pyrido [2,3-b]indol-1-ium-8-yl)-N-methyl-carbamate (4.8 g, 96% yield) as a brown solid. MS (ESI): 368.1 ([{³⁷Cl}M+H]⁺), 366.1 ([{³⁵Cl}M+H]⁺).

To the solution of *tert*-butyl N-(3-chloro-6-fluoro-1-oxido-9H-pyrido[2,3-b] indol-1-ium-8-yl)-N-methyl-carbamate (4.8 g, 13.1 mmol) in dimethylformamide (100 mL) was added phosphorus(V) oxychloride (22.1 g, 144 mmol) dropwise at -5 °C. The mixture was stirred at - 5 °C to 0 °C for 1 h before poured into sodium bicarbonate aqueous solution (saturated, 350 mL) at 0 °C. The mixture was extracted by EtOAc (500 mL) three times and the combined organic layer was washed with water (200 mL) three times and brine (150 ml) two times, dried over anhy. sodium sulfate and concentrated *in vacuo.* The crude product was purified by washing with methanol (120 mL) to give tert-butyl N-(3,4-dichloro-6-fluoro-9H-pyrido[2,3-b]indol-8-yl)-N-methyl-carbamate (2.16 g, 42.9 % yield) as a pale yellow solid. MS (ESI): 388.1 ([{³⁷Cl+³⁷Cl }M+H]⁺), 386.1 ([{³⁷Cl+³¹Cl }M+H]⁺), 384.1 ([{³⁵Cl+³⁵Cl }M+H]⁺). ¹H NMR (400 MHz, DMSO-d⁶) δppm: 12.55 (s, 1H), 8.65 (s, 1H), 8.05 (d, J=7.0 Hz, 1H) 7.49 (dd, J=10.3, 2.5 Hz, 1H) 3.26 (s, 3 H) 1.19 - 1.62 (m, 9H).

### Intermediate A2

### tert-Butyl N-(3,4-dichloro-5,6-difluoro-9H-pyrido[2,3-b]indol-8-yl)-N-methyl- carbamate

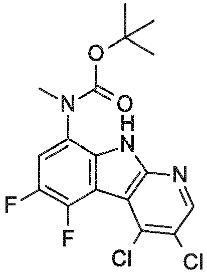

*tert*-Butyl N-(3,4-dichloro-5,6-difluoro-9H-pyrido[2,3-b]indol-8-yl)-N-methyl- carbamate (Intermediate A2) was prepared in analogy to Intermediate A1, by replacing 2,4-difluoronitrobenzene with 2,4,5-trifluoronitrobenzene in step (a). MS (ESI): 406.1 ([{³⁷Cl+³⁷Cl }M+H]⁺), 404.1 ([{³⁷Cl+³⁵Cl }M+H]⁺), 402.1 ([{³⁵Cl+³⁵Cl }M+H]⁺). ¹H NMR (400 MHz, DMSO-d⁶) δ ppm: 12.84 (br. s, 1H), 8.64 (s, 1H), 7.70 (m, 1H), 3.22 (s, 3H), 1.22-1.50 (m, 9H).

### Intermediate A2-Br

### tert-Butyl (3-bromo-4-chloro-5,6-difluoro-9H-pyrido[2,3-b]indol-8-yl) (methyl)carbamate

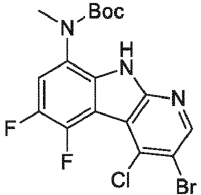

The titled compound was synthesized according to the following scheme:

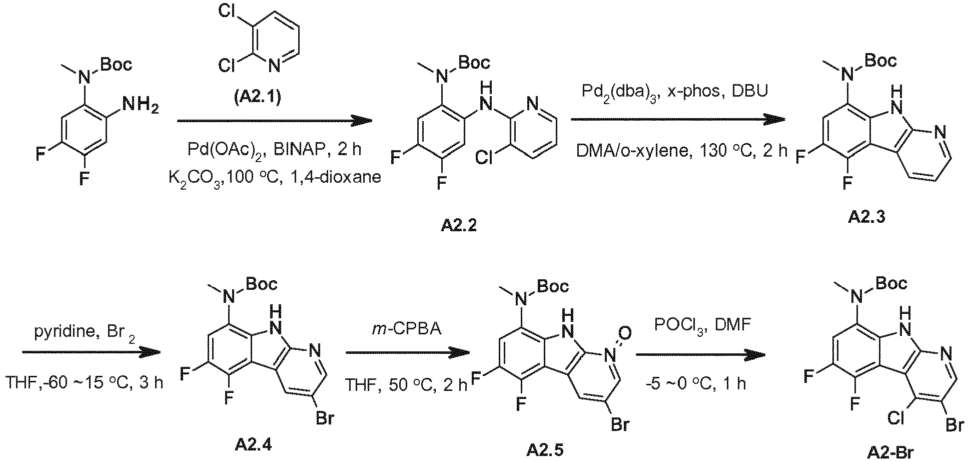

### Step (a) Preparation of tert-butyl (2-((3-chloropyridin-2-yl)amino)-4,5-difluorophenyl)(methyl)carbamate (compound A2.2)

To a stirred solution of *tert*-butyl (2-amino-4,5-difluorophenyl) (methyl)carbamate (142.0 g, 0.550 mol) and 2,3-dichloropyridine (81.4 g, 0.550 mol, compound A2.1) in dioxane (1.5 L) was added cesium carbonate (358.6 g, 1.10 mol). The mixture was degassed with nitrogen for 2 min before palladium(II) acetate (12.3 g, 0.055 mol) and BINAP (68.5 g, 0.110 mmol, CAS: 98327-87-8) were added under nitrogen. The resulting suspension was then heated to 110 °C and stirred for 3 h. The reaction mixture was then cooled to r.t., diluted with EtOAc (1.0 L) and filtered. The filtrate was concentrated *in vacuo* to give a crude product, which was purified by silica gel flash chromatography (petroleum ether: EtOAc = 500:1-20:1) to give *tert*-butyl (2-((3-chloropyridin-2-yl)amino)-4,5-difluorophenyl) (methyl)carbamate (175.0 g, 86% yield) as a white solid. MS (ESI): 370.1 ([{³⁵Cl}M+H]⁺), 372.1 ([{³⁷Cl}M+H]⁺).

### Step (b) Preparation of tert-butyl (5,6-difluoro-9H-pyrido[2,3-b]indol-8-yl) (methyl)carbamate (compound A2.3)

*tert*-Butyl (2-((3-chloropyridin-2-yl)amino)-4,5-difluorophenyl)(methyl) carbamate (175.0g, 473.24 mmol, compound A2.2), Pd₂(dba)₃ (65.0 g, 71.0 mmol, CAS: 51364-51-3), x-phos (67.7 g, 142.0 mmol, CAS: 564483-18-7) and DBU (144.0 g, 946.5 mmol, CAS: 6674-22-2) were dissolved in a mixture solution of o-xylene/ DMA (v/v=1:1, 260.0 mL). After the mixture solution was degassed with nitrogen for 5 min, it was heated at 130 °C for 3 h before cooled to r.t. and diluted with EtOAc (500 mL). The mixture was filtered to remove part of catalyst and ligand and the filtrate was poured into water (1.0 L) and extracted with EtOAc (2.0 L) three times. The combined organic phase was washed with aq. calcium chloride solution (1N, 500 mL) four times, brine (500 mL) twice, and dried over anhy. sodium sulfate. After filtration, the filtrate was concentrated *in vacuo* to give a crude product. It was then recrystallized with MeOH (800 mL) to give *tert*-butyl (5,6-difluoro-9H-pyrido[2,3-b]indol-8-yl) (methyl)carbamate (110.0 g, 69.7% yield) as a white solid. The mother liquid was purified by silica gel flash chromatography (petroleum ether: EtOAc = 50:1-1:1) to give additional *tert*-butyl (5,6-difluoro-9H-pyrido[2,3-b]indol-8-yl)(methyl)carbamate (35.0 g, yield: 22.2%) as a white solid. In total, the title compound (145.0 g, yield: 91.9%) was obtained. MS (ESI): 334.1 ([M+H]⁺).

### Step (c) Preparation of tert-butyl (3-bromo-5,6-difluoro-9H-pyrido[2,3-b]indol-8-yl)(methyl)carbamate (compound A2.4)

To a stirred solution of *tert*-butyl (5,6-difluoro-9H-pyrido[2,3-b]indol-8-yl) (methyl)carbamate (140.0 g, 0.420 mol) and pyridine (166.1 g, 2.1 mol) in tetrahydrofuran (2.0 L) was added bromine (335.6 g, 2.1 mol) at -60 °C. After the addition, the mixture was warmed up and stirred at 15 °C for 2 h. After LCMS showed starting material was consumed, the mixture was added dropwise into aq. sodium sulfite solution (5.0 L, 10%) and adjusted pH = 8.0 with aq. sodium carbonate solution at 0 °C and stirred at 25 °C for additional 1 h. The mixture was extracted by EtOAc (2.0 L) three times. The combined organic layer was then washed with aq. sodium carbonate solution (1.0 L) twice and brine (1.0 L) three times. Separated organic layer was dried over anhy. sodium sulfate, filtered, and concentrated *in vacuo* to give a crude product, which was recrystallized in MeOH (1.0 L) to give *tert*-butyl (3-bromo-5,6-difluoro-9H-pyrido[2,3-b]indol-8-yl)(methyl)carbamate (150.0 g, 86.6% yield) as a yellow solid. MS (ESI): 412.1 ([{⁷⁹Br}M+H]⁺), 414.1 ([{⁸¹Br}M+H]⁺).

### Step (d) Preparation of 3-bromo-8-((tert-butoxycarbonyl)(methyl)amino)- 5,6-difluoro-9H-pyrido[2,3-b]indole 1-oxide (compound A2.5)

To a stirred solution of *tert*-butyl (3-bromo-5,6-difluoro-9H-pyrido[2,3-b]indol-8-yl)(methyl)carbamate (150.0 g, 363.9 mmol) in tetrahydrofuran (1.2 L) was added 3-chlorobenzoperoxoic acid (148.0 g, 727.8 mmol, 85% wt). The mixture was degassed with nitrogen for 2 min before stirred at 50 °C for 2 h. After TLC (petroleum ether: EtOAc = 2:1) showed the starting material was consumed completely, the reaction was cooled back to r.t. and the mixture was poured into aq. sodium sulfite solution (6.0 L, 10%) and stirred for 1 h. The resulting precipitate was collected by filtration and washed by aq. sodium sulfite solution (1.0 L, 5%). The filter cake was evaporated to dryness under reduced pressure to give 3-bromo-8-((*tert-*butoxycarbonyl)(methyl)amino)-5,6-difluoro-9H-pyrido[2,3-b]indole 1-oxide (150.0 g, 96.3% yield) as a yellow solid. MS (ESI): 428.1 ([{⁷⁹Br}M+H]⁺), 430.1 ([{⁸¹Br}M+H]⁺).

### Step (e) Preparation of tert-butyl (3-bromo-4-chloro-5,6-difluoro-9H-pyrido [2,3-b]indol-8-yl)(methyl)carbamate (Intermediate A2-Br)

To a stirred solution of 3-bromo-8-((*tert*-butoxycarbonyl)(methyl)amino)- 5,6-difluoro-9H-pyrido[2,3-b]indole 1-oxide (150 g, 0.35 mol) in dimethylformamide / tetrahydrofuran (2.5 L, v/v = 1:1) was added phosphorus(V) oxychloride (421.3 g, 2.75 mol) dropwise in ice-salt bath. The mixture was stirred at -5 ~ 0 °C for 4 h until TLC (petroleum ether: EtOAc = 2:1) showed the starting material was consumed completely. The mixture was poured into saturated aq. solution of sodium carbonate (5.0 L) at 0 °C and extracted with EtOAc (3.0 L) twice. The combined organic layer was washed sequentially with saturated aq. solution of sodium carbonate (1.0 L) twice, aq. calcium chloride solution (1N, 1.5 L) four times, and brine (1.0 L) twice. The organic layer was dried over anhy. sodium sulfate, filtered, and concentrated *in vacuo* to give a crude product, which was recrystallized with MeOH (1.2 L) to give *tert*-butyl (3-bromo-4-chloro-5,6-difluoro-9H-pyrido[2,3-b]indol-8-yl)(methyl)carbamate (142.0 g, 90.8% yield) as a white solid. ¹H NMR (400 MHz, DMSO-d6) δ: 12.85 (br. s., 1 H), 8.75 (s, 1 H), 7.69 ~ 7.74 (dd, *J*=11.6, 6.8 Hz, 1 H), 3.22 (s, 3 H), 1.22 ~ 1.50 (m, 9 H). MS (ESI): 446.0 ([{⁷⁹Br} M+H]⁺), 448.0 ([{⁸¹Br} M+H]⁺).

### Intermediate A1-Br

### tert-Butyl N-(3-bromo-4-dichloro-6-fluoro-9H-pyrido[2,3-b]indol-8-yl)-N-methylcarbamate

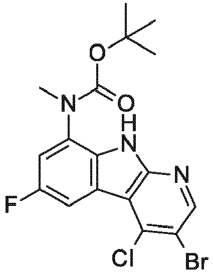

*tert*-Butyl N-(3-bromo-4-dichloro-6-fluoro-9H-pyrido[2,3-b]indol-8-yl)-N-methylcarbamate (Intermediate A1-Br) was prepared in analogy to Intermediate A2-Br, by replacing 2, 4,5-trifluoronitrobenzene with 2,4-difluoronitrobenzene in step (a). MS (ESI): 428.1 ([{⁸¹Br }M+H]⁺), 426.1 ([{⁷⁹Br }M+H]⁺). ¹H NMR (400 MHz, DMSO-d⁶) δ ppm: 12.82 (s, 1H), 8.65 (s, 1H), 8.01 (d, J=7.0 Hz, 1H) 7.48 (dd, J=7.0, 2.5 Hz, 1H) 3.26 (s, 3 H) 1.20 - 1.60 (m, 9H).

### Intermediate A3

### tert-Butyl (3-bromo-4,5-dichloro-6-fluoro-9H-pyrido[2,3-b]indol-8-yl)(methyl) carbamate

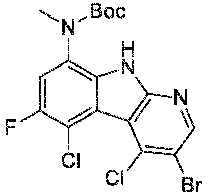

To a solution of *tert*-butyl N-(3-bromo-4-dichloro-6-fluoro-9H-pyrido[2,3-b]indol-8-yl)-N-methyl- carbamate (2.50 g, 5.83 mmol, Intermediate A1-Br) in DMF (20.0 mL) was added TsOH•H₂O (700 mg, 3.68 mmol) and NCS (1.70 g, 11.46 mmol). The resulting mixture was stirred at 50 °C for 2 h before it was cooled to r.t. and poured into water (200 mL), extracted with EtOAc (100 mL) three times. The combined organic layer was washed with brine (100 mL) twice, dried over anhydrous Na₂SO₄, filtered, and concentrated *in vacuo* to give a residual, which was purified by silica gel chromatography with petroleum ether/EtOAc (20/1 to 5/1 gradient) to give tert-butyl (3-bromo-4,5-dichloro-6-fluoro-9H-pyrido[2,3-b]indol-8-yl)(methyl)carbamate (1.80 g, 66.6%) as a white solid. MS (ESI): 464.0 [{³⁵Cl^{∗}2+⁸¹Br & ³⁵Cl+³⁷Cl+ 7⁹Br}(M+H)+]. ¹H NMR (400 MHz, DMSO) *δ*ppm: 12.94 (s, 1H), 8.78 (s, 1H), 7.72-7.70 (d, 1 H), 3.22 (s, 1H), 1.49 (s, 3H), 1.25 (s, 6H).

### Intermediate A4

### tert-Butyl (3,4-dichloro-5-cyano-6-fluoro-9H-pyrido[2,3-b]indol-8-yl)(methyl) carbamate

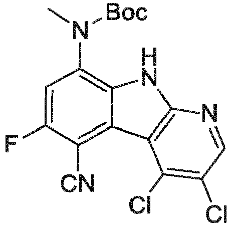

The titled compound was synthesized according to the following scheme:

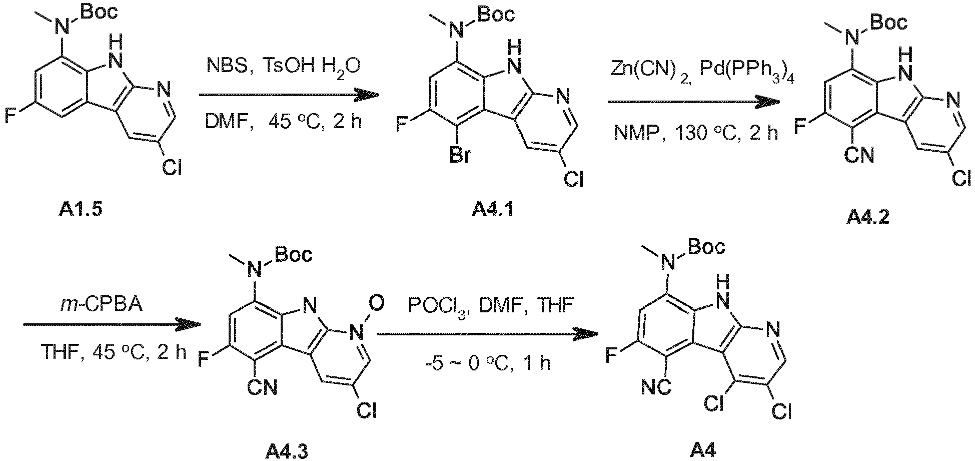

### Step (a) Preparation of tert-butyl (5-bromo-3-chloro-6-fluoro-9H-pyrido[2,3-b] indol-8-yl)(methyl)carbamate (compound A4.1)

To a solution of *tert*-butyl (3-chloro-6-fluoro-9H-pyrido[2,3-b]indol-8-yl) (methyl)carbamate (4.5 g, 12.86 mmol, compound A1.5) in anhydrous DMF (60.0 mL) was added NBS (4.58 g, 25.72 mmol) and TsOH•H₂O (1.22 g, 6.43 mmol). The reaction mixture was stirred at 45 °C for 2 h before it was cooled back to r.t. and poured into aq. Na₂SO₃ solution (10%, 200.0 mL) and adjusted to pH = 8 with aq. Na₂CO₃ solution. The mixture was stirred at 25 °C for additional 0.5 h before it was extracted with EtOAc (150 mL) three times. The combined organic layer was washed with satd. aq. CaCl₂ solution (100.0 mL) four times, brine (100.0 mL) three times, dried over anhy. Na₂SO₄, filtered, and concentrated *in vacuo* to give a crude product. It was then recrystallized in MeOH (40.0 mL) to give *tert*-butyl (5-bromo-3-chloro-6-fluoro-9H-pyrido[2,3-b]indol-8-yl)(methyl)carbamate (4.8 g, 87.0% yield) as a yellow solid. MS (ESI): 428.0 ([{⁷⁹Br}M+H]⁺), 430.0 ([{⁸¹Cl}M+H]⁺).

### Step (b) Preparation of tert-butyl (3-chloro-5-cyano-6-fluoro-9H-pyridor2,3-b1 indol-8-yl)(methyl)carbamate (compound A4.2)

To a solution of *tert*-butyl (5-bromo-3-chloro-6-fluoro-9H-pyrido[2,3-b] indol-8-yl)(methyl)carbamate (1.3 g, 3.03 mmol), Zn(CN)₂ (1.42 g, 12.13 mmol) in NMP (10.0 mL) was added Pd(PPh₃)₄ (350.0 mg, 0.303 mmol) in glovebox under argon. The resulting reaction mixture was stirred at 130 °C for 2 h under argon. After LCMS showed the starting material was consumed completely, the reaction was cooled to r.t., and the mixture was diluted with EtOAc (200 mL) and filtered. The filtrate was washed with brine (80 mL) three times, and the organic layer was dried over anhy. Na₂SO₄, filtered, and concentrated *in vacuo* to give a crude product, which was recrystallized with MeOH to give *tert*-butyl (3-chloro-5-cyano-6-fluoro-9H-pyrido[2,3-b]indol-8-yl) (methyl)carbamate (1.0 g, 87.98% yield) as a yellow solid. MS (ESI): 375.0 ([{³⁵Cl}M+H]⁺), 377.0 ([{³⁷Cl}M+H]⁺).

### Step (c) Preparation of 8-((tert-butoxycarbonyl)(methyl)amino)-3-chloro-5-cyano-6-fluoro-9H-pyrido[2,3-b]indole 1-oxide (compound A4.3)

To a solution of *tert*-butyl (3-chloro-5-cyano-6-fluoro-9H-pyrido[2,3-b]indol-8-yl)(methyl)carbamate (1.0 g, 2.67 mmol) in THF (10.0 mL) was added m-CPBA (1.08 g, 5.34 mmol, 85% wt). After the reaction mixture was degassed with nitrogen for 2 min, it was stirred at 45 °C for 2 h. After TLC (petroleum ether: EtOAc = 2:1) showed the starting material was consumed completely, the reaction mixture was poured into aq.Na₂SO₃ solution (10%, 50.0 mL) and stirred at r.t. for 1 h. The resulting precipitate was collected by filtration and the filter cake was washed with 5% aq. Na₂SO₃ solution (5%, 50.0 mL). The filter cake was dried under vacuum to give a crude product of 8-((*tert*-butoxycarbonyl)(methyl)amino)-3-chloro-5-cyano-6-fluoro-9H-pyrido[2,3-b]indole 1-oxide (1.0 g, 95.9% yield) as a yellow solid. MS (ESI): 391.1 ([{³⁵Cl}M+H]⁺), 393.1 ([{³⁷Cl}M+H]⁺). The crude product was used directly in the next step without further purification.

### Step (d) Preparation of tert-butyl (3,4-dichloro-5-cyano-6-fluoro-9H-pyrido[2,3-b] indol-8-yl)(methyl)carbamate (Intermediate A4)

To a solution of 8-((*tert*-butoxycarbonyl)(methyl)amino)-3-chloro-5-cyano-6-fluoro-9H-pyrido[2,3-b]indole 1-oxide (1.0 g, 2.56 mmol) in DMF/THF (10.0 mL, v/v = 1:1) was added POCl₃ (4.26 g, 27.8 mmol) dropwise in ice-salt bath and the resulting reaction mixture was stirred at -5 ~ 0 °C for 1 h. After TLC (petroleum ether: EtOA= 2:1) showed the starting material was consumed completely, the reaction mixture was poured into satd. aq. NaHCO₃ solution (100 mL) at 0 °C and subsequently extracted with EtOAc (200 mL) twice. The combined organic layer was washed with satd. aq. NaHCO₃ solution (50.0 mL) twice, aq. CaCl₂ solution (1 N, 50 mL) four times and brine (50 mL) twice. The organic layer was dried over anhy. Na₂SO₄, filtered, and concentrated *in vacuo* to give a crude product, which was purified by silica gel flash chromatography (petroleum ether: EtOAc = 100:1-2:1, 20% DCM as additive) to give *tert*-butyl (3,4-dichloro-5-cyano-6-fluoro-9H-pyrido [2,3-b]indol-8-yl)(methyl)carbamate (900.0 mg, 85.94% yield) as a yellow solid. MS (ESI): 409.2 ([{³⁵Cl}M+H]⁺), 411.2 ([{³⁷Cl}M+H]⁺).

### Intermediate A5

### tert-Butyl (3-bromo-4, 6-dichloro-9H-pyrido[2,3-b]indol-8-yl)(methyl)carbamate

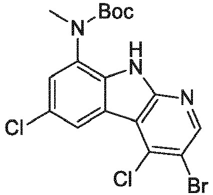

The titled compound was synthesized according to the following scheme:

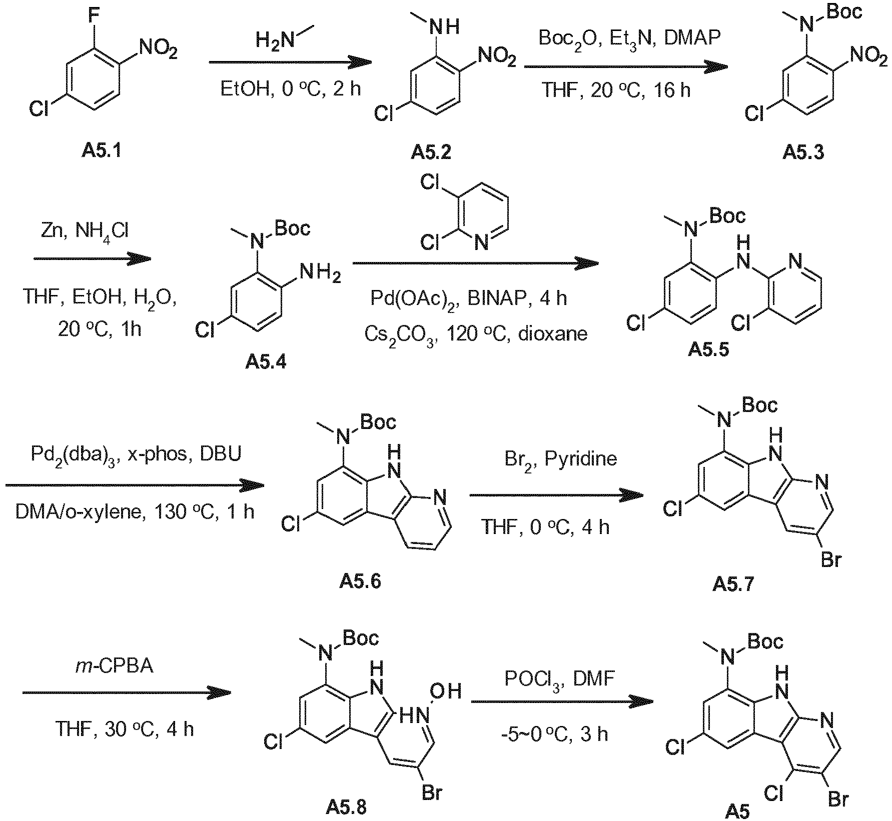

### Step (a) Preparation of 5-chloro-N-methyl-2-nitroaniline (compound A5.2)

Methylamine solution (50.0 g, 0.28 mol, 25% in ethanol) was added dropwise to 4-chloro-2-fluoro-1-nitrobenzene (26.6 g, 0.86 mmol, compound A5.1) at 0 °C over 15 min. After completion of the addition, the reaction mixture was stirred at 0 °C for 2 h before diluted with ethanol (2.5 L). After TLC (petroleum ether/EtOAc=10:1) showed he reaction was completed, the reaction mixture was poured into ice-water (2L). The resulting precipitate were collected by filtration and the filter cake was re-dissolved in ethyl acetate (1L) and washed with brine. The organic layer was then dried over anhy. Na₂SO₄, filtered, and evaporated to dryness to give 5-chloro-N-methyl-2-nitroaniline (49.2 g, 92.7% yield) as a yellow solid. ¹H NMR (400 MHz, CDCl₃) *δ*ppm: 2.94 - 2.95 (d, J=4.8 Hz, 3 H) 6.53 - 6.56 (d, J=8.8 Hz, 1 H) 6.755 (s, 1 H) 8.035 (br, 1 H) 8.06 - 8.07 (m, 1 H).

### Step (b) Preparation of tert-butyl (5-chloro-2-nitrophenyl)(methyl)carbamate (compound A5.3)

To a solution of 5-chloro-N-methyl-2-nitroaniline (40.2 g, 0.216 mol, compound A5.2) in dry THF (2.0 L) was added Boc₂O (141.5 g, 0.648 mol), DMAP (13.4 g, 0.11 mmol) and Et₃N (25.9 g, 1.08 mmol). The resulting reaction mixture was stirred at 20 °C for 16 h before it was poured into 1 L of ice-water carefully and extracted with EtOAc (2 L) twice. The combined organic phase was dried over anhy. Na₂SO₄, filtered, and concentrated *in vacuo* to give a crude product, which was purified by silica gel flash chromatography (petroleum ether: EtOAc = 100:1 ~ 20:1) to give tert-butyl (5-chloro-2-nitrophenyl)(methyl)carbamate (45.2 g, 73.1% yield) as a yellow solid. ¹H NMR (400 MHz, CDCl₃) *δ*ppm: 1.24 - 1.43 (m, 9 H) 3.23 (s, 3 H) 7.26 - 7.28 (m, 2 H) 7.80 - 7.82 (m, 1 H).

### Step (c) Preparation of tert-butyl (2-amino-5-chlorophenyl)(methyl)carbamate (compound A5.4)

To a solution of tert-butyl (5-chloro-2-nitrophenyl)(methyl)carbamate (45.2 g, 0.16 mol, compound A5.3) in THF (500.0 mL), EtOH (500.0 mL) and H₂O (500.0 mL) was added Zn (60.7 g, 0.95 mol) and NH₄Cl (67.8 g, 1.28 mol) and the resulting reaction mixture was stirred at 20 °C for 1 h. The reaction mixture was then filtered and the filtrate was evaporated to dryness under reduced pressure. The residual was re-dissolved in EtOAc (500mL) and H₂O (500 mL). The organic layer was separated and the aqueous phase was extracted with EtOAc (500 mL) three times. The combined organic layer was dried over anhy. Na₂SO₄, filtered, and concentrated *in vacuo* to give tert-butyl (2-amino-5-chlorophenyl)(methyl)carbamate (35.2 g, 85.9% yield) as crude yellow oil. MS (ESI) [(M-Boc)⁺]: 157.1 ({³⁵Cl} M-Boc)⁺, 159.1 ({³⁷Cl} M-Boc)⁺. The crude product was used directly in the next step without further purification.

### Step (d) Preparation of tert-butyl (5-chloro-2-((3-chloropyridin-2-yl)amino)phenyl) (methyl)carbamate (compound A5.5)

To a solution of tert-butyl (2-amino-5-chlorophenyl)(methyl)carbamate (34.2 g, 133 mmol, compound A5.4) and 2,3-dichloropyridine (21.6 g, 146 mmol) in dioxane (100.0 mL) was added Cs₂CO₃ (87.1 g, 266 mmol). The mixture was degassed with nitrogen for 2 min before Pd(OAc)₂ (1.5 g, 6.65 mmol) and BINAP (8.3 g, 13.3 mmol) was add under nitrogen. The resulting suspension was then heated to 120 °C and stirred for 4 h. After cooling to r.t., the reaction mixture was diluted with EtOAc (500 mL) and filtered. The filtrate was concentrated *in vacuo* to give a crude product, which was purified by silica gel flash chromatography (petroleum ether: EtOAc = 50:1-15:1) to give *tert-butyl* (5-chloro-2-((3-chloropyridin-2-yl)amino)phenyl)(methyl)carbamate (32.2 g, 66.0% yield). MS (ESI) [(M-Boc)⁺]: 268.0 ({³⁵Cl} M-Boc)⁺, 270.0 ({³⁷Cl} M-Boc)⁺, 368.0 ({³⁵Cl} M+H)⁺, 370.0 ({³⁷Cl} M+H)⁺. ¹H NMR (400 MHz, CDCl₃) *δ*ppm: 1.17 - 1.29 (m, 9 H) 3.13 (s, 3 H) 6.66 (br, 1 H) 7.13 - 7.17 (m, 2 H) 7.50 (m, 1 H) 7.71 - 7.73 (m, 1 H) 8.24 - 8.25 (m, 1 H) 8.25 (m, 1 H).

### Step (e) Preparation of tert-butyl (6-chloro-9H-pyrido[2,3-b]indol-8-yl)(methyl) carbamate (compound A5.6)

*tert*-butyl (5-chloro-2-((3-chloropyridin-2-yl)amino)phenyl)(methyl)carbamate (25.0 g, 68.1 mmol, compound A5.5), Pd₂(dba)₃ (12.5 g, 13.6 mmol), x-phos (13.0 g, 27.2 mmol) and DBU (20.7 g, 136.2 mmol) were dissolved in a mixture of o-xylene (60 mL) and dimethylacetamide (60 mL). After degassed with argon for 5 min, the reaction mixture was heated at 130 °C for 1 h. The reaction was cooled back to r.t. and poured into ethyl acetate (300 mL) and filtered. The filtrate was evaporated under reduced pressure to give a crude product, which was recrystallized in a mixture of MeOH and petroleum ether (100 mL, 4:1) to give tert-butyl (6-chloro-9H-pyrido[2,3-b]indol-8-yl)(methyl)carbamate (15.1 g, 56.1% yield) as a white solid. MS (ESI) [(M-Boc)⁺]: 232.0 ({³⁵Cl} M-Boc)⁺, 234.0 ({³⁷Cl} M-Boc)⁺, 332.0 ({³⁵Cl} M+H)⁺, 334.0 ({³⁷Cl} M+H)⁺.¹H NMR (400 MHz, DMSO) *δ*ppm: 1.15 - 1.51 (m, 9 H) 3.25 (s, 3 H) 7.22 - 7.27 (m, 1 H) 7.40- 7.41 (m, 1 H) 8.23 (m, 1 H) 8.44 - 8.49 (m, 1 H) 8.56- 8.58 (m, 1 H) 12.06 (s, 1 H).

### Step (f) Preparation of tert-butyl (3-bromo-6-chloro-9H-pyrido[2,3-b]indol-8-yl) (methyl)carbamate (compound A5.7)

To a solution of tert-butyl (6-chloro-9H-pyrido[2,3-b]indol-8-yl)(methyl)carbamate (7.0 g, 21.1 mmol, compound A5.6) in THF was added Br₂ (16.7 g, 105.7 mmol) and pyridine (8.4 g, 105.7 mmol) at -76 °C. After the addition, the reaction mixture was stirred at 0 °C for 4 h before poured into water (500 mL), and extracted with EtOAc (500 mL) three times. The combined organic layer was dried over anhy Na₂SO₄, filtered, and concentrated *in vacuo* to give a crude product, which was recrystallized in a mixture of MeOH and petroleum ether (100 mL, 4;1) to give *tert*-butyl (3-bromo-6-chloro-9H-pyrido[2,3-b]indol-8-yl)-(methyl)carbamate (13.2 g, 75.9% yield) as a white solid. MS (ESI) [(M-Boc)⁺]: 410.0 ({³⁵Cl, ⁷⁹Br} M+H)⁺, 412.0 ({³⁵Cl, ⁷⁹Br} M+H⁺, {³⁷Cl, ⁸¹Br} M+H)⁺, 414.0 ({³⁷Cl, ⁸¹Br} M+H)⁺.¹H NMR (400 MHz, DMSO) *δ* ppm: 1.19 - 1.50 (m, 9 H) 3.25 (s, 3 H) 7.46 (s, 1 H) 8.26 (s, 1 H) 8.56 (s, 1 H) 8.88 (s, 1 H) 9.01 (s, 1 H) 12.27 (s, 1 H).

### Step (g) Preparation of tert-butyl (3-bromo-6-chloro-9H-pyrido[2,3-b]indol-8-yl) (methyl)carbamate (compound A5.8)

To a solution of tert-butyl (3-bromo-6-chloro-9H-pyrido[2,3-b]indol-8-yl)-(methyl)carbamate (12.7 g, 31.1 mmol, compound A5.7) in THF (130 mL) was added m-CPBA (8.1 g, 46.6 mmol). The resulting reaction mixture was stirred at 30 °C for 4 h before poured into 5% aq.Na₂SO₃ (500.0 mL) and stirred for another 30 min. The reaction mixture was then filtered and filter cake was dried under vacuum to give a crude product of *tert*-butyl (3-bromo-6-chloro-9H-pyrido[2,3-b]indol-8-yl)(methyl)carbamate (12.1 g, 91.7% yield) as a yellow solid. MS (ESI) [(M-Boc)⁺]: 425.8 ({³⁵Cl, ⁷⁹Br} M+H)⁺, 427.8 ({³⁵Cl, ⁷⁹Br} M+H, {³⁷Cl, ⁸¹Br} M+H)⁺, 429.8 ({³⁷Cl, ⁸¹Br} M+H)⁺. It was used directly in the next step without further purification.

### Step (h) Preparation of tert-butyl (3-bromo-4,6-dichloro-9H-pyrido[2,3-b]indol-8-yl) (methyl)carbamate (Intermediate AS)

To a solution of tert-butyl (3-bromo-6-chloro-9H-pyrido[2,3-b]indol-8-yl) (methyl)carbamate (11.6 g, 27.3 mmol, compound A5.8) in DMF (100.0 mL) and THF (100.0 mL) was added POCl₃ (29.0 g, 191 mmol) under N₂ at 0 °C, and the reaction mixture was stirred at -5-0 °C for 3 h. Afterwards, the reaction mixture was quenched with satd. aq. NaHCO₃ solution (800 mL), and extracted with EtOAc (500 mL) three times. The combined organics were then washed with brine, dried over anhy. Na₂SO₄, filtered, and concentrated *in vacuo* to give a crude product, which was recrystallized in MeOH (100 mL) to give *tert*-butyl (3-bromo-4,6-dichloro-9H-pyrido [2,3-b]indol-8-yl)(methyl)carbamate (10.2 g, 84.3% yield) as a yellow solid. MS (ESI) [(M-Boc)⁺]: 443.8 ({³⁵Cl, ⁷⁹Br} M+H)⁺, 445.8 ({³⁵Cl, ⁷⁹Br} M+H⁺, {³⁷Cl, ⁸¹Br} M+H)⁺, 447.8 ({³⁷Cl, ⁸¹Br} M+H)⁺. ¹H NMR (400 MHz, DMSO) *δ*ppm: 1.18 - 1.51 (m, 9 H) 3.24-3.26 (d, J=10.8Hz, 3 H) 7.59 (s, 1 H) 8.25Tan (s, 1 H) 8.72 (s, 1 H) 12.65 (s, 1 H).

### Intermediate A6

### tert-Butyl (3-bromo-4,6-dichloro-5-cyano-9H-pyrido[2,3-b]indol-8-yl)(methyl) carbamate

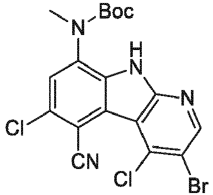

The titled compound was synthesized according to the following scheme:

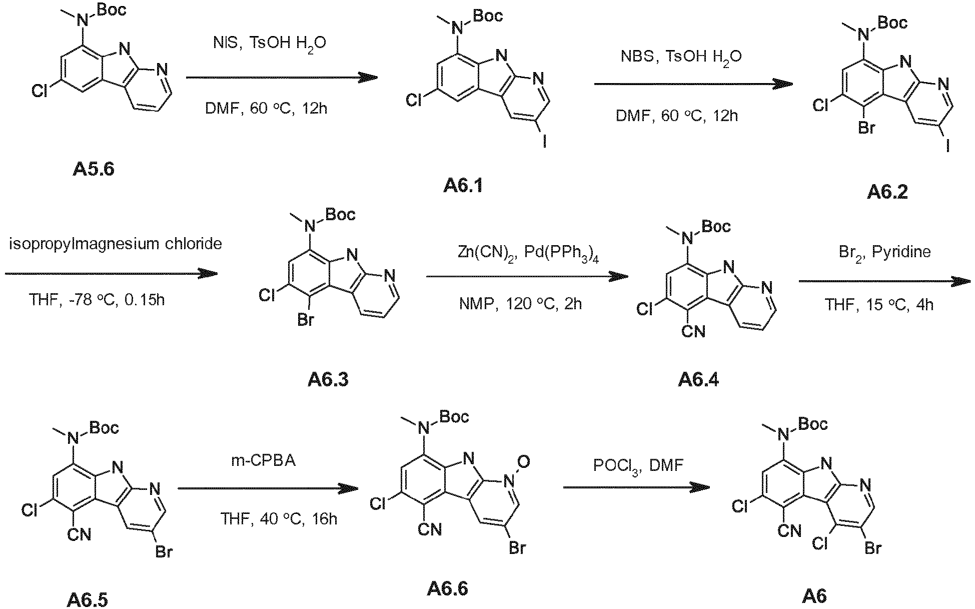

### Step (a) Preparation of tert-butyl (6-chloro-3-iodo-9H-pyrido[2,3-b]indol-8-yl)(methyl)carbamate (compound A6.1)

A solution of *tert*-butyl N-(6-chloro-9H-pyrido[2,3-b]indol-8-yl)-N-methyl-carbamate (compound A5.6, 15.0 g, 45.21 mmol), NIS (3.0 g, 135.62 mmol) and PTSA (3.4 g, 18.08 mmol) in DMF (5 mL) was stirred at 60 °C for 3 h. After it was cooled down to r.t., the reaction mixture was poured into aq. NaHSO₃ solution and the precipitated solid was collected and washed with MeOH to give a crude *tert*-butyl (6-chloro-3-iodo-9H-pyrido[2,3-b]indol-8-yl)(methyl)carbamate (20 g, 43.7 mmol, 96.66 % yield) as a yellow solid. MS (ESI): 458.0 ([{³⁵Cl}M+H]⁺), 460.0([{³⁷Cl}M+H]⁺).

### Step (b) Preparation of tert-butyl (5-bromo-6-chloro-3-iodo-9H-pyrido[2,3-b]indol-8-yl)(methyl)carbamate (compound A6.2)

To a stirred solution of *tert*-butyl (6-chloro-3-iodo-9H-pyrido[2,3-b]indol-8-yl)(methyl)carbamate compound A6.1, 1.0 g, 2.18 mmol) in DMF (10 mL) was added NBS (583.4 mg, 3.28 mmol) and PTSA (166.2 mg, 0.87 mmol, 0.4 eq). The resulting mixture was stirred at 40 °C for 3 h before it was poured into aq. Na₂SO₃ solution. The precipitated solid was collected and washed with EtOAc, and dried under vacuum to give *tert*-butyl (5-bromo-6-chloro-3-iodo-9H-pyrido[2,3-b]indol-8-yl)(methyl)carbamate (800 mg, 1.49 mmol, 68.24% yield) as a yellow solid. MS (ESI): 535.9 ([{⁷⁹Br}M+H]⁺), 537.9 ([{⁸¹Br}M+H]⁺).

### Step (c) Preparation of tert-butyl (5-bromo-6-chloro-9H-pyrido[2,3-b]indol-8-yl)(methyl)carbamate (compound A6.3)

To a solution of *tert*-butyl (5-bromo-6-chloro-3-iodo-9H-pyridor2,3-b]indol-8-yl)(methyl)carbamate (compound A6.2, 3.5 g, 6.5 mmol) in DMF (150 mL) was added Pd/C (350.0 mg ) and triethylamine (2.73 mL, 19.6 mmol). After purged with N₂ several times, the reaction mixture was stirred at 10 °C for 5 h under a H₂ balloon. After the reaction, the mixture was filtered through a Celite, and the filtrate was concentrated under reduced pressure to give a crude product, which was then purified by silica gel flash chromatography (petroleum ether / ethyl acetate = 2 :1) to give *tert*-butyl (5-bromo-6-chloro-9H-pyrido[2,3-b] indol-8-yl)(methyl)carbamate (4 g, 9.74 mmol, 103 % yield) as a yellow solid. MS (ESI): 412.1 ([{⁸¹Br}M+H]⁺), 410.1([{⁷⁹Br}M+H]⁺).

### Step (d) Preparation of tert-butyl (6-chloro-5-cyano-9H-pyridor2,3-b]indol-8-yl)(methyl)carbamate (compound A6.4)

A mixture solution of *tert*-butyl (5-bromo-6-chloro-9H-pyrido[2,3-b]indol-8-yl)(methyl)carbamate (compound A6.3, 1.9 g, 4.63 mmol), zinc cyanide (2.72 g, 23.13 mmol), tetrakis (triphenylphosphine) palladium (0) (801.9 mg, 0.69 mmol) in NMP (13 mL) was stirred at 120 °C for 1.5 h under Ar. After it was cooled back to r.t., the reaction mixture was diluted with 50 mL of EtOAc and filtered. The filtrate was then washed with aq. CaCl₂ solution (30 mL) five times, and the combined organics were dried over anhy. Na₂SO₄, filtered, and concentrated in vacuo. The residue was suspended in MeOH (30 mL) and stirred for 20 min before filtered. The filter cake was then dried under vacuum to give *tert*-butyl (6-chloro-5-cyano-9H-pyrido[2,3-b]indol-8-yl)(methyl)carbamate (1.9 g, 5.32 mmol, 92.08 % yield) as a yellow solid. MS (ESI): 357.2 ([{³⁷Cl}M+H]⁺), 359.2([{³⁵Cl}M+H]⁺).

### Step (e) Preparation of tert-butyl (3-bromo-6-chloro-5-cyano-9H-pyrido[2,3-b]indol-8-yl)(methyl)carbamate (compound A6.5)

To a solution of *tert*-butyl (6-chloro-5-cyano-9H-pyrido[2,3-b]indol-8-yl)(methyl)carbamate (compound A6.4, 1.8 g, 5.04 mmol) in THF (36 mL) was added pyridine (6.12 mL, 75.67 mmol). After the reaction mixture was cooled to -78 °C under N₂, bromine (8.06 g, 50.45 mmol) was added using a syringe. The resulting mixture solution was allowed to warm up to 10 °C and stirred at for 2 h. The reaction was then quenched by the addition of 5% aq. Na₂SO₃ solution (100 mL). The reaction mixture was extracted with EtOAc (100 mL) two times, and the combined organic layer was dried with anhy. Na₂SO₄, filtered, and concentrated in vacuo. The residue was suspended in MeOH (15 mL) and filtered. The filter cake was dried under vacuum to give *tert*-butyl (3-bromo-6-chloro-5-cyano-9H-pyrido[2,3-b]indol-8-yl)(methyl) carbamate (1.4 g, 3.21 mmol, 43.31% yield) as a yellow solid. MS (ESI): 437.0 ([{⁸¹Br}M+H]⁺), 435.0([{⁷⁹Br}M+H]⁺).

### Step (f) Preparation of 3-bromo-8-((tert-butoxycarbonyl)(methyl)amino)-6-chloro-5-cyano-9H-pyrido[2,3-b] indole 1-oxide (compound A6.6)

To a solution of *tert*-butyl (3-bromo-6-chloro-5-cyano-9H-pyrido[2,3-b]indol-8-yl)(methyl)carbamate (compound A6.5, 1.35 g, 3.1 mmol) in THF (0.5 mL) was added 3-chloroperoxybenzoic acid (1.6 g, 9.3 mmol), and the reaction mixture was stirred at 40 °C for 16 h before it was poured into 5% aq. Na₂SO₃ solution (100 mL) and stirred vigorously for 0.5 h. The precipitate was collected by filtration and dried under vacuum to give a crude product of 3-bromo-8- ((*tert*-butoxycarbonyl)(methyl)amino)-6-chloro-5-cyano-9H-pyrido[2,3-b]indole 1-oxide (1.4 g, 3.1 mmol, 68.02% yield) as a yellow solid. MS (ESI): 453.0 ([{⁸¹Br}M+H]⁺), 451.0([{⁷⁹Br}M+H]⁺). It was used directly in the next step without further purification.

### Step (g) Preparation of tert-butyl (3-bromo-4,6-dichloro-5-cyano-9H-pyrido[2,3-b]indol-8-yl)(methyl) carbamate (Intermediate A6)

To a solution of 3-bromo-8-((*tert*-butoxycarbonyl)(methyl)amino)-6-chloro-5-cyano-9H-pyrido[2,3-b] indole 1-oxide (compound A6.6, 1.4 g, 3.1 mmol) in DMF (20 mL) was added phosphorus oxychloride (0.43 mL, 4.65 mmol) at -30 °C under N₂. The resulting reaction mixture was stirred first at -30 °C for 2 h, and then 10 °C for 14 h before it was poured into 5% aq. NaHCO₃ solution (100 mL). The precipitate was collected by filtration, and the filter cake was washed with water (20 mL) and dried under vacuum to give *tert*-butyl (3-bromo-4,6-dichloro-5-cyano-9H-pyrido[2,3-b]indol-8-yl)(methyl)carbamate (1.4 g, 2.98 mmol, 63.41% yield) as a yellow solid. MS (ESI): 471.0 ([{⁸¹Br}M+H]⁺), 469.0([{⁷⁹Br}M+H]⁺).

### Intermediate B1

### Ethyl 4-oxo-7-(4,4,5,5-tetramethyl-1,3,2-dioxaborolan-2-yl)-4H-quinolizine-3-carboxylate

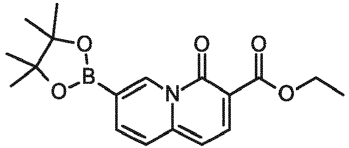

The titled compound was synthesized according to the following scheme:
**Method A:** 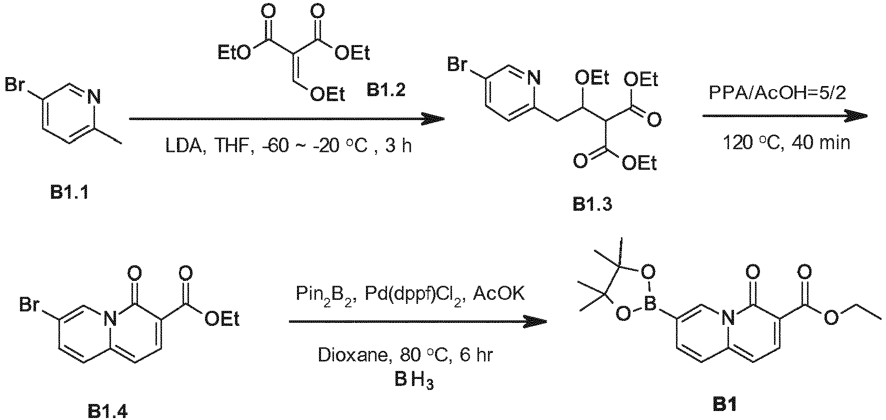
**Method B:**

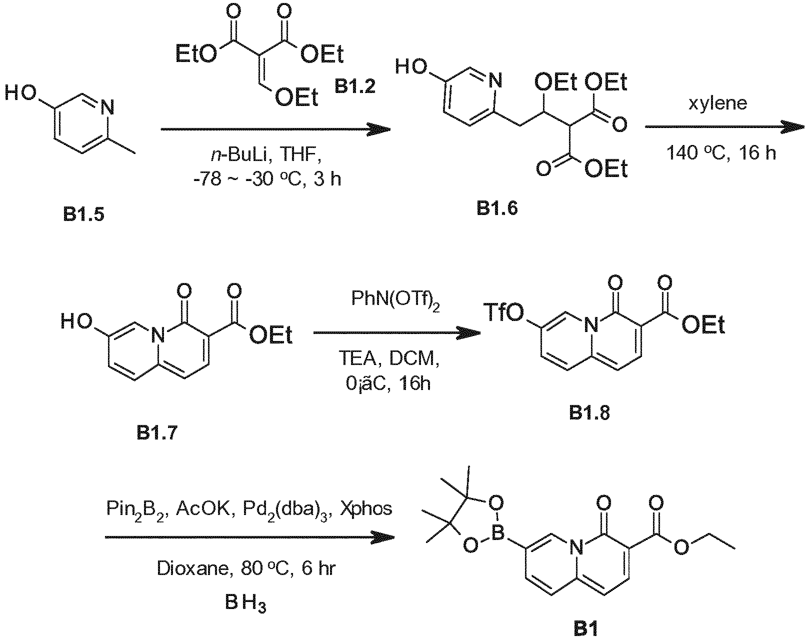

### Method A:

### Step (a) Preparation of diethyl 2-[2-(5-bromo-2-pyridyl)-1-ethoxy-ethyl]propanedioate (compound B1.3)

To a solution of lithium diisopropylamide (160 mL, 320 mmol, 2M in THF) stirred at -60 °C was added dropwise a solution of 5-bromo-2-methyl-pyridine (50 g, 290.66 mmol, compound B1.1) in THF (500 mL) over 15 min under N₂. The mixture was stirred at this temperature for 45 min before diethyl 2-(ethoxymethylene)propanedioate (65.99 g, 305.2 mmol, compound B1.2) was added slowly and the resulting mixture solution was stirred at -20 °C for another 2 h. The reaction mixture was then quenched with satd. aq. NH₄Cl solution (100 mL) and extracted with EtOAc (500 mL) twice. The combined organic layer was washed with brine (200 mL) twice, dried over anhydrous Na₂SO₄, filtered and concentrated *in vacuo* to give a crude product, which was purified by silica gel flash chromatography (EtOAc in petroleum ether from 5% to 50% gradient) to give diethyl 2-[2-(5-bromo-2-pyridyl)-1-ethoxy-ethyl]propanedioate (30 g, 26.58% yield) as yellow oil. MS (ESI): 390.2 ([{⁸¹Br}M+H]⁺), 388.1([{⁷⁹Br}M+H]⁺).

### Step (b) Preparation of ethyl 7-bromo-4-oxo-quinolizine-3-carboxylate (compound B1.4)

A solution of diethyl 2-[2-(5-bromo-2-pyridyl)-1-ethoxy-ethyl]propanedioate_ (26 g, 66.97 mmol, compound B1.3) in polyphosphoric acid/AcOH (100 mL, v/v=5/2) was stirred at 120 °C for 40 min. After the reaction was cooled back to r.t., the mixture was diluted with EtOAc (300 mL), washed with water (100 mL), brine (100 mL). The separated organic layer was then dried over anhydrous Na₂SO₄, filtered and concentrated *in vacuo* to give a crude product, which was triturated with a mixture of MeCN and petroleum ether (50 mL, 10:1, 50) to give ethyl 7-bromo-4-oxo-quinolizine-3-carboxylate (3.5 g, 17.1% yield) as a yellow solid. The mother liquid was further purified by silica gel flash chromatography (EtOAc in petroleum ether from 10% to 60% gradient) to give additional ethyl 7-bromo-4-oxo-quinolizine-3-carboxylate (0.5 g, 2.4% yield) as a yellow solid. MS (ESI): 298.1 ([{⁸¹Br}M+H]⁺), 296.1([{⁷⁹Br}M+H]⁺). ¹H NMR (400 MHz, CHLOROFORM-*d*) δ ppm: 9.50 (d, 1 H), 8.41 (d, 1 H), 7.61 (dd, 1 H), 7.45 (d, 1 H), 6.65 (d, 1 H), 4.43 (q, 2 H), 1.43 (t, 3 H).

### Step (c) Preparation of ethyl 4-oxo-7-(4,4,5,5-tetramethyl-1,3,2-dioxaborolan-2-yl)-4H-quinolizine-3-carboxylate (Intermediate B1)

To a solution of ethyl 7-bromo-4-oxo-quinolizine-3-carboxylate_(2.01 g, 6.79 mmol, compound B1.4) and Pin₂B₂ (6.95 g, 27.37 mmol) in dioxane (60.0 mL) was added AcOK (2.75 g, 28.02 mmol) and Pd(dppf)Cl₂ (148.0 mg, 0.202 mmol), and the resulting mixture was stirred at 80 °C for 6 hr under Argon. After the reaction was cooled back to r.t., the reaction mixture was diluted with water (200 mL) and extracted with DCM (100 mL) three times. The combined organic layer was dried over anhydrous Na₂SO₄, filtered and concentrated *in vacuo* to give a crude material, which was re-dissolved in DCM (100 mL) and filtered. The filtrate was concentrated under reduced pressure to give a crude product, which was triturated with a mixture of MTBE and petroleum ether (50 mL, 10:1) to give ethyl 4-oxo-7-(4,4,5,5-tetramethyl-1,3,2-dioxaborolan-2-yl)-4H-quinolizine-3-carboxylate (2.35 g, 70% yield) as a yellow solid. MS (ESI): 262.0 ([M-82+H]⁺). (corresponding boronic acid) ¹H NMR (400 MHz, DMSO-d⁶) *δ* ppm: 9.37 (s, 1 H), 8.30-8.28 (d, 1 H), 7.87-7.81 (m, 2 H), 6.87-6.85 (d, 1 H), 4.27-4.21 (q, 2 H), 1.34 (s,12 H), 1.30 (t, 3 H).

### Method B:

### Step (a) Preparation of diethyl 2-[1-ethoxy-2-(5-hydroxy-2-pyridyl)ethyl]propanedioate (compound B1.6)

To a stirred solution of 6-methylpyridin-3-ol compound B1.5 (10.0 g, 91.63 mmol, compound B1.5) in THF (100 mL)at -78 °C was added n-BuLi (80.64 mL, 201.59 mmol) slowly. After the addition, the reaction mixture was stirred at -30 °C for 2 h before it was cooled back to -78 °C followed by the addition of a solution of diethyl ethoxymethylenemalonate (21.8 g, 100.8 mmol) in THF (40 mL) dropwise. The resulting mixture was stirred at -30 °C for another 1 h before quenched by satd. aq. NH₄Cl solution (30 mL) and water (200 mL). The mixture was then extracted with DCM (100 mL) three times and the combined organic layer was washed with brine (50 mL), dried over anhydrous Na₂SO₄, filtered and concentrated *in vacuo* to give a crude product. It was then purified by silica gel flash chromatography (5% to 30% EtOAc in petroleum ether) to give diethyl 2-[1-ethoxy-2-(5-hydroxy-2-pyridyl)ethyl]propanedioate (15 g, 50.31% yield) as orange oil. MS (ESI): 326.3 ([M+H]⁺).

### Step (b) Preparation of ethyl 7-hydroxy-4-oxo-quinolizine-3-carboxylate (compound B1.7)

A suspension of diethyl 2-[1-ethoxy-2-(5-hydroxy-2-pyridyl)ethyl]propanedioate (15 g, 46.1 mmol, compound B1.6) in xylenes (80 mL, 46.1 mmol) was stirred at 140 °C for 16 h. After the reaction mixture was cooled down to temperature.t., the formed precipitate was collected by filtration. The filter cake was washed with MTBE (50 mL) and dried under vacuum to give ethyl 7-hydroxy-4-oxo-quinolizine-3-carboxylate (5.7 g, 50.79% yield) as a yellow power. MS (ESI): 234.2 ([M+H]⁺). ¹H NMR (400 MHz, DMSO-d⁶) *δ* ppm: 10.74 (s, 1 H), 8.81 (d, 1 H), 8.07 (d, 1 H), 7.89 (d, 1 H), 7.61 (dd, 1 H), 6.82 (d, 1 H), 4.23 (q, 2 H), 1.28 (t, 3 H).

### Step (c) Preparation of ethyl 4-oxo-7-(trifluoromethylsulfonyloxy)quinolizine-3-carboxylate (compound B1.8)

To a solution of ethyl 7-hydroxy-4-oxo-quinolizine-3-carboxylate_(4.0 g, 17.15 mmol, compound B1.7) in DCM (64 mL) was added triethylamine (3.47 g, 34.3 mmol) and *N,N-*bis(trifluoromethylsulfonyl)aniline (12.25 g, 34.3 mmol) slowly at 0 °C. The resuting mixture was stirred at 0 °C for 1 h before quenched by satd aq. NaHCO₃ solution (50 mL). The mixture was extracted with DCM (100 mL) twice and the combined organic layer was washed with brine (50 mL), dried over anhydrous Na₂SO₄, filtered, and concentrated *in vacuo* to give a crude product. It was purified by trituration with MTBE (15 mL) to give ethyl 4-oxo-7-(trifluoromethylsulfonyloxy)quinolizine-3-carboxylate (7.0 g, 94.97% yield) as a yellow solid. MS (ESI): 366.1 ([M+H]⁺).

### Step (d) Preparation of ethyl 4-oxo-7-(4,4,5,5-tetramethyl-1,3,2-dioxaborolan-2-yl)-4H-quinolizine- 3-carboxylate (Intermediate B1)

To a solution of ethyl 4-oxo-7-(trifluoromethylsulfonyloxy)quinolizine-3-carboxylate (6.5 g, 17.79 mmol, compound B1.8) in 1,4-dioxane (100 mL) was added Pin₂B₂ (13.0 g, 51.19 mmol), AcOK (6.5 g, 66.23 mmol), Pd₂(dba)₃ (650.0 mg, 0.710 mmol) and X-phos (650.0 mg, 1.36 mmol) under Argon and the resulting mixture was stirred at 70 °C for 6 h. After reaction was cooled back to r.t., the mixture was diluted with water (100 mL), and extracted with DCM three times. The combined organic layer was dried over anhydrous Na₂SO₄, filtered and concentrated under reduced pressure to dryness. The residue was re-suspended in DCM (100 mL) and filtered. The filtrate was concentrated *in vacuo* to give a crude material, which was triturated with a mixture of MTBE and petroleum ether (50 mL, 1:5) to give ethyl 4-oxo-7-(4,4,5,5-tetramethyl-1,3,2-dioxaborolan-2-yl) quinolizine-3-carboxylate (5.9 g, 17.19 mmol, 72.46% yield) as a yellow solid. MS (ESI): 262.0 ([M-82+H]⁺). (Boric acid) ¹H NMR (400 MHz, COCl3) *δ* ppm: 9.72 (s, 1 H), 8.39-8.37 (d, 1 H), 7.81-7.79 (d, 2 H), 7.50-7.47 (d, 1 H), 6.59-6.57 (d, 1 H), 4.42-4.37 (q, 2 H), 1.43-1.41 (t, 3 H), 1.37 (s,12 H).

### Intermediate B2

### Ethyl 9-methyl-4-oxo-7-(4,4,5,5-tetramethyl-1,3,2-dioxaborolan-2-yl)-4H-quinolizine-3-carboxylate

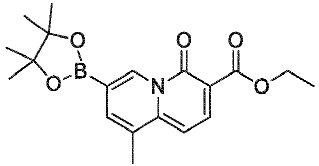

The titled compound was synthesized according to the following scheme:

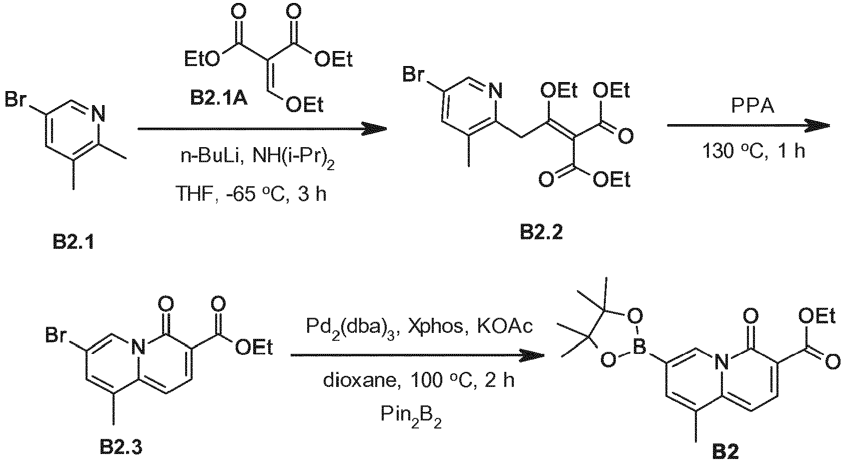

### Step (a) Preparation of diethyl 2-(2-(5-bromo-3-methylpyridin-2-yl)-1-ethoxyethylidene)malonate (compound B2.2)

To a solution of bis(isopropyl)amine (4.9 g, 0.049 mol) in anhydrous THF (30 mL) was added *n*-BuLi (21.6 mL, 2.0 M in THF) at -65 °C and the mixture was stirred for 1 h under argon. After that, a solution of 5-bromo-2,3-dimethyl-pyridine (5.0 g, 0.027 mol, compound B2.1) in anhydrous THF (10.0 mL) was added slowly and the resulting mixture was stirred for another 1 h under argon. Then, a solution of diethyl 2-(ethoxymethylene)propanedioate (8.8 g, 0.041 mol, compound B2.1A) in anhydrous THF (10.0 mL) was added slowly and the combined reaction mixture was stirred for another 1 h under argon. The reaction mixture was poured into aq. NH₄Cl solution (100 mL) and extracted by EtOAc (100 mL) three times. The combined organic layer was washed with brine, dried over anhy. Na₂SO₄, filtered, and concentrated *in vacuo* to give a crude product of diethyl 2-(2-(5-bromo-3-methylpyridin-2-yl)-1-ethoxyethylidene)malonate (6.5 g, 60.2% yield). MS (ESI): 402.0 ({⁷⁹Br} M+H)⁺, 404.0 ({⁸¹Br} M+H)⁺. It was used directly in the next step without further purification.

### Step (b) Preparation of ethyl 7-bromo-9-methyl-4-oxo-4H-quinolizine-3-carboxylate (compound B2.3)

To a solution of PPA (20 mL) was added diethyl 2-(2-(5-bromo-3-methylpyridin-2-yl)-1-ethoxyethylidene)malonate (6.5 g, 0.016 mol, compound B2.2) and the reaction mixture was stirred at 130 °C for 1 h. After cooled down to the r.t., the reaction mixture was poured into water (100 mL) and extracted by EtOAc (75 mL) three times. The organic layer was washed with brine, dried over anhy. Na₂SO₄, filtered, and concentrated *in vacuo* to give a crude product, which was purified by Prep-HPLC to give ethyl 7-bromo-9-methyl-4-oxo-4H-quinolizine-3-carboxylate (3.0 g, 59.6% yield) as a solid. MS (ESI) [(M)⁺]: 309.9 ({⁷⁹Br} M+H)⁺, 311.9 ({⁸¹Br} M+H)⁺.

### Step (c) Preparation of ethyl 9-methyl-4-oxo-7-(4,4,5,5-tetramethyl-1,3,2-dioxaborolan-2-yl)-4H-quinolizine-3-carboxylate (Intermediate B2)

A mixture solution of ethyl 7-bromo-9-methyl-4-oxo-4H-quinolizine-3-carboxylate_(100 mg, 0.32 mmol, compound B2.3), 4,4,4',4',5,5,5',5'-octamethyl- 2,2'-bi(1,3,2-dioxaborolane) (164 mg, 0.65 mmol), KOAc (94 mg, 0.96 mmol), X-phos (30 mg, 0.06 mmol) and Pd₂(dba)₃ (57 mg, 0.06 mmol) in dioxane (10 mL) was stirred at 100 °C for 2 h under N₂. After cooled back to r.t., the reaction mixture was diluted with EtOAc (50 mL) and filtered. The filtrate was concentrated *in vacuo* to give a crude product, which was purified by Prep-HPLC (0.5 % TFA as additive) to give ethyl 9-methyl-4-oxo-7-(4,4,5,5-tetramethyl-1,3,2-dioxaborolan-2-yl)-4H-quinolizine-3-carboxylate as a solid (75 mg, 85.2% yield). MS (ESI): 276.2 ([M-81]⁺).

### Intermediate B3

### Ethyl 4-oxo-7-(4,4,5,5-tetramethyl-1,3,2-dioxaborolan-2-yl)-4H-quinolizine-3-carboxylate

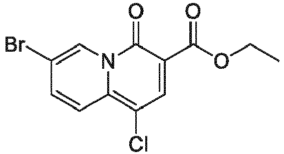

The titled compound was synthesized according to the following scheme:

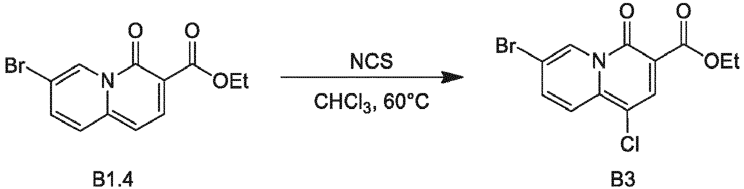

To a solution of ethyl 7-bromo-4-oxo-quinolizine-3-carboxylate_(200.0 mg, 0.680 mmol, compound B1.4) in chloroform (5 mL) was added N-chlorosuccinimide (120.0 mg, 0.810 mmol) and the resulting reaction mixture was stirred at 50 °C for 18 h. After the reaction was cooled down to r.t., the mixture was diluted with DCM (40 mL), washed with satd. aq. NaHCO₃ solution (40 mL) two times, brine (40 mL) two times. The organic layer was then dried over anhydrous Na₂SO₄, filtered, and concentrated in vacuo to give a crude product, which was triturated with a mixture of TBME and petroleum ether (20 mL, 10:1) to give ethyl 7-bromo-1-chloro-4-oxo-quinolizine-3-carboxylate (195 mg, 0.590 mmol, 87.34% yield) as a yellow solid. MS (ESI): 331.8 [{³⁵Cl+⁸¹Br}&{³⁷Cl+⁷⁹Br} (M+H)]⁺. ¹H NMR (400 MHz, CDCl₃) *δ*ppm: 9.56 (d, *J=* 2.0 Hz, 1H), 8.48 (s, 1H), 7.95-7.93 (d, *J* = 9.6 Hz, 1H), 7.80-7.77 (d, *J=* 9.6, 2.0 Hz, 1H), 4.50-4.39 (q, *J=* 6.8 Hz, 2H), 1.43-1.40 (t, *J=* 6.8 Hz, 3H).

### Intermediate B4

### Ethyl 7-bromo-9-fluoro-4-oxo-4H-quinolizine-3-carboxylate

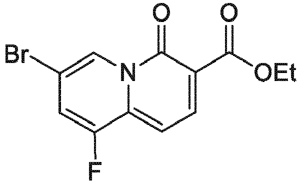

The titled compound was synthesized according to the following scheme:

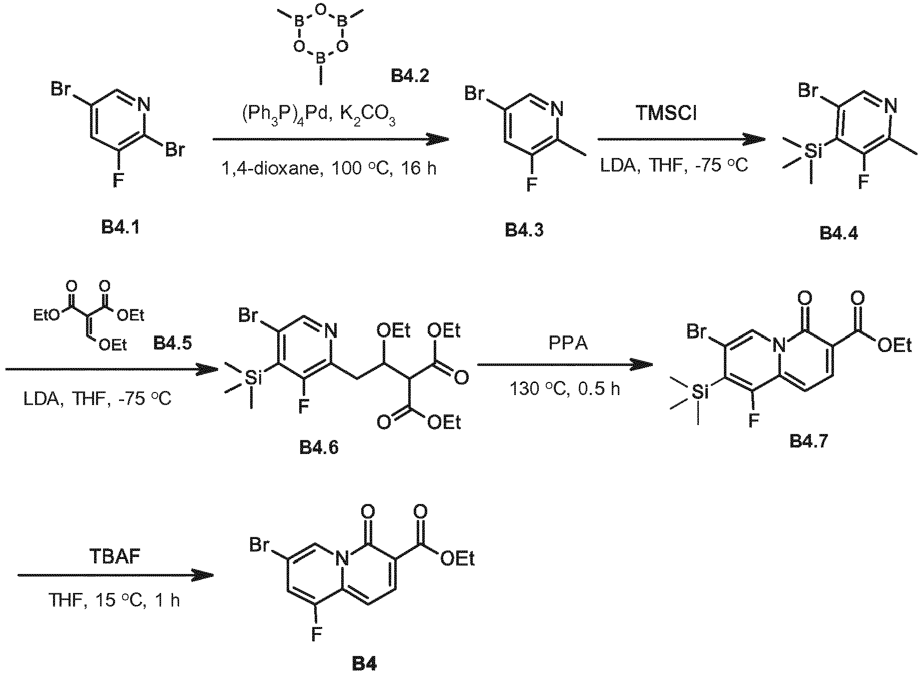

### Step (a) Preparation of 5-bromo-3-fluoro-2-methylpyridine hydrochloride (compound B4.3)

A mixture solution of 2,4,6-trimethyl-1,3,5,2,4,6-trioxatriborinane (8.87 g, 70.62 mmol, 2 eq), 2,5-dibromo-3-fluoropyridine (9.0 g, 35.31 mmol, 1 eq), Pd(Ph₃P)₄ (2.04 g, 1.77 mmol, 0.050 eq) andK₂CO₃ (14.64 g, 105.93 mmol, 3 eq) in 1,4-dioxane (90 mL) was stirred at 100 °C for 16 h under Ar. After it was cooled down to r.t., the reaction mixture was diluted with 40 mL EtOAc and filtered. The filtrate was washed with brine (20 mL), dried over anhy. Na₂SO₄, and filtered. 4 M HCl in EtOAc (20 mL) was added and the resulting solution was evaporated to dryness under reduced pressure. The residue was then treated with EtOAc (10 mL) and TBME (10 mL), and the precipitate was collected by vacuum filtration to give 5-bromo-3-fluoro-2-methylpyridine hydrochloride (4.8 g, 21.19 mmol, 33.61% yield) as a yellow solid. MS (ESI): 189.9 ([{⁷⁹Br}M+H]⁺), 191.9 ([{⁸¹Br} +H]⁺). ¹H NMR (400 MHz, DMSO-d6) δ ppm 8.47 (s, 1 H), 8.10 (dd, J=9.22, 1.82 Hz, 1 H), 2.42 (d, J=3.01 Hz, 3 H).

### Step (b) Preparation of 5-bromo-3-fluoro-2-methyl-4-(trimethylsilyl)pyridine (compound B4.4)

To a solution of bis(isopropyl)amine (0.5 mL, 3.53 mmol, 1.6 eq) in THF (2 mL) was added n-butyllithium in hexane (1.41 mL, 3.53 mmol) at -75 °C under N₂. The mixture solution was stirred at -75 °C for 0.5 h before it was added to a suspension of 5-bromo-3-fluoro-2-methylpyridine hydrochloride (500.0 mg, 2.21 mmol) in THF (6 mL) at -75 °C under N₂. After the mixture was stirred at - 75 °C for 0.5 h, trimethylchlorosilane (479.71 mg, 4.42 mmol) in THF (2 mL) was added by a syringe, and the resulting reaction mixture was stirred at - 75 °C for additional 1 h. After the reaction was warmed up to r.t., the mixture was poured into aq. NH₄Cl solution (50 mL) and extracted with EtOAc (50 mL) two times. The organic layer was dried over anhy. Na₂SO₄, filtered, and concentrated *in vacuo* to get a crude product, which was purified by Prep-HPLC (NH₃.H₂O as additive) to give 5-bromo-3-fluoro-2-methyl-4-(trimethylsilyl)pyridine (compound B4.4) (860 mg, 3.28 mmol, 49.5% yield) as colorless oil. MS (ESI): 262.0 ([{⁷⁹Br}M+H]+), 264.0 ([{⁸¹Br} +H]+). ¹H NMR (400 MHz, CHLOROFORM-d) δ ppm 8.36 (s, 1 H), 2.45 (d, J=3.42 Hz, 3 H), 0.48 (d, J=2.20 Hz, 9 H).

### Step (c) Preparation of diethyl 2-(2-(5-bromo-3-fluoro-4-(trimethylsilyl)pyridin-2-yl)-1-ethoxyethyl)malonate (compound B4.6)

To a solution of bis(isopropyl)amine (0.4 mL, 2.85 mmol) in THF (2 mL) was added n-butyllithium in hexane (1.14 mL, 2.85 mmol) at -75 °C under N₂. The resulting solution was stirred at - 75 °C for 0.5 h before a solution of 5-bromo-3-fluoro-2-methyl-4-(trimethylsilyl)pyridine (680.0 mg, 2.59 mmol) in THF (7 mL) was added and the mixture was stirred at -75 °C for another 0.5 h. Afterwards, diethyl 2-(ethoxymethylene)malonate (616.88 mg, 2.85 mmol) in THF (1 mL) was added dropwise, and the reaction mixture was stirred at - 75 °C for additional 1 h. After it was allowed to warm up to r.t., the reaction mixture was poured into aq. NH₄Cl solution (30 mL) and extracted with EtOAc (30 mL) two times. The organic layer was dried over anhy. Na₂SO₄, filtered, and concentrated in vacuo to give a crude product of diethyl 2-(2-(5-bromo-3-fluoro-4-(trimethylsilyl)pyridin-2-yl)-1-ethoxyethyl) malonate (1240 mg, 2.59 mmol, 81.95% yield) as yellow oil. MS (ESI) : 478.1 ([{⁷⁹Br}M+H]⁺), 490.1 ([{⁸¹Br} +H]+). ¹H NMR (400 MHz, CHLOROFORM-d) δ ppm 8.40 (s, 1 H), 4.40 - 4.47 (m, 1 H), 4.20 (quin, J=7.00 Hz, 4 H), 3.67 (d, J=8.56 Hz, 1 H), 3.39 - 3.54 (m, 2 H), 3.09 - 3.21 (m, 2 H), 1.27 (t, J=7.15 Hz, 6 H), 1.00 (t, J=6.97 Hz, 3 H), 0.47 (s, 9 H). It was used directly in the next step without further purification.

### Step (d) Preparation of ethyl 7-bromo-9-fluoro-4-oxo-8-(trimethylsilyl)-4H-quinolizine-3-carboxylate (compound B4.7)

A mixture solution of 2-(2-(5-bromo-3-fluoro-4-(trimethylsilyl)pyridin-2-yl)-1-ethoxyethyl)malonate (350 mg, 0.730 mmol), acetic acid (0.5 mL) and polyphosphoric acid (1 mL) was stirred at 130 °C for 0.75 h. After it was cooled back to r.t., the reaction mixture was treated with EtOAc (30 mL) and ice-cold water (30 mL). After extracting the aqueous phase with additional EtOAc (30 mL), the combined organic layer was dried over anhy. Na₂SO₄, filtered, and concentrated in vacuo to give a crude product of ethyl 7-bromo-9-fluoro-4-oxo-8-(trimethylsilyl)-4H- quinolizine-3-carboxylate as dark-brown oil (63.9% yield). MS (ESI): 386.0 ([{⁷⁹Br}M+H]⁺), 388.0 ([{⁸¹Br} +H]+).

### Step (e) Preparation of ethyl 7-bromo-9-fluoro-4-oxo-4H-quinolizine-3-carboxylate (Intermediate B4)

A solution of ethyl 7-bromo-9-fluoro-4-oxo-8-(trimethylsilyl)-4H-quinolizine- 3-carboxylate (950 mg, 2.46 mmol)and tetrabutylammonium fluoride(4.92 mL, 4.92 mmol, ? M in THF) in THF (10 mL) was stirred at 15 °C for 1 h. Afterwards, the reaction mixture was diluted with water (30 mL) and extracted with EtOAc (50 mL) two times. The combined organic layer was concentrated in vacuo to give a crude product, which was purified by Prep-HPLC (NH₃.H₂O as additive) to give ethyl 7-bromo-9-fluoro-4-oxo-4H-quinolizine-3-carboxylate (330 mg, 1.05 mmol, 34.18% yield) as a yellow solid. MS (ESI): 314.0 ([{⁷⁹Br}M+H]⁺), 316.0 ([{⁸¹Br} +H]+).

### Intermediate B5

### Ethyl 7-bromo-1-((dimethylamino)methyl)-4-oxo-4H-quinolizine-3-carboxylate

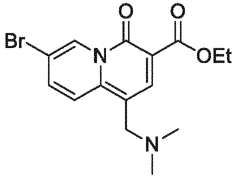

The titled compound was synthesized according to the following scheme:

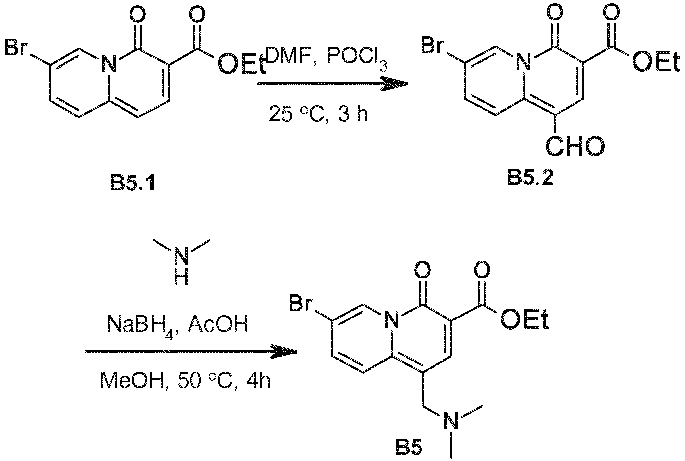

### Step (a) Preparation of ethyl 7-bromo-1-formyl-4-oxo-4H-quinolizine-3-carboxylate (compound B5.2)

POCl₃ (1.3 g, 8.45 mmol) was added to DMF (5.0 mL) at 0 °C and the mixture was stirred at 0 °C for 0.5 h before ethyl 7-bromo-4-oxo-4H- quinolizine-3-carboxylate (500.0 mg, 1.69 mmol) was added and the resulting reaction mixture was allowed to stir at 25 °C for 3 h. The reaction mixture was then poured into 1 N aq. Na₂CO₃ solution (100 mL) and extracted by EtOAc (50 mL) three times. The organic phase was washed with 1 N aq. CaCl₂ solution (50 mL) five times and brine (50 mL) three times before it was concentrated in vacuo to give a crude product, which was triturated in 50 mL MeOH to give ethyl 7-bromo-1-formyl-4-oxo-4H-quinolizine-3-carboxylate (400.0 mg, 73.1% yield) as a yellow solid. MS (ESI): 324.0 ([{⁷⁹Br}M+H]⁺), 326.0 ([{⁸¹Br}M+H]⁺).

### Step (b) Preparation of ethyl 7-bromo-1-((dimethylamino)methyl)-4-oxo-4H- quinolizine-3-carboxylate (Intermediate B5)

To a solution of ethyl 7-bromo-1-formyl-4-oxo-4H-quinolizine-3-carboxylate (150.0 mg, 0.460 mmol) in THF (15 mL) was added dimethylamine in THF (1.16 mL, 2.31 mmol) and AcOH (69.5 mg, 1.16 mmol). After it was stirred at 50 °C for 4 h, NaBH(OAc)₃ (294.2 mg, 1.39 mmol) was added and the resulting reaction mixture was further stirred at 15 °C for 12 h. It was then concentrated in vacuo to give a crude product, which was then purified by silica gel flash chromatography (0.5% FA as additive) to give ethyl 7-bromo-1-((dimethylamino)methyl)-4-oxo-4H-quinolizine-3-carboxylate (140 mg, 74.24% yield) as a yellow solid. MS (ESI): 353.0 ([{⁷⁹Br}M+H]⁺), 355.0 ([{⁸¹Br}M+H]⁺).

### Intermediate B 6

### Ethyl 7-bromo-1-methyl-4-oxo-quinolizine-3-carboxylate

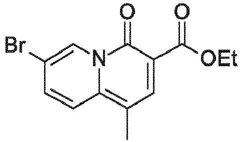

The titled compound was synthesized according to the following scheme:

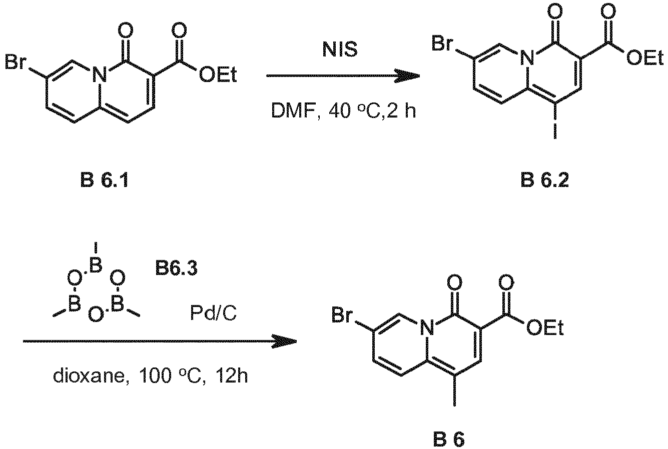

### Step (a) Preparation of ethyl -7-bromo-1-iodo-4-oxo-quinolizine-3-carboxylate (compound B 6.2)

To a solution of ethyl 7-bromo-4-oxo-4H-quinolizine-3-carboxylate (1.00 g, 3.38 mmol, compound B 6.1) in DMF (15 mL) was added N-iodosuccinimide (1.90 g, 8.44 mmol). The reaction mixture was stirred at 40 °C for 2 h before it was poured into 10% aq. Na₂SO₃ solution (150 mL) and stirred for additional 30 min.. The resulting yellow precipitate was collected by filtration and filter cake was washed by 5% of aq. Na₂SO₃ solution (20 mL) and water (20 mL). The filter cake was dried under vacuum to give ethyl 7-bromo-1- iodo-4-oxo-quinolizine-3-carboxylate (1200 mg, 2.84 mmol, 80.84% yield) as a dark brown solid. MS (ESI): 422.1 ([{⁷⁹Br}M+H]⁺), 424.1 ([{⁸¹Br}M+H]⁺).

### Step (b) Preparation of ethyl 7-bromo-1-methyl-4-oxo-quinolizine-3-carboxylate (Intermediate B 6)

To a mixture of ethyl 7-bromo-1-iodo-4-oxo-quinolizine-3-carboxylate (150.0 mg, 0.360 mmol, compound B 6.2), trimethylboroxine (178.47 mg, 1.42 mmol) and Na₂CO₃ (188.36 mg, 1.78 mmol) in 1,4-dioxane (5 mL) was added Pd/C (30.26 mg, 0.280 mmol, 0.800 eq) in glovebox under argon and the reaction mixture was stirred at 90 °C for 10 h. After it was cooled down to r.t., the reaction mixture was diluted by EtOAc (100 mL) and filtered. The filtrate was concentrated *in vacuo* to give a crude product, which was purified by Prep-HPLC (TFA as additive) to give ethyl 7-bromo-1-methyl-4-oxo- quinolizine-3-carboxylate (Intermediate B6) (50 mg, 0.160 mmol, 45.36% yield) as a yellow solid. MS (ESI): 310.1 ([{⁷⁹Br}M+H]⁺).

### Intermediate B7

### Ethyl 7-bromo-1-cyclopropyl-4-oxo-quinolizine-3-carboxylate

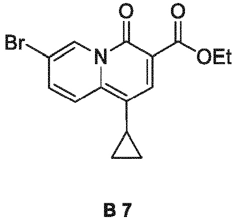

The title compound was prepared in analogy to the preparation of ethyl 7-bromo-1-methyl-4-oxo-quinolizine-3-carboxylate (Intermediate B6) by replacing_trimethylboroxine with cyclopropylboronic acid (326 mg, 3.79 mmol) to give ethyl 7-bromo-1-cyclopropyl-4-oxo-quinolizine-3-carboxylate (52 mg, 0.150 mmol, 31.36% yield) as a yellow solid after Prep-TLC (DCM:MeOH = 30:1) purification. MS (ESI): 338.1 ([{⁷⁹Br}M+H]⁺).

### Intermediate B8

### Ethyl 7-bromo-4-oxo-1-(trifluoromethyl)quinolizine-3-carboxylate

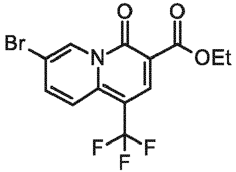

To a solution of 7-bromo-1-iodo-4-oxo-quinolizine-3-carboxylate (200 mg, 0.47 mmol, compound B6.2) and methyl 2,2-difluoro-2-(fluorosulfonyl)acetate (300 mg, 1.56 mmol, compound B8.1) in DMF (8 mL) was added CuI (271mg, 1.42 mmol) in glovebox under argon. The reaction mixture was stirred at 70 °C for 3 h before it was cooled back to r.t. and diluted with EtOAc(100 mL) and filtered. The filtrate was concentrated *in vacuo* to give a crude product, which was purified by Prep-TLC (DCM: MeOH= 30: 1) to give ethyl 7-bromo-4-oxo-1-(trifluoromethyl)quinolizine-3-carboxylate (110 mg, 0.30 mmol, 60.15% yield) as a yellow solid. MS (ESI): 364.1 ([{⁷⁹Br}M+H]⁺).

### Intermediate B9

### Ethyl 7-bromo-1-(morpholinomethyl)-4-oxo-4H-quinolizine-3-carboxylate

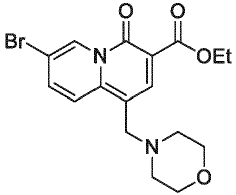

To a solution of ethyl 7-bromo-1-formyl-4-oxo-4H-quinolizine-3-carboxylate (150 mg, 0.46 mmol, compound B5.2) in THF (20 mL) was added morpholine (202 mg, 2.31 mmol). After the mixture was stirred at 70 °C for 12 h, NaBH(OAc)₃ (117.7 mg, 0.560 mmol) and AcOH (55.58 mg, 0.930 mmol) were added and the reaction mixture was stirred at 15 °C for another 12 h. The mixture solution was then concentrated in vacuo to give a crude product, which was purified by silica gel flash chromatography (0.5% FA as additive) to give ethyl 7-bromo-1-(morpholinomethyl)-4- oxo-4H-quinolizine-3-carboxylate (140 mg, 74.24% yield) as a yellow solid. MS (ESI): 395.1 ([{⁷⁹Br}M+H⁺), 397.1 ([{⁸¹Br}M+H]⁺).

### Intermediate B10

### Ethyl 4-oxo-1-(pyrrolidin-1-ylmethyl)-7-(((trifluoromethyl)sulfonyl)oxy)-4H-quinolizine-3-carboxylate

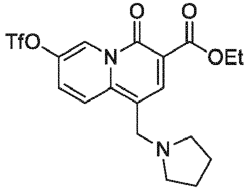

The titled compound was synthesized according to the following scheme:

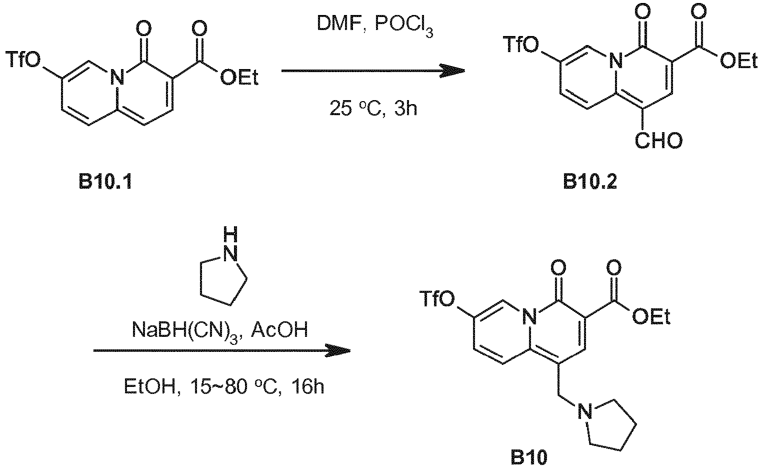

### Step (a) Preparation of ethyl 1-formyl-4-oxo-7-(((trifluoromethyl)sulfonyl)oxy)-4H-quinolizine-3-carboxylate (compound B10.2)

POCl₃ (0.51 mL, 5.48 mmol) was added to DMF (30.0 mL) at 0 °C and the mixture was stirred at 0 °C for 0.5 h before ethyl 4-oxo-7-(((trifluoromethyl)sulfonyl)oxy)-4H-quinolizine-3-carboxylate (400.0 mg, 1.1 mmol) was added. The reaction mixture was allowed to warm up and stirred at 40 °C for 12 h. After it was cooled down to r.t., the reaction mixture was poured into 1 N aq. Na₂CO₃solution (100 mL) and extracted by EtOAc (50 mL) three times. The combined organic phase was then washed with 1 N aq. CaCl₂ solution (50 mL) five times and brine (50 mL) three times, and concentrated in vacuo to give a crude product, which was triturated in 50 mL MeOH to give ethyl 1-formyl-4-oxo-7-(((trifluoromethyl)sulfonyl) oxy)-4H-quinolizine-3-carboxylate (400.0 mg, 74.3% yield) as a yellow solid. MS (ESI): 393.9 ([M+H]⁺).

### Step (b) Preparation of ethyl 4-oxo-1-(pyrrolidin-1-ylmethyl)-7-(((trifluoromethyl) sulfonyl)oxy)-4H-quinolizine-3 -carboxylate (Intermediate B10)

To a solution of ethyl 1-formyl-4-oxo-7-(((trifluoromethyl)sulfonyl)oxy)-4H- quinolizine-3-carboxylate (50.0 mg, 0.13 mmol) and pyrrolidine in ethanol (5 mL) was added acetic acid (0.2 mL, 3.5 mmol) and the mixture was stirred at 50 °C for 4 h under N₂. After it was cooled to r.t., NaBH₃CN (47.93 mg, 0.760 mmol, 6 eq) was added and the resulting mixture was stirred at 15 ° C for another 12 h. The mixture was concentrated in vacuo to give a crude product, which was purified by silica gel flash chromatography (FA as additive) to ethyl 4-oxo-1-(pyrrolidin-1-ylmethyl)-7-(((trifluoromethyl)sulfonyl)oxy)-4H-quinolizine-3-carboxylate (47 mg, 74.41% yield) as a yellow solid. MS (ESI): 449.1 (M+H]⁺).

### Intermediate C1

### (4R)-cis-N,N-dimethyl-1,2,3,3a,4,5,6,6a-octahydrocyclopenta[c]pyrrol-4-amine

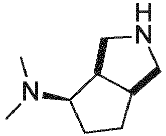

The titled compound was synthesized according to the following scheme:

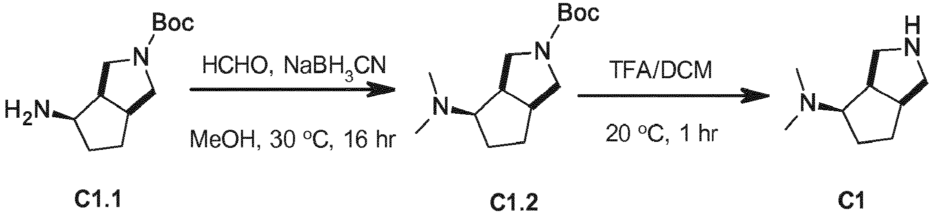

### Step (a) Preparation of tert-butyl-(4R)-cis-(dimethylamino)-3,3a,4,5,6,6a-hexahydro-1H-cyclopenta[c]pyrrole-2-carboxylate (compound C 1.2)

To a mixture of tert-butyl rac-(3aR,4R,6aS)-4-amino-3,3a,4,5,6,6a-hexahydro-1H-cyclopenta[c]pyrrole-2-carboxylate (900 mg, 3.98 mmol, compound C1.1) and formaldehyde (900 mg, 8.99 mmol) in methanol (10 mL) was added NaBH₃CN (500 mg, 7.95 mmol) and the mixture was stirred at 30 °C for 16 hr. Afterwards, the reaction was concentrated in vacuo to give a crude product, which was then purified by Prep-HPLC to give *cis-tert-*butyl*-*(4*R*)*-cis-*(dimethylamino)-3,3a,4,5,6,6a-hexahydro- 1H-cyclopenta[c]pyrrole-2-carboxylate (650 mg, 64.26% yield) as colorless oil. MS (ESI): 255.3 [(M⁺H)⁺].

### Step (b) Preparation of (4R)- cis-N,N-dimethyl-1,2,3,3a,4,5,6,6a-octahydrocyclopenta[c]pyrrol-4-amine (Intermediate C1)

To a solution of tert-butyl-(4*R*)-*cis*-(dimethylamino)-3,3a,4,5,6,6a-hexahydro-1H-cyclopentarclpyrrole-2-carboxylate (200 mg, 0.79 mmol, compound C1.2) in DCM (3 mL) was added TFA (0.33 mL, 4.33 mmol), and the mixture was stirred at 25 °C for 1 hr. The reaction was then concentrated in vacuo to give a crude product of (4R)- cis-N,N-dimethyl-1,2,3,3a,4,5,6,6a-octahydrocyclopenta[c] pyrrol-4-amine (121.28 mg, 100% yield) as colorless oil. It was used directly in the next step without further purification.

### Intermediate C2

### cis-N,N-dimethyl-1,2,3,3a,4,5,6,6a-octahydrocyclopenta[c]pyrrol-4-amine

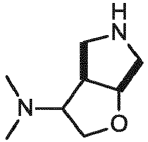

The titled compound was synthesized according to the following scheme:

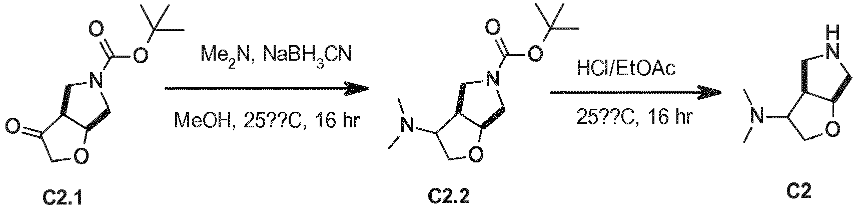

### Step (a) Preparation of cis-tert-butyl 3-(dimethylamino)-2,3,3a,4,6,6a-hexahydrofuro[2,3-c] pyrrole-5-carboxylate (compound C2.2)

To a solution of tert-butyl rac-(3aS,6aS)-3-oxo-3a,4,6,6a-tetrahydrofuro[2,3-c]pyrrole-5-carboxylate (700 mg, 3.08 mmol, compound C2.1) in methanol (10 mL) was added dimethylamine (7.7 mL, 15.4 mmol, 2.0 M solution in THF). After the mixture was stirred at 25 °C for 1 h, NaBH₃CN (581 mg, 9.25 mmol) was added slowly and the resulting mixture was stirred for another 16 h under N₂ atmosphere. The mixture was then diluted with EtOAc (80 mL) and the organic layer was washed with water (30 mL), brine (30 mL), dried over anhydrous Na₂SO₄, filtered, and concentrated in vacuo to give a crude product of *cis*-*tert*-butyl 3-(dimethylamino)-2,3,3a,4,6,6a-hexahydrofuro[2,3-c]pyrrole-5- carboxylate (600 mg, 75.99% yield) as colorless oil. MS (ESI): 257.2 [(M+H)+]. It was used directly in the next step without further purification.

### Step (b) Preparation of cis-N,N-dimethyl-3,3a,4,5,6,6a-hexahydro-2H-furo[2,3-c]pyrrol-3-amine hydrochloride (Intermediate C2)

A solution of *cis*-*tert*-butyl 3-(dimethylamino)-2,3,3a,4,6,6a-hexahydrofuro[2,3-c] pyrrole-5-carboxylate (600 mg, 2.34 mmol, compound C2.2) in HCl/EtOAc (10 mL, 4 M) was stirred at 25 °C for 16 h before it was concentrated in vacuo to give a crude product of cis-N,N-dimethyl-3,3a,4,5,6,6a-hexahydro-2H-furo[2,3-c]pyrrol-3-amine hydrochloride (300 mg, 66.52% yield) as a white solid. MS (ESI): 157.2 [(M+H)+]. It was used directly in the next step without further purification.

### Intermediate C3

### (4S)-cis-N,N-dimethyl-1,2,3,3a,4,5,6,6a-octahydrocyclopenta[c]pyrrol-4-amine

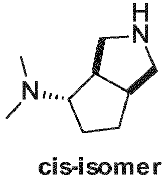

The title compound was prepared in analogy to Intermediate C1 by replacing (4*R*)*-cis-tert-*butyl 4-aminohexahydrocyclopenta[c]pyrrole-2(1H)-carboxylate (compound C1.1) with (4*S*)*-cis-tert*-butyl 4-aminohexahydrocyclopenta[c]pyrrole-2(1H)-carboxylate.

### Intermediate C4

### 3a,5-Dimethyloctahydropyrrolo [3,4-b] pyrrole

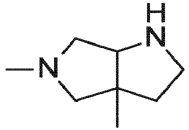

The titled compound was synthesized according to the following scheme:

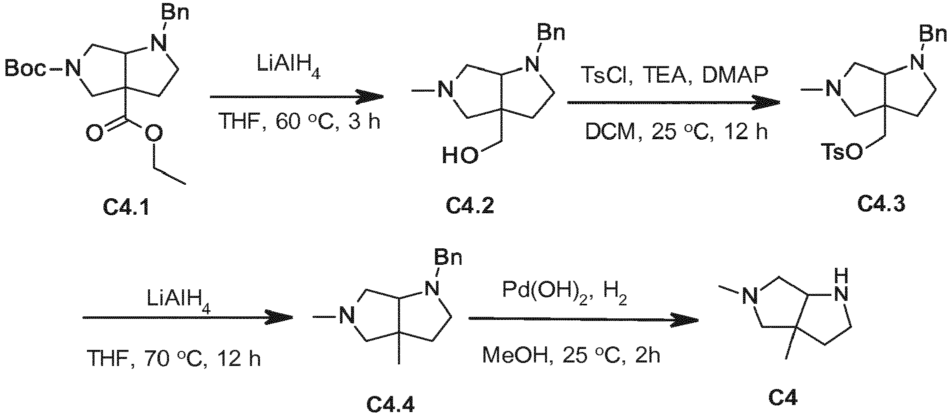

### Step (a) Preparation of (1-benzyl-5-methyloctahydropyrrolo[3,4-b]pyrrol-3a-yl) methanol (compound C4.2)

To a solution of 5-tert-butyl 3a-ethyl 1-benzylhexahydropyrrolo[3,4-b]pyrrole-3a,5(1H)-dicarboxylate (800 mg, 2.14 mmol) in THF (2.0 mL) was added LiAlH₄ (815 mg, 21.4 mmol) and the resulting mixture was stirred at 60 °C for 3 h. After it was cooled down to 0 °C he reaction mixture was poured into EtOAc (10 mL) followed by the addition of MgSO₄. After the mixture was stirred for additional 1 h, it was filtered and the filtrate was concentrated in vacuo to give a crude product of (1-benzyl-5-methyloctahydropyrrolo[3,4-b]pyrrol-3a-yl)methanol (450 mg, crude) as colourless oil. MS (ESI):247.1 (M+H)⁺. It was used directly in the next step without further purification.

### Step (b) Preparation of (1-benzyl-5-methyloctahydropyrrolo[3,4-b]pyrrol-3a-yl) methyl4-methylbenzenesulfonate (compound C4.3)

To a mixture solution of (1-benzyl-5-methyloctahydropyrrolo[3,4-b]pyrrol-3a-yl)methanol (700.0 mg, 2.84 mmol, compound C4.2), TsCl (650.0 mg, 3.41 mmol) and TEA (862.2 mg, 8.52 mmol) in DCM (5.0 mL) was added DMAP (69.0 mg, 0.568 mmol) and the resulting mixture was for 3 h at 28 °C for 3 h. The reaction mixture was then diluted with EtOAc (50.0 mL) and filtered. The filtrate was concentrated *in vacuo* to give a crude product of (1-benzyl-5-methyloctahydropyrrolo[3,4-b] pyrrol-3a-yl)methyl 4-methylbenzenesulfonate (900.0 mg, 79.08% yield) as yellow oil. MS (ESI): 401.1 (M+H)⁺. It was used directly in the next step without further purification.

### Step (c) Preparation of 1-benzyl-3a,5-dimethyloctahydropyrrolo[3,4-b]pyrrole (compound C4.4)

To a solution of (1-benzyl-5-methyloctahydropyrrolo[3,4-b]pyrrol-3a-yl)methyl 4-methylbenzenesulfonate (900.0 mg, 2.25 mmol, compound C4.3) in THF (15.0 mL) was added LiAlH₄ (853.0 mg, 22.5 mmol) slowly at 0 °C. After the addition, the mixture was allowed to warm up and stirred at 70 °C for 12 h. Afterwards, the reaction mixture was diluted with THF (300.0 mL) and added slowly and successively H₂O (0.85 mL), 10% aq. NaOH solution (1.70 mL) and H₂O (0.85 mL) under ice-water bath. After additional EtOAc (300 mL) was added, the mixture solution was then filtered. The filtrate was dried over anhy. Na₂SO₄, filtered, and concentrated in vacuo to give a crude product, which was purified by silica gel flash chromatography (petroleum ether: EtOAc = 10:1∼1:2, 1% NH₃•H₂O as additive) to give 1-benzyl-3a,5-dimethyloctahydropyrrolo[3,4-b]pyrrole (400.0 mg, 77.28% yield) as yellow oil. MS (ESI): 231.0 (M+H)⁺.

### Step (d) Preparation of 3a,5-dimethyloctahydropyrrolo[3,4-b]pyrrole (Intermediate C4)

To a solution of 1-benzyl-3a,5-dimethyloctahydropyrrolo[3,4-b]pyrrole (400.0 mg, 0.521 mmol, compound C4.4) in EtOH (5.0 mL) was added Pd(OH)₂/C (400.0 mg, 50% in H₂O). After the suspension was degassed under vacuum and purged with H₂ several times, the reaction mixture was stirred at 60 °C for 2 h under H₂ atmosphere (20 psi). After it was cooled back to r.t., the reaction mixture was filtered through celite and the filtrate was concentrated in vacuo to give a crude product of 3a,5-dimethyloctahydropyrrolo[3,4-b]pyrrole (200.0 mg, 82.13% yield) as pale yellow oil. MS (ESI): 141.0 ([M+H]⁺). It was used directly in the next step without further purification.

### Intermediate C5

### cis-1-Methyloctahydropyrrolo[3,4-b]pyrrole

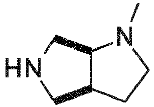

The titled compound was synthesized according to the following scheme:

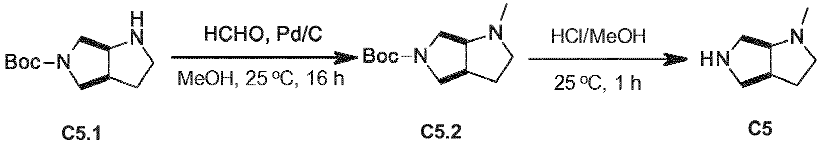

### Step (a) Preparation of tert-butyl cis-1-methyl-2,3,3a,4,6,6a-hexahydropyrrolo[3,4-b]pyrrole-5-carboxylate (compound CS.2)

To a solution of Pd/C (50.0 mg, 0.240 mmol, 0.100 eq) in methanol (0.500 mL) was added *cis-tert*-butyl (2,3,3a,4,6,6a-hexahydro-1H-pyrrolo[3,4-b]pyrrole-5-carboxylate (500.0 mg, 2.36 mmol) and HCHO in MeOH (706.58 mg, 23.55 mmol) under N₂. The suspension was degassed under vacuum and purged with H₂ three times before it was stirred at 25 °C for 16 h under a _{H2} balloon. Afterwards, the reaction mixture was filtered through celite and the filtrate was concentrated in vacuo to give a crude product of *tert-*butyl *cis*-1-methyl-2,3,3a,4,6,6a-hexahydropyrrolo[3,4-b]pyrrole-5-carboxylate (500 mg, 2.21 mmol, 93.8% yield) as colorless oil. It was used directly in the next step without further purification.

### Step (b) Preparation of cis-1-methyloctahydropyrrolo[3,4-b]pyrrole (Intermediate CS)

A mixture of *tert*-butyl cis-1-methyl-2,3,3a,4,6,6a-hexahydropyrrolo [3,4-b]pyrrole-5-carboxylate (500.0 mg, 2.21 mmol, compound C5.2) in HCl/MeOH (20.0 mL, 80 mmol, 2 M) was stirred at 25 °C for 1 h. Afterwards, the mixture was concentrated in vacuo to give a crude product of 1-methyl-3,3a,4,5,6,6a-hexahydro-2H-pyrrolo [3,4-b]pyrrole (300 mg, 2.38 mmol, 107.6% yield) as yellow oil. It was used directly in the next step without further purification.

### Intermediate C6

### (1R,5S,6S)-N,N-dimethyl-3-azabicyclo[3.1.0]hexan-6-amine hydrochloride

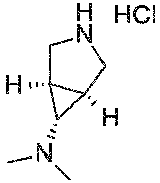

The titled compound was synthesized according to the following scheme:

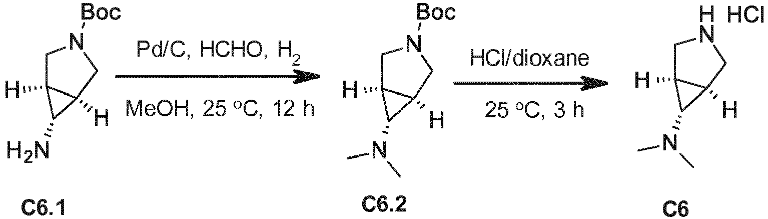

### Step (a) Preparation of (1R,5S,6S)-tert-butyl 6-(dimethylamino)-3-azabicyclo[3.1.0]hexane-3-carboxylate (compound C6.2)

To a solution of (1*R*,5*S*,6*S*)-*tert*-butyl 6-amino-3-azabicyclo[3.1.0]hexane-3- carboxylate (400.0 mg, 2.017 mmol) in anhydrous EtOH (10.0 mL) was added HCOH/EtOH (1.64 g, 20.17 mmol, 37% in H₂O) and 10% Pd/C (431.0 mg, 0.403 mmol, 50% H₂O). The suspension was degassed under vacuum and purged with H₂ several times before it was stirred at 25 °C for 12 h under H₂ atmosphere (20 psi). The mixture was then filtered and the filtrate was concentrated *in vacuo* to give a crude product of (1*R*,5*S*,6*S*)-*tert*-butyl 6-(dimethylamino)-3-azabicyclo[3.1.0]hexane-3-carboxylate (450.0 mg, 98.2% yield) as a white solid. MS (ESI): 227.1 ([M+H]⁺). It was used directly in the next step without further purification.

### Step (b) Preparation of (1R,5S,6S)-N,N-dimethyl-3-azabicyclo[3.1.0]hexan-6-amine hydrochloride (Intermediate C6)

A solution of (1*R*,5*S*,6*S*)-*tert*-butyl 6-(dimethylamino)-3-azabicyclo[3.1.0] hexane-3-carboxylate (400.0 mg, 1.77 mmol, compound C6.2) in HCl/dioxane (2 mL, 4 M) was stirred at 25 °C for 3 h. Afterwards, the mixture was then concentrated in vacuo to give a crude product of (1*R*,5*S*,6*S*)-N,N-dimethyl-3-azabicyclo[3.1.0]hexan-6-amine hydrochloride (400.0 mg) as brown oil. MS (ESI): 127.1 ([M+H]⁺). It was used directly in the next step without further purification.

### Intermediate C7

### cis-1-Methyl-2,3,3a,4,5,6,7,7a-octahydropyrrolo[2,3-c] pyridine hydrochloride

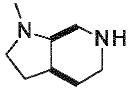

The title compound was prepared in analogy to Intermediate C5 by replacing *cis-tert-butyl* (2,3,3a,4,6,6a-hexahydro-1H-pyrrolo[3,4-b]pyrrole-5-carboxylate (compound C5.1) with *cis-*tert-butyl hexahydro-1H-pyrrolo[2,3-c]pyridine-6(2H)- carboxylate.

### Intermediate C8

### (S)-N,N-dimethyl-1-(morpholin-2-yl)methanamine hydrochloride

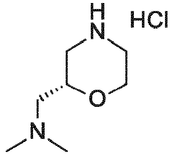

The titled compound was synthesized according to the following scheme:

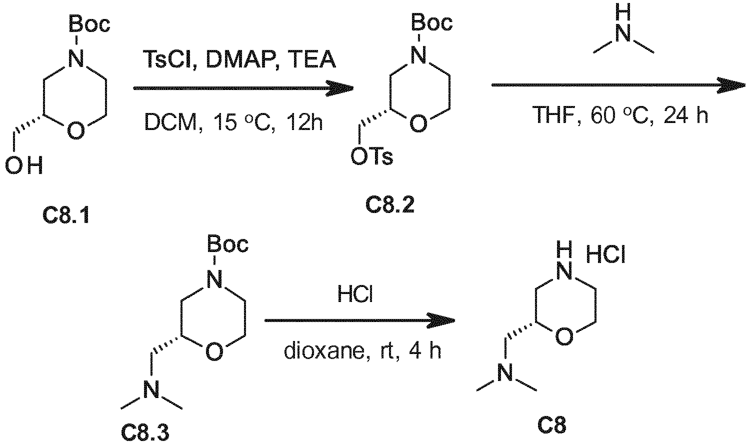

### Step (a) Preparation of (S)-tert-butyl 2-((tosyloxy)methyl)morpholine-4-carboxylate (compound C8.2)

To a solution of (S)-tert-butyl 2-(hydroxymethyl)morpholine-4-carboxylate (1.0 g, 4.6 mmol, compound C8.1) in DCM (5 mL) was added p-toluenesulfonyl chloride (0.97 g, 5.06 mmol), triethylamine (0.64 mL, 4.6 mmol) and 4-dimethylaminopyridine (56.23 mg, 0.460 mmol) and the resulting reaction mixture was stirred at 25 °C for 16 h. The mixture was diluted with 20 mL EtOAc and filtered, the filtrate was then concentrated in vacuo to give a crude product of (*S*)-*tert*-butyl 2-((tosyloxy)methyl)morpholine-4-carboxylate (2 g, 5.38 mmol, 95.92% yield). MS (ESI) : 394.2 ([M+Na]⁺). It was used directly in the next step without further purification.

### Step (b) Preparation of (R)-tert-butyl 2-((dimethylamino)methyl)morpholine-4-carboxylate (compound C8.3)

A mixture solution of (*S*)-*tert*-butyl 2-((tosyloxy)methyl)morpholine-4-carboxylate (1.9 g, 5.12 mmol, 1 eq) and dimethylamine/THF (20.46 mL, 40.92 mmol) was stirred at 60 °C for 24 h. After it was cooled back to r.t., the reaction mixture was concentrated in vacuo to give a crude product, which was purified by silica gel flash chromatography to give (*R*)-*tert*-butyl 2-((dimethylamino)methyl) morpholine-4-carboxylate (1 g, 4.09 mmol, 80.01% yield) as yellow oil. MS (ESI): 245.1 ([M+H]⁺). ¹H NMR (400 MHz, CHLOROFORM-d) δ ppm 3.73 - 4.00 (m, 3 H), 3.47 - 3.59 (m, 2 H), 2.45 - 2.64 (m, 3 H), 2.19 - 2.32 (m, 7 H), 1.47 (s, 9 H).

### Step (c) Preparation of (S)-N,N-dimethyl-1-(morpholin-2-yl)methanamine hydrochloride (Intermediate C8)

A solution of (R)-*tert*-butyl 2-((dimethylamino)methyl) morpholine-4-carboxylate (1.0 g, 4.09 mmol) in HCl/1,4-dioxane (5.0 mL, 20 mmol, 4 M) was stirred at 25 °C for 4 h. Afterwards, the mixture was diluted with 20 mL EtOAc and filtered. The filter cake was dried under vacuum to give a crude product of (*S*)-N,N-dimethyl- 1-(morpholin-2-yl)methanamine hydrochloride (620 mg, 3.43 mmol, 83.84% yield) as a white solid. It was used directly in the next step without further purification.

### Intermediate C9

### (R)-N,N-dimethyl-1-(morpholin-2-yl)methanamine hydrochloride

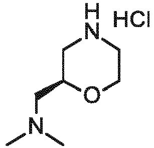

The title compound was prepared in analogy to Intermediate C8 by replacing (S)-tert-butyl 2-(hydroxymethyl)morpholine-4-carboxylate (compound C8.1) with (*R*)-tert-butyl 2-(hydroxymethyl)morpholine-4-carboxylate.

### Example 1.01

### 7-(4-(Dimethylamino)-5,6-difluoro-8-(methylamino)-9H-pyrido[2,3-b]indol-3-yl)-4-oxo-4H-quinolizine-3-carboxylic acid

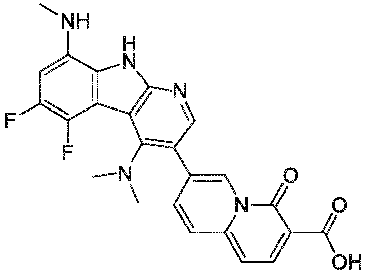

### Step (a) Preparation of tert-butyl N-[3-chloro-4-(dimethylamino)-5,6-difluoro-9H-pyrido[2,3-b]indol-8-yl]-N-methyl-carbamate

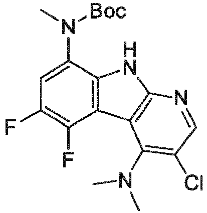

To a solution of *tert*-Butyl N-(3,4-dichloro-5,6-difluoro-9H-pyrido[2,3-b]indol-8-yl)-N-methyl-carbamate (**Intermediate A2**, 2.80 g, 6.96 mmol, as the "**CORE**" in table 1) and DIPEA (11.69 g, 90.48 mmol) in DMSO (6.0 mL) was added N-methylmethanamine hydrochloride (5.60 g, 69.60 mmol, as the "**AMINE**" in table 1), the reaction solution was stirred at 100 °C for 18 h. After it was cooled back to r.t., the mixture was poured into water (150 mL) and extracted with EtOAc (100 mL) two times. The combined organic layer was washed with brine (100 mL) three times, dried over anhy. Na₂SO₄, filtered, and concentrated in vacuo to give a crude product, which was purified by silica gel flash chromatography (5% to 25% EtOAc in petroleum ether) to give *tert*-butyl N-[3-chloro-4-(dimethylamino)-5,6-difluoro- 9H-pyrido[2,3-b]indol-8-yl]-N-methyl-carbamate (2.30 g, 88% yield) as a yellow solid. MS (ESI): 411.0 ([{³⁵Cl}M+H]⁺), 413.0 ([{³⁷Cl}M+H]⁺). ¹H NMR (400 MHz, DMSO-d₆) *δ* ppm: 12.35 (s, 1H), 8.36 (s, 1H), 7.57-7.52 (m, 1H), 3.19 (s, 3H), 3.05 (s, 6H), 1.49 (s, 3H), 1.42 (s, 6H).

### Step (b) Preparation of ethyl 7-(8-((tert-butoxycarbonyl)(methyl)amino)-4-(dimethylamino)-5,6-difluoro-9H-pyrido[2,3-b]indol-3-yl)-4-oxo-4H-quinolizine-3-carboxylate

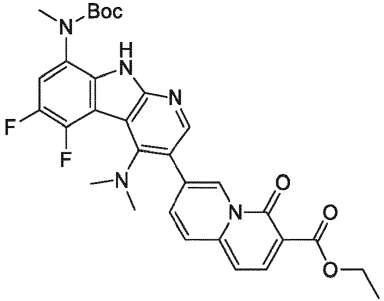

A mixture solution of *tert*-butyl N-[3-chloro-4-(dimethylamino)-5,6-difluoro-9H-pyrido [2,3-b]indol-8-yl]-N-methyl-carbamate (250.0 mg, 0.610 mmol), ethyl 4-oxo-7-(4,4,5,5-tetramethyl-1,3,2-dioxaborolan-2-yl)quinolizine-3-carboxylate (313 mg, 0.730 mmol) (**Intermediate B1,** 1.80 g, 3.80 mmol, as the **"BORONIC REAGENT"** in table 1), K₃PO₄ (388 mg, 1.83 mmol) and Pd-Ad₂nBuP Biphenyl Precat (40 mg, 0.060 mmol, CAS#:1310584-14-5, as the **"CATALYST"** in table 1) in THF/H₂O (5.5 mL, v/v=10:1 as the **"SOLVENT"** in table 1) was stirred at 60 °C under argon for 40 h. After it was cooled back to r.t., the reaction mixture was diluted with EtOAc (50 mL), and washed with brine (30 mL) two times. The combined organic layer was dried over anhydrous Na₂SO₄, filtered, and concentrated in vacuo to give a crude product, which was purified by Prep-TLC (DCM:MeOH:MeCN=10:1:1) to give ethyl 7-(8-((*tert*-butoxycarbonyl)(methyl)amino)-4-(dimethylamino)-5,6-difluoro-9H-pyrido[2,3-b]indol-3-yl)-4-oxo-4H-quinolizine-3-carboxylate (200.0 mg, 55.56% yield) as a yellow solid. MS (ESI): 592.4 ([M+H]⁺).

### Step (c) Preparation of 7-(8-((tert-butoxycarbonyl)(methyl)amino)-4-(dimethylamino)-5,6-difluoro-9H-pyrido[2,3-b]indol-3-yl)-4-oxo-4H-quinolizine-3-carboxylic acid

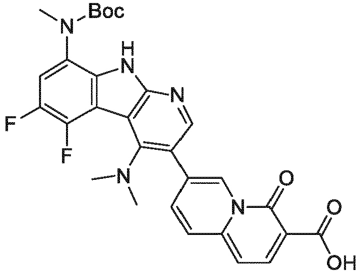

To a solution of ethyl 7-(8-((*tert*-butoxycarbonyl)(methyl)amino)-4- (dimethylamino)-5,6-difluoro-9H-pyrido[2,3-b]indol-3-yl)-4-oxo-4H-quinolizine-3-carboxylate (200.0 mg, 0.340 mmol) in ethanol (5 mL) was added aq. NaOH solution (1.0 Ml, 1 N) and the mixture was stirred at 15 °C for 16 hr. Afterwards, the mixture was concentrated *in vacuo* and the residual was acidified with 1 N HCl to pH= 3∼6 and extracted with EtOAc (30 mL) three times. The combined organic layer was dried over anhydrous Na₂SO₄, filtered, and concentrated in vacuo to give a crude product of 7-(8-((*tert*-butoxycarbonyl)(methyl)amino)-4-(dimethylamino)-5,6-difluoro-9H-pyrido[2,3-b]indol-3-yl)-4-oxo-4H-quinolizine-3-carboxylic acid (150 mg, 78.73% yield) as a yellow solid. MS (ESI): 564.2 ([M+H]⁺). It was used directly in the next step without further purification.

### Step (d) Preparation of 7-(4-(dimethylamino)-5,6-difluoro-8-(methylamino)-9H-pryido[2,3-b] indol-3-yl)-4-oxo-4H-quinolizine-3-carboxylic acid

To a solution of 7-[8-[*tert*-butoxycarbonyl(methyl)amino]-4-(dimethylamino)- 5,6-difluoro-9H-pyrido[2,3-b]indol-3-yl]-4-oxo-quinolizine-3-carboxylic acid (150.0 mg, 0.270 mmol) in DCM (5 mL) was added trifluoroacetic acid (1.0 mL, 12.98 mmol). After the reaction mixture was stirred at 15 °C for 1 hr, the solution was concentrated in vacuo to give a crude product, which was purified by RP-18 (water-CAN, 0.5% NH₃•H₂O as additive) to give 7-(4-(dimethylamino)-5,6-difluoro-8-(methylamino)-9H-pyrido[2,3-b]indol-3-yl)-4-oxo-4H-quinolizine-3-carboxylic acid (52.4 mg, 41.33% yield) as a yellow solid. MS (ESI): 464.3 ([M+H]⁺). ¹H NMR (400 MHz, DMSO-d₆) *δ* ppm: 12.27 (s, 1H), 9.18 (s, 1H), 8.46 (d, 1H), 8.34-8.19 (m, 2H), 8.03 (d, 1H), 7.37 (d, 1H), 6.59 (m, 1H), 5.67 (s, 1H), 2.90 (d, 3H), 2.81 (s, 6H). ¹⁹F NMR (400 MHz, DMSO-d₆) *δ* ppm: -148.94 (d, 1F), -154.94 (d, 1F).

The following examples were prepared in analogy to **Example 1.01,** replacing *tert*-Butyl N-(3,4-dichloro-5,6-difluoro-9H-pyrido[2,3-b]indol-8-yl)-N-methyl- carbamate (**Intermediate A2**) with the **"CORE"** in step (a), N-methylmethanamine hydrochloride with the **"AMINE"** in step (a), THF/H₂O with the **"SOLVENT"** in step (b), Ad₂nBuP Biphenyl with the **"CATALYST"** in step (b) and (6-ethoxycarbonyl-8-methyl-5-oxo-1,8-naphthyridin-3-yl)boronic acid with the **"BORONIC REAGENT"** in step (b) by the reagents indicated in Table 1.

**Table 1. Compound synthesis and characterization**

| **No.** | **Compound Name and Structure** | **CORE, AMINE, BORONIC REAGENT, CATALYST and SLOVENT** | **¹H NMR and MS (ESI)** |
|---|---|---|---|
| **1.02** | **7-(6-fluoro-8-(methylamino)-4-morpholino-9H-pyrido [2,3-b]indol-3-yl)-4-oxo-4H-quinolizine-3-carboxylic acid** | **CORE:** Intermediate A1 | ¹H NMR (400MHz, DMSO-d₆) δ11.65 (d, *J*=4.9 Hz, 1H), 9.21 (s, 1H), 8.47 (d, *J*=8.4 Hz, 1H), 8.30 - 8.18 |
| | | **AMINE:** Morpholine | |
| | | **BORONIC REAGENT:** | |
| | 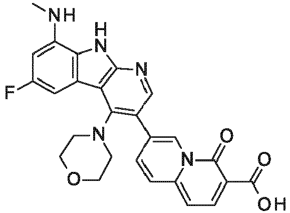 | Intermediate B1 | (m, 2H), 8.13 (d, *J*=9.8 Hz, 1H), 7.39 (d, *J*=8.5 Hz, 1H), 7.13 - 7.04 (m, 1H), 6.47 (d, *J*=11.8 Hz, 1H), 3.77 - 3.69 (m, 1H), 3.94 - 3.69 (m, 2H), 3.69 - 3.68 (m, 1H), 3.06 (s, 4H), 2.92 (s, 3H) MS (ESI): 488.2([M+H]⁺). |
| | | **CATALYST:** | |
| | | cataCXium^{®} A Pd G2 (Sigma-Aldrich, Catalog #: 761311) | |
| | | **SOLVENT:** THF/H₂O | |
| **1.03** | **7-[5,6-difluoro-4-(*cis*-5-methyl-2,3,3a,4,6,6a-hexahydropyrrolo [2,3-c]pyrrol-1-yl)-8-(methylamino)-9H-pyrido[2,3-b]indol-3-yl]-4-oxo-quinolizine-3-carboxylic acid** | **CORE:** Intermediate A2 | ¹H NMR (400 MHz, DMSO-d*₆*) *δ* ppm: 11.99-11.92 (m, 1H), 9.59 (m, 1H), 9.23-9.20 (m, 1H), 8.50 (m, 1H), 8.34-8.23 (m, 2H), 8.12-8.09 (m, 1H), 7.42-7.39 (m, 1H), 6.65-6.62 (m, 1H), 4.30 (m, 1 H), 3.55-3.36 (m, 4H), 3.27-3.14 (m, 2H), 2.91 (s, 3H), 2.84 (m, 1H), 2.69 (dd, 3 H), 2.02 (m, 1H), 1.86-1.79 (m, 1H). |
| | | **AMINE:** *cis-*5-Methyloctahydropyrrolo[ 2,3-c]pyrrole | |
| | | **BORONIC REAGENT:** | |
| | | Intermediate B1 | |
| | 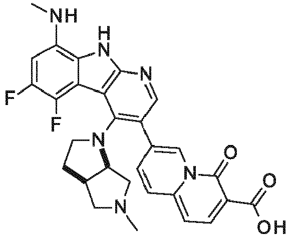 | **CATALYST:** | |
| | | cataCXium^{®} A Pd G2 (Sigma-Aldrich, Catalog #: 761311) | |
| | | **SOLVENT:** THF/H₂O | ¹H NMR (400 MHz, DMSO-d*₆*, T=80°C) *δ* ppm: 11.75 (s, 1H), 9.25 (s, 1H), 8.50 (d, 1H), 8.31 (s, 1H), 8.19 (d, 1H), 8.08-8.03 (m, 1H), 7.36 (d, 1H), 6.62 (m, 1H), 4.36 (s, 1H), 3.63-3.54 (m, 5H), 3.46-3.40 (m, 1H), 2.95 (s, 3H), 2.93-2.74 (m, 1H), 2.71 (s, 3H), 2.17 (s, 1H), 1.89 (s, 1H). |
| | | | MS (ESI): 545.2 ([M+H]⁺). |
| **1.04** | **7-[4-[(3a*S*,6a*S*)-5-methyl-2,3,3a,4,6,6a-hexahydropyrrolo[2,3-c]pyrrol-1-yl]-5,6-difluoro-8-(methylamino)-9H-pyrido [2,3-b]indol-3-yl]-4-oxo-quinolizine-3-acid carboxylic** | **CORE:** Intermediate A2 | ¹H NMR (400 MHz, DMSO-d*₆*) *δ* ppm: 14.14 (s, 1H), 11.75 (s, 1H), 9.20 (s, 1H), 8.45-8.43 (d, *J* = 8.4 Hz, 1 H), 8.22-8.16 (m, 3H), 7.35-7.33 (d, *J* = 8.4 Hz, 1 H), 6.60-6.55 (m, 1H), 5.66-5.65 (d, 1H), 4.47 (s, 1H), 3.09 (m, 2H), 2.90 (d, 3H), 2.86 (m, 1H), 2.46 (m, 2H), 2.15 (m, 1H), 2.07 (s, 3H), 2.00 (m, 1H), 1.77 (m, 1H), 1.64 (m, 1H). |
| | | **AMINE:** (3aS,6aS)-5-methyloctahydropyrrolo [ 2,3-c]pyrrole | |
| | | **BORONIC REAGENT:** | |
| | | Intermediate B1 | |
| | 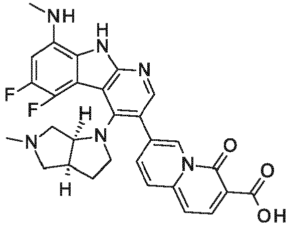 | **CATALYST:** | |
| | | cataCXium^{®} A Pd G2 (Sigma-Aldrich, Catalog #: 761311) | |
| | | **SOLVENT:** THF/H₂O | |
| | | | MS (ESI): 545.1 ([M+H]⁺). |
| **1.05** | **7-[4-[*cis*-(4*R*)-4-(dimethylamino)-3,3a,4,5,6,6a-hexahydro-lH-cyclopenta[c]pyrrol-2-yl]-5,6-difluoro-8-(methylamino)-9H-pyrido [2,3-b] indol-3-yl]-4-oxo-quinolizine-3-carboxylic acid** | **CORE:** Intermediate A2-Br | ¹H NMR (400 MHz, DMSO-d*₆*) *δ* ppm: 14.15 (s, 1H), 11.95 (s, 1H), 9.22 (s, 1H), 9.17 (s, 1H), 8.48 (d, 1H), 8.40 (s, 1H), 8.19-8.23 (m, 1H), 8.12-8.17 (m, 1H), 7.39 (d, 1H), 6.63 (m, 1H), 3.71 (s, 1H), 3.50-3.51 (m, 2H), 3.11 (s, 2H), 2.90 (s, 3H), 2.85 (s, 2H), 2.74 (m, 6H), 1.96 (s, 1H), 1.76 (m, 1H), 1.49-1.60 (m, 1H), 1.41 (m, 1H). |
| | | **AMINE:** Intermediate C1 | |
| | | **BORONIC REAGENT:** | |
| | | Intermediate B1 | |
| | | **CATALYST:** | |
| | 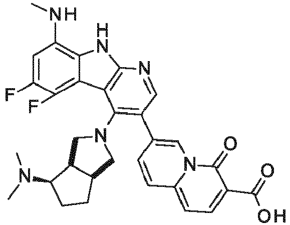 | cataCXium^{®} A Pd G2 (Sigma-Aldrich, Catalog #: 761311) | |
| | | **SOLVENT:** THF/H₂O | |
| | | | MS (ESI): 573.3 ([M+H]⁺). |
| **1.06** | **7-[4-[*cis*-3-(dimethylamino)-2,3,3a,4,6,6a-hexahydrofuro[2,3-c]pyrrol-5-yl]-5,6-difluoro-8-(methylamino)-9H-pyrido [2,3-b] indol-3-yl]-4-oxo-quinolizine-3-carboxylic acid** | **CORE:** Intermediate A2-Br | ¹H NMR (400 MHz, DMSO-d*₆*) *δ* ppm: 14.14 (s, 1H), 11.98 (s, 1H), 9.47 (s, 1H), 9.23 (s, 1H), 8.46-8.50 (m, 2H), 8.19-8.23 (m, 1H), 8.11-8.15 (m, 1H), 7.38 (d, 1H), 6.65 (m, 1H), 4.74-4.79 (m, 1H), 4.08 (m, 2H), 3.72 (m, 1H), 3.63 (m, 2H), 3.27 (m, 2H), 3.18 (m, 1H), 2.91 (s, 3H), 2.68-2.86 (m, 6H). |
| | | **AMINE:** Intermediate C2 | |
| | | **BORONIC REAGENT:** | |
| | | Intermediate B1 | |
| | | **CATALYST:** | |
| | 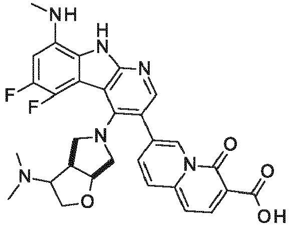 | cataCXium^{®} A Pd G2 (Sigma-Aldrich, Catalog #: 761311) | |
| | | **SOLVENT:** THF/H₂O | MS (ESI): 575.3 ([M+H]⁺). |
| **1.07** | **7-(5-chloro-6-fluoro-8-(methylamino)-4-((3a*S*,6a*S*)-5-methyl-2,3,3a,4,6,6a-hexahydropyrrolo[2,3-c]pyrrol-1-yl)-9H-pyrido [2,3-b]indol-3-yl)-4-oxo-4H-quinolizine-3-carboxylic acid** | **CORE:** Intermediate A3 | ¹H NMR (400 MHz, DMSO-d*₆*) *δ* ppm: 14.11 (brs, 1H), 11.92 (s, 1H), 9.59-9.49 (m, 1H), 9.23-9.19 (m, 1H), 8.50 (m, 1H), 8.29-8.09 (m, 3H), 7.41-7.39 (d, 1 H), 6.66-6.63 (d, 1 H), 4.76 (m, 1H), 3.60 (m, 1H), 3.60 (m, 1H), 3.40-3.00 (m, 4H), 2.92 (s, 3H), 2.69 (m, 4H), 2.05 (m, 1H), 1.99 (m, 1H), 1.63 (m, 1 H). |
| | | **AMINE:** (3aS,6aS)-5-methyloctahydropyrrolo[ 2,3-c]pyrrole | |
| | | **BORONIC REAGENT:** | |
| | | Intermediate B1 | |
| | | **CATALYST:** | |
| | 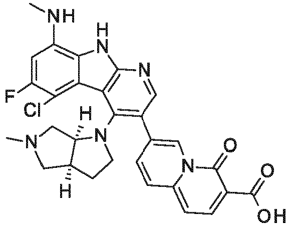 | cataCXium^{®} A Pd G2 (Sigma-Aldrich, Catalog #: 761311) | |
| | | **SOLVENT:** THF/H₂O | MS (ESI): 561.1 ({³⁵Cl} [M+H]⁺). |
| **1.08** | **7-[4-[*cis*-(4*R*)-4-(dimethylamino)-3,3a,4,5,6,6a-hexahydro-lH-cyclopenta[c]pyrrol-2-yl]-5-chloro-6-fluoro-8-(methylamino)-9H-pyrido[2,3-b]indol-3-yl]-4-oxo-quinolizine-3-carboxylic acid** | **CORE:** Intermediate A3 | ¹H NMR (400 MHz, DMSO-d*₆*) *δ* ppm: 11.88 (s, 1H), 9.23 (s, 1H), 9.17 (s, 1H), 8.48 (d, 1H), 8.21 (m, 1 H), 8.08 (m, 1 H), 7.38 (d, 1 H), 6.65 (d, 1 H), 3.58-3.14 (m, 4H), 2.92 (s, 3H), 2.80 (m, 2H), 2.73 (s, 3H), 2.66 (s, 3H), 1.98 (m, 2H), 1.75 (m, 1H), 1.42 (m, 2H). |
| | | **AMINE:** Intermediate C1 | |
| | | **BORONIC REAGENT:** | |
| | | Intermediate B1 | |
| | | **CATALYST:** | |
| | | cataCXium^{®} A Pd G2 (Sigma-Aldrich, Catalog #: 761311) | |
| | 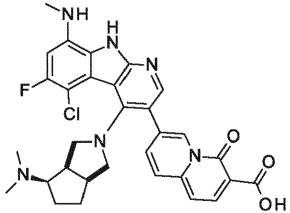 | **SOLVENT:** THF/H₂O | MS (ESI): 589.2 ({³⁵Cl} [M+H]⁺). |
| **1.09** | **7-[4-[*cis*-(4*S*)-4-(dimethylamino)-3,3a,4,5,6,6a-hexahydro-1H-cyclopenta[c]pyrrol-2-yl]-5,6-difluoro-8-(methylamino)-9H-pyrido[2,3-b]indol-3-yl]-4-oxo-quinolizine-3-carboxylic acid** | **CORE:** Intermediate A2-Br | ¹H NMR (400 MHz, DMSO-d*₆*) *δ* ppm: 14.11 (s, 1H), 11.93 (s, 1H), 9.46 (s, 1H), 9.24 (s, 1H), 8.48 (d, 1H), 8.38 (s, 1H), 8.20-8.24 (m, 1H), 8.13-8.17 (m, 1H), 7.39 (d, 1H), 6.63 (m, 1H), 3.71 (m, 1 H), 3.58 (m, 2H), 3.32-3.35 (m, 2H), 2.93-2.97 (m, 1H), 2.90 (s, 3H), 2.80 (m, 1H), 2.72 (m, 6H), 2.01 (m, 1H), 1.86 (m, 1H), 1.77 (m, 1H), 1.23 (m, 1H). |
| | | **AMINE:** Intermediate C3 | |
| | | **BORONIC REAGENT:** | |
| | | Intermediate B1 | |
| | | **CATALYST:** | |
| | 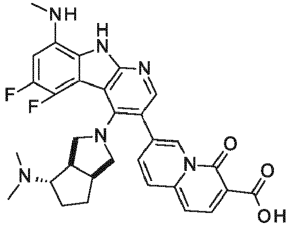 | cataCXium^{®} A Pd G2 (Sigma-Aldrich, Catalog #: 761311) | |
| | | **SOLVENT:** THF/H₂O | MS (ESI): 573.3 ([M+H]⁺). |
| **1.10** | **7-[4-(3a,5-dimethyl-3,4,6,6a-tetrahydro-2H-pyrrolo[2,3-c]pyrrol-1-yl)-6-fluoro-8-(methylamino)-9H-pyrido [2,3-b] indol-3-yl] -4-oxo-quinolizine-3-carboxylic acid** | **CORE:** Intermediate A1 | **¹H NMR** (400 MHz, DMSO-d6) δ ppm: 11.63 ∼ 11.71 (m, 1 H), 9.69 ∼ 9.98 (m, 1 H), 9.19 ~ 9.27 (m, 1 H), 8.49 ~ 8.54 (m, 1 H), 8.24 ∼ 8.29 (m, 2 H), 8.05 ∼ 8.13 (m, 1 H), 7.40 ∼ 7.46 (m, 1 H), 6.88 ∼ 7.02 (m, 1 H), 6.47 ∼ 6.55 (m, 1 H), 4.01 -4.12 (m, 1 H), 3.61 (br s, 1 H), 3.58 (br s, 2 H), 3.17 (br dd, J=12.0, 4.5 Hz, 1 H), 2.94 (s, 3 H), 2.71 ∼ 2.79 (m, 1 H), 2.62 ∼ 2.69 (m, 4 H), 2.32 ∼ 2.37 (m, 1 H), 1.87 (br dd, J=12.1, 4.5 Hz, 1H), 0.99-1.13 (m, 3 H). |
| | | **AMINE:** Intermediate C4 | |
| | | **BORONIC REAGENT:** | |
| | | Intermediate B1 | |
| | 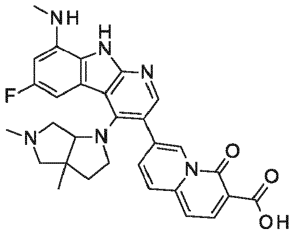 | **CATALYST:** Pd-Ad₂nBuP Biphenyl Precat (CAS#: 1375477-29-4) | |
| | | **SOLVENT:** THF/H2O | |
| | | | MS: 541.3 ([M+H]⁺). |
| **1.11** | **7-[5-cyano-6-fluoro-4-(*cis-*1-methyl-2,3,3a,4,6,6a-hexahydropyrrolo [3,4-b]pyrrol-5-yl)-8-(methylamino)-9H-pyrido[2,3-b]indol-3-yl]-4-oxo-quinolizine-3-carboxylic acid** | **CORE:** Intermediate A4 | 1H NMR (400 MHz, DMSO-d6) δ12.52 - 12.11 (m, 1H), 10.91 - 10.31 (m, 1H), 9.29 - 9.20 (m, 1H), 8.52 - 8.42 (m, 1H), 8.29 - 8.10 (m, 3H), 7.43 - 7.36 (m, 1H), 7.34 - 7.15 (m, 1H), 6.67 (d, J=12.9 Hz, 1H), 4.23 (br s, 1H), 4.02 (br d, J=9.8 Hz, 1H), 3.67 (br t, J=8.8 Hz, 1H), 3.47 - 3.23 (m, 2H), 3.49 - 3.11 (m, 1H), 3.19 (br dd, J=5.6, 11.7 Hz, 1H), 3.07 - 2.87 (m, 4H), 2.82 - 2.65 (m, 3H), 2.24 - 2.03 (m, 1H), 1.73 - 1.49 (m, 1H) MS (ESI): 552.4 ([M+H]⁺). |
| | | **AMINE:** Intermediate C5 | |
| | | **BORONIC REAGENT:** | |
| | | Intermediate B1 | |
| | | **CATALYST:** | |
| | 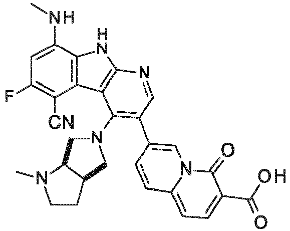 | cataCXium^{®} A Pd G2 (Sigma-Aldrich, Catalog #: 761311) | |
| | | **SOLVENT:** THF/H₂O | |
| **1.12** | **7-(5-cyano-4-(*cis*-(4R)-4 (dimethylamino)-3,3a,4,5,6,6a-hexahydro-1H-cyclopenta[c]pyrrol-2-yl)-6-fluoro-8-(methylamino)-9H-pyrido[2,3-b]indol-3-yl)-4-oxo-4H-quinolizine-3-carboxylic acid** | **CORE:** Intermediate A4 | **¹H NMR** (400 MHz, DMSO-d6) δ ppm 12.08 (br s, 1 H) 9.43 (br s, 1 H) 9.29 (br s, 1 H) 9.26 (s, 1 H) 9.20 (br s, 1 H) 8.81 (br s, 1 H) 8.10 (s, 1 H) 6.58 (br dd, J=13.4, 6.1 Hz, 1 H) 4.30 (br s, 2 H) 4.16 (s, 3 H) 3.13 - 3.27 (m, 1 H) 2.89 (s, 3 H) 2.52 - 2.56 (m, 6 H) 2.08 (br s, 2 H) 1.69 (br d, J=7.4 Hz, 2 H) 1.53 (br s, 2 H) 1.21 - 1.33 (m, 2 H) MS: 588.3 ([M+H]+), 294.8 ([2M+H]+) |
| | | **AMINE:** Intermediate C1 | |
| | | **BORONIC REAGENT:** Intermediate B1 | |
| | | **CATALYST:** Pd-Ad₂nBuP Biphenyl Precat (CAS#: 1375477-29-4) | |
| | 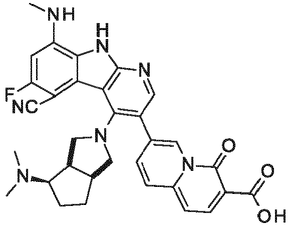 | **SOLVENT:** THF/H2O | |
| **1.13** | **7-(4-((1*R*,5*S*,6*S*)-6-(dimethylamino)-3-azabicyclo[3.1.0]hexan-3-yl)-5,6-difluoro-8-(methylamino)-9H-pyrido[2,3-b]indol-3-yl)-4-oxo-4H-quinolizine-3-carboxylic acid** | **CORE:** Intermediate A2 | **¹H NMR** (400 MHz, DMSO-d6) δ ppm: 12.05 (br s, 1 H), 9.79 (br s, 1 H), 9.26 (s, 1 H), 8.44 ~ 8.52 (m, 2 H), 8.29 (d, J=8.0 Hz, 1 H), 8.12 (br d, J=8.8 Hz, 1 H), 7.38 (d, J=8.3 Hz, 1 H), 6.67 (br dd, J=13.2, 6.0 Hz, 1 H), 3.54 (br d, J=8.0 Hz, 2 H), 3.29 (br dd, J=8.2, 3.7 Hz, 2 H), 2.91 (s, 3 H), 2.84 (s, 6 H), 2.65 (br s, 1 H), 2.25 (br s, 2 H). |
| | | **AMINE:** Intermediate C6 | |
| | | **BORONIC REAGENT:** | |
| | | Intermediate B1 | |
| | | **CATALYST:** Pd-Ad₂nBuP Biphenyl Precat (CAS#: 1375477-29-4) | |
| | | **SOLVENT:** THF/H2O | |
| | 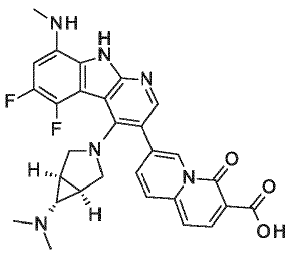 | | MS: 545.1 ([M+H]+). |
| **1.14** | **7-[4-[(3a*S*,6a*S*)-5-methyl-2,3,3a,4,6,6a-hexahydropyrrolo [2,3-c] pyrrol-1-yl]-5-cyano-6-fluoro-8-(methylamino)-9H-pyrido[2,3-b]indol-3-yl]-4-oxo-quinolizine-3-carboxylic acid** | **CORE:** Intermediate A4 | **¹H NMR** (400 MHz, DMSO-d6) δ ppm 9.09 (br s, 1 H), 8.45 (br d, *J*=8.1 Hz, 1 H), 7.66 ∼ 8.05 (m, 4 H), 6.99 (br d, *J*=8.1 Hz, 1 H), 6.53 (d, *J*=13.0 Hz, 1 H), 4.61 (br s, 1 H), 3.09 (br s, 2 H), 3.01 (s, 3 H), 2.96 (br d, *J*=7.6 Hz, 1 H), 2.35 ∼ 2.44 (m, 1 H), 2.11 ∼ 2.27 (m, 3 H), 2.05 (s, 3 H), 1.41 ∼ 1.63 (m, 2 H). MS (ESI): 552.3 ([M+H]⁺). |
| | | **AMINE:** (3aS,6aS)-5-methyloctahydropyrrolo[ 2,3-c]pyrrole | |
| | | **BORONIC REAGENT:** | |
| | | Intermediate B1 | |
| | | **CATALYST:** Pd-Ad₂nBuP Biphenyl Precat (CAS#: 1375477-29-4) | |
| | 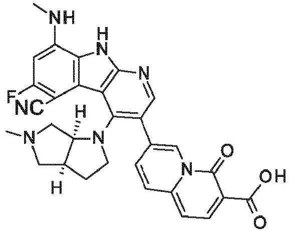 | | |
| | | **SOLVENT:** dioxane/H₂O | |
| **1.15** | **7-[5-cyano-4-[*cis*-3-(dimethylamino)-2,3,3a,4,6,6a-hexahydrofuro[2,3-c]pyrrol-5-yl]-6-fluoro-8-(methylamino)-9H-pyrido[2,3-b]indol-3-yl]-4-oxo-quinolizine-3-carboxylic acid** | **CORE:** Intermediate A4 | ¹H NMR (400 MHz, DMSO-d*₆*) *δ* ppm: 14.11 (s, 1H), 12.05 (s, 1H), 9.49 (s, 1H), 9.21 (s, 1H), 8.50 (d, 1H), 8.30 (s, 1H), 8.25 (d, 1H), 8.07 (m, 1H), 7.38 (d, 1H), 6.91 (m, 1H), 6.71 (d, 1H), 4.53 (m, 1H), 4.10 (m, 1H), 3.98 (m, 1H), 3.69-3.74 (m, 3H), 3.28-3.39 (m, 2H), 3.18 (m, 1 H), 3.01 (m, 3H), 2.69-2.84 (m, 6H). |
| | | **AMINE:** Intermediate C2 | |
| | | **BORONIC REAGENT:** | |
| | | Intermediate B1 | |
| | | **CATALYST:** | |
| | | cataCXium^{®} A Pd G2 (Sigma-Aldrich, Catalog #: 761311) | |
| | 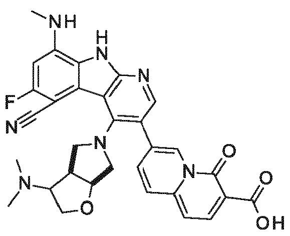 | **SOLVENT:** THF/H₂O | MS (ESI): 582.2 ([M+H]⁺). |
| **1.16** | **7-[4-[*cis*-1-methyl-2,3,3a,4,6,6a-hexahydropyrrolo[3,4-b]pyrrol-5-yl]-5-cyano-6-fluoro-8-(methylamino)-9H-pyrido[2,3-b]indol-3-yl]-4-oxo-quinolizine-3-carboxylic acid** | **CORE:** Intermediate A4 | 1H NMR (400 MHz, DMSO-d6) δ ppm 11.56 - 12.62 (m, 2 H), 9.12 (s, 1 H), 8.46 (d, J=8.56 Hz, 1 H), 8.20 - 8.29 (m, 2 H), 8.11 - 8.19 (m, 1 H), 7.34 (d, J=8.56 Hz, 1 H), 6.94 (d, J=3.55 Hz, 1 H), 6.66 (d, J=13.08 Hz, 1 H), 3.82 (t, J=8.74 Hz, 1 H), 3.49 (dd, J=10.09, 5.44 Hz, 1 H), 3.08 - 3.19 (m, 1 H), 3.00 (d, J=4.77 Hz, 3 H), 2.84 - 2.94 (m, 2H), 2.61 - 2.77 (m, 2 H), 2.55 (s, 1 H), 1.98 - 2.09 (m, 1 H), 1.78 - 1.88 (m, 1 H), 1.55 (s, 3 H), 1.39 - 1.50 (m, 1 H). |
| | | **AMINE:** Intermediate C5 | |
| | | **BORONIC REAGENT:** Intermediate B1 | |
| | | **CATALYST:** Pd-Ad₂nBuP Biphenyl Precat (CAS#: 1375477-29-4) | |
| | 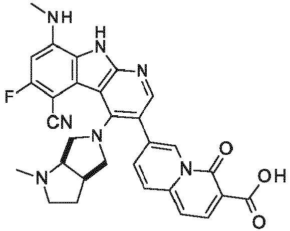 | **SOLVENT:** THF/H₂O | |
| | | | MS: 552.2 ([M+H]⁺). |
| **1.17** | **7-[4-[*cis*-1-methyl-3,3a,4,5,7,7a-hexahydro-2H-pyrrolo[2,3-c] pyridin-6-yl]-5-cyano-6-fluoro-8-(methylamino)-9H-pyrido[2,3-b]indol-3-yl]-4-oxo-quinolizine-3-carboxylic acid** | **CORE:** Intermediate A4 | ¹H NMR (400 MHz, DMSO-d*₆*) *δ* ppm: 14.10 (m, 1H), 11.90 (s, 1H), 9.25 (s, 1H), 8.48 (d, 1H), 8.26 (s, 2H), 8.16 (s, 1H), 7.38 (d, 1H), 6.88 (m, 1 H), 6.66 (d, 1H), 3.51 (m, 2H), 3.22 (m, 4H), 3.01 (d, 3H), 2.77 (m, 1H), 2.02 (m, 1H), 1.85 (m, 2H), 1.72 (s, 3H), 1.51 (m, 1H), 1.14 (m, 1H). MS (ESI): 566.5 ([M+H]⁺). |
| | | **AMINE:** Intermediate C7 | |
| | | **BORONIC REAGENT:** | |
| | | Intermediate B1 | |
| | 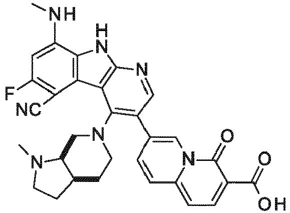 | **CATALYST:** | |
| | | cataCXium^{®} A Pd G2 (Sigma-Aldrich, Catalog #: 761311) | |
| | | **SOLVENT:** THF/H₂O | |
| **1.18** | **7-[5-cyano-4-(dimethylamino)-6-fluoro-8-(methylamino)-9H-pyrido [2,3-b] indol-3-yl]-4-oxo-quinolizine-3-carboxylic acid** | **CORE:** Intermediate A4 | ¹H NMR (400 MHz, DMSO-*d₆*) δ ppm 14.09 (br s, 1 H), 11.94 (br s, 1 H), 9.19 (s, 1 H), 8.47 (d, *J*=8.6 Hz, 1 H), 8.23 - 8.35 (m, 2 H), 8.05 (dd, *J*=8.8, 1.7 Hz, 1 H), 7.38 (d, *J*=8.3 Hz, 1 H), 6.84 (br d, *J*=3.2 Hz, 1 H), 6.66 (d, *J*=13.2 Hz, 1 H), 3.01 (d, *J*=4.6 Hz, 3 H), 2.76 (s, 6 H). |
| | | **AMINE:** Dimethylamine hydrochloride | |
| | | **BORONIC REAGENT:** | |
| | | Intermediate B1 | |
| | 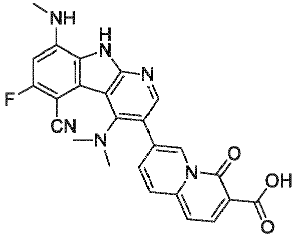 | **CATALYST:** Pd-Ad₂nBuP Biphenyl Precat (CAS#: 1375477-29-4) | |
| | | **SOLVENT:** dioxane/H₂O | MS (ESI): 471.3 ([M+H]⁺). |
| **1.19** | **7-[6-chloro-4-(*cis*-5-methyl-2,3,3a,4,6,6a-hexahydropyrrolo [2,3-c] pyrrol-1-yl)-8-(methylamino)-9H-pyrido [2,3-b] indol-3-yl]-4-oxo-quinolizine-3-carboxylic acid** | **CORE:** Intermediate A5 | ¹H NMR (400 MHz, DMSO-d*₆*) *δ* ppm: 14.17 (s, 1H), 11.73-11.80 (m, 1H), 9.44-9.69 (m, 1H), 9.23 (s, 1H), 8.50 (d, 1H), 8.21-8.28 (m, 2H), 8.02-8.13 (m, 1H), 7.38-7.44 (m, 1H), 7.07-7.16 (m, 1H), 6.61 (s, 1H), 4.37 (m, 1H), 3.94-4.04 (m, 2H), 3.52 (m, 1H), 3.38-3.48 (m, 1H), 3.26 (m, 1H), 3.03-3.14 (m, 1H), 2.93 (s, 3H), 2.74-2.81 (m, 1H), 2.65-2.73 (m, 3H), 2.10-2.31 (m, 1H), 1.81-1.99 (m, 1H). |
| | | **AMINE:** *cis*-5-Methyloctahydropyrrolo [ 2,3-c]pyrrole | |
| | | **BORONIC REAGENT:** | |
| | | Intermediate B1 | |
| | | **CATALYST:** | |
| | | cataCXium^{®} A Pd G2 (Sigma-Aldrich, Catalog #: 761311) | |
| | 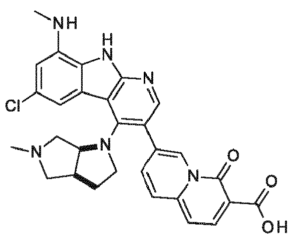 | **SOLVENT:** THF/H₂O | MS (ESI): 543.3 ([M+H]⁺). |
| **1.20** | **7-[4-(dimethylamino)-5,6-difluoro-8-(methylamino)-9H-pyrido[2,3-b]indol-3-yl]-9-methyl-4-oxo-quinolizine-3-carboxylic acid** | **CORE:** Intermediate A2 | **¹H NMR** (400 MHz, DMSO-d6): δ ppm: 11.71 (br s, 1H), 9.05 (s, 1H), 8.42 (d, 1H), 8.20 (s, 1H), 7.88 (s, 1H), 7.28 (d, 1H), 6.52 (m, 1H), 5.62 (br d, 1H), 2.83 (d, 3H), 2.74 (d, 6H), 2.63 (s, 3H) |
| | | **AMINE:** Dimethylamine hydrochloride | |
| | | **BORONIC REAGENT:** | |
| | | Intermediate B2 | |
| | 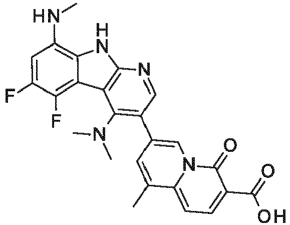 | **CATALYST:** Ad₂nBuP Biphenyl Precat (CAS#: 1375477-29-4) | |
| | | | MS: 477.8 ([M+H]⁺) |
| | | **SOLVENT:** THF/H₂O | |
| **1.21** | **7-[4-[3-(1-aminocyclopropyl)pyrrolidi n-1-yl]-5,6-difluoro-8-(methylamino)-9H-pyrido[2,3-b]indol-3-yl]-4-oxo-quinolizine-3-carboxylic acid** | **CORE:** Intermediate A2 | ¹H NMR (400MHz, DMSO-d₆) δ11.85 (s, 1H), 9.23 (s, 1H), 8.47 (d, *J*=8.4 Hz, 1H), 8.36 (s, 1H), 8.13-8.35 (m, 2H), 7.37 (d, *J*=8.5 Hz, 1H), 6.60- 6.64 (m, 1H) 5.70 (s, 1H), 2.99 (s, 3H), 1.98 (m, 2H), 1.51 (m, 2H), 1.23 (m, 2H), 0.84 (m, 1H), 0.69 (m, 4H) |
| | | **AMINE:** *tert*-butyl N-(1-pyrrolidin-3-ylcyclopropyl)carbamate | |
| | | **BORONIC REAGENT:** | |
| | | Intermediate B1 | |
| | 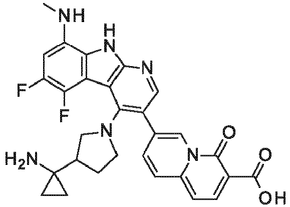 | **CATALYST:** cataCXium^{®} A Pd G2 (Sigma-Aldrich, Catalog #: 761311) | |
| | | **SOLVENT:** | MS 545.3 (ESI): ([M+H]⁺). |
| | | Dioxane/H₂O | |
| **1.22** | **7-[4-[(4a*S*,7a*R*)-4-methyl-2,3,4a,5,7,7a-hexahydropyrrolo [3,4-b] [1,4]oxazin-6-yl]-5,6-difluoro-8-(methylamino)-9H-pyrido [2,3-b] indol-3-yl] -4-oxo-quinolizine-3-carboxylic acid** | **CORE:** Intermediate A2 | ¹H NMR (400 MHz, DMSO-d*₆*) *δ* ppm: 14.2 (s, 1H), 11.8 (s, 1H), 9.20 (s, 1H), 8.16-8.47 (m, 3H), 7.36 (m, 1H), 6.62 (m, 1H), 5.70 (s, 1H), 3.25-4.00 (m, 6H), 2.91 (s, 3H), 2.05-2.70 (m, 7H). |
| | | **AMINE:** (4a*S*,7a*R*)-4-methyl-3,4a,5,6,7,7a-hexahydro-2H-pyrrolo[3,4-b][1,4]oxazine | |
| | | **BORONIC REAGENT:** | |
| | 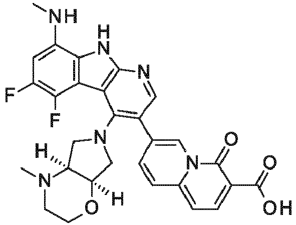 | Intermediate B1 | MS (ESI): 561.2 ([M+H]⁺). |
| | | **CATALYST:** | |
| | | cataCXium^{®} A Pd G2 (Sigma-Aldrich, Catalog #: 761311) | |
| | | **SOLVENT:** | |
| | | Dioxane/H₂O | |
| **1.23** | **7-[4-[(3aS,6aS)-5-methyl-2,3,3a,4,6,6a-hexahydropyrrolo[2,3-c]pyrrol-1-yl]-6-cyano-5-fluoro-8-(methylamino)-9H-pyrido [2,3-b] indol-3-yl] -4-oxo-quinolizine-3-carboxylic acid** | **CORE:** Intermediate A 6 | 1H NMR (400 MHz, DMSO-d6) δ ppm 13.82 - 14.52 (m, 1 H), 12.03 (s, 1 H), 9.22 (s, 1 H) 8.47 (d, J=8.56 Hz, 1 H), 8.24 - 8.30 (m, 1 H), 8.17 - 8.22 (m, 1 H) 8.14 (s, 1 H), 7.37 (d, J=8.56 Hz, 1 H), 6.75 - 6.85 (m, 2 H), 4.65 (t, J=6.24 Hz, 1 H), 3.12 (s, 1 H), 3.01 (d, J=4.77 Hz, 5 H), 2.41 - 2.47 (m, 1 H), 2.07 - 2.28 (m, 6 H), 1.62 - 1.78 (m, 1 H), 1.51 (s, 1 H). MS: 568.2 |
| | | **AMINE:** (3aS,6aS)-5-methyloctahydropyrrolo[ 2,3-c]pyrrole | |
| | | **BORONIC REAGENT:** | |
| | | Intermediate B1 | |
| | | **CATALYST:** Pd-Ad₂nBuP Biphenyl Precat (CAS#: 1375477-29-4) | |
| | | **SOLVENT:** THF/H₂O | |
| | 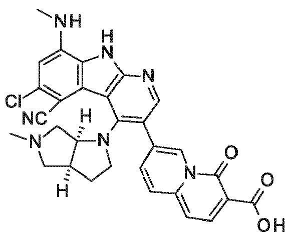 | | ([M+H]⁺). |

### Example 2.01

### 7-(5,6-Difluoro-8-(methylamino)-4-(cis-5-methyl-2,3,3a,4,6,6a-hexahydropyrrolo[2,3-c]pyrrol-1-yl)-9H-pyrido[2,3-b]indol-3-yl)-9-methyl-4-oxo-4H-quinolizine-3-carboxylic acid

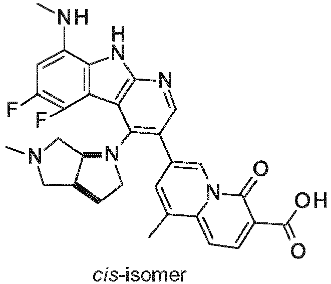

### Step (a) Preparation of tert-butyl(3-bromo-5,6-difluoro-4-(cis-5-methyl-2,3,3a,4,6,6a-hexahydropyrrolo[2,3-c]pyrrol-1-yl)-9H-pyrido[2,3-b]indol-8-yl)(methyl)carbamate

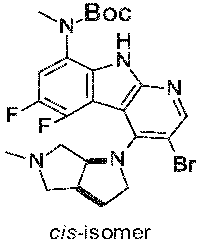

To a solution of tert-butyl (3-bromo-4-chloro-5,6-difluoro-9H-pyrido[2,3-b]indol-8-yl) (methyl)carbamate (**Intermediate A2-Br,** 210.0 mg, 0.47 mmol, as the "CORE" in Table 2) and cis-5-methyloctahydropyrrolo[2,3-c]pyrrole (118.0 mg, 0.94 mmol, as the "**AMINE**" in Table 2) in DMSO (2.0 mL) was added DIPEA (181.9 mg, 1.41 mmol) and the resulting mixture was heated to 120 °C for 12 hr. After it was cooled back to r.t., the reaction mixture was poured into water (100 mL) and extracted with EtOAc (100 mL) three times. The combined organic layer was dried over anhy. Na₂SO₄, filtered, and concentrated in vacuo to give a crude product, which was purified by Prep-TLC (petroleum ether: EtOAc = 1:1) to give *cis*-*tert*-buty](3-bromo-5,6-difluoro-4-(*cis*-5-methyl-2,3,3a,4,6,6a-hexahydropyrrolo[2,3-c]pyrrol-1-yl)-9H-pyrido[2,3-b]indol-8-yl)(methyl)carbamate (155.6 mg, 61.9% yield) as a yellow solid. MS. (ESI⁺): 518.2 (({⁷⁹Br} M+H)⁺), 520.2( ({⁸¹Br} M+H)⁺).Step (b) Preparation of *tert*-butyl(5,6-difluoro-4-(*cis*-5-methyl-2,3,3a,4,6,6a-hexahydropyrrolo[2,3-c]pyrrol-1-yl)-3-(4,4,5,5-tetramethyl-1,3,2-dioxaborolan-2-yl)-9H-pyrido[2,3-b]indol-8-yl)(methyl)carbamate

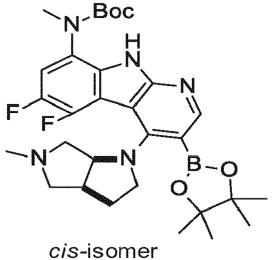

To a solution of *tert*-butyl(3-bromo-5,6-difluoro-4-(*cis*-5-methyl-2,3,3a,4,6,6a-hexahydropyrrolo[2,3-c]pyrrol-1-yl)-9H-pyrido[2,3-b]indol-8-yl)(methyl)carbamate (155.6 mg, 0.291 mmol) in anhydrous THF (1.0 mL) was added i-Pr-MgCl-LiCl (0.67 mL, 1.3 mol in THF) at 0 °C under argon. After completion of the addition, the reaction mixture was stirred at 25 °C for 3 hr before 2-isopropoxy-4,4,5,5-tetramethyl-1,3,2- dioxaborolane (162.3 mg, 0.872 mmol) was added and the reaction mixture was stirred at 25 °C for another 1 hr. The mixture was then poured into aq. NH₄Cl solution (50 mL) and extracted by EtOAc (100 mL) two times. The combined organic layer was washed by aq. NH₄Cl solution (50 mL), brine (50 ml) two times, dried over anhy. Na₂SO₄, filtered, and concentrated in vacuo to give a crude product of *tert-*butyl(5,6-difluoro-4-(*cis*-5-methylhexahydropyrrolo[2,3-c]pyrrol-1(2H)-yl)-3-(4,4,5,5-tetramethyl-1,3,2-dioxaborolan-2-yl)-9H-pyrido[2,3-b]indol-8-yl)(methyl)carbamate (118.2 mg, 69.7% yield) as a yellow solid. MS (ESI): 584.4 ([M+H]⁺). It was used directly in the next step without further purification.

### Step (c) Preparation of ethyl 2-(6-(8-((tert-butoxycarbonyl)(methyl)amino)-6-fluoro-4-(cis-5-methyl-2,3,3a,4,6,6a-hexahydropyrrolo[2,3-c]pyrrol-1-yl)-9H-pyrido[2,3-b]indol-3-yl)-4-oxo-1,8-naphthyridin-1(4H)-yl)acetate

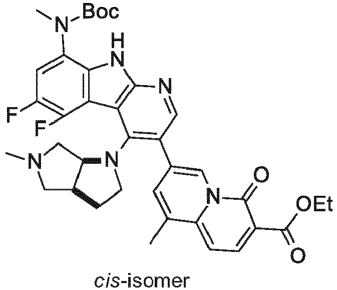

A solution of *tert*-butyl (5,6-difluoro-4-((cis-5-methyl-2,3,3a,4,6,6a-hexahydropyrrolo[2,3-c]pyrrol-1-yl)-3-(4,4,5,5-tetramethyl-1,3,2-dioxaborolan-2-yl)-9H-pyrido[2,3-b]indol-8-yl)(methyl)carbamate (100.0 mg, 0.172 mmol), ethyl 7-bromo-9-methyl-4-oxo-4H-quinolizine-3-carboxylate (79.5 mg, 0.257 mmol) **(Intermediate B2/step b,** 75 mg, 0.16 mmol, as the **"BROMIDE REAGENT"** in table 2) and K₃PO₄ (146.1 mg, 0.688 mmol) in THF/H₂O (3.0 mL, v/v=10:1 as the **"SOLVENT"** in table 4) was degassed with N₂ for five times before Pd-Ad₂nBuP Biphenyl (23.0 mg, 0.034 mmol, as the **"CATALYST"** in table 2) was added to the mixture at 0 °C under N₂. The reaction solution was degassed with N₂ for five times again before the resulting mixture was stirred at 70 °C for 12 h. After it was cooled back to r.t., the reaction mixture was poured into water (50 mL) and extracted with EtOAc (50 mL) three times. The combined organic layer was dried over anhy. Na₂SO₄, filtered, and concentrated in vacuo to give a crude product, which was purified by Prep-TLC (petroleum ether: EtOAc = 1:1) to give ethyl2-(6-(8-((*tert*-butoxycarbonyl)(methyl)amino)-6-fluoro-4-(*cis*-5-methyl-2,3,3a,4,6,6a-hexahydropyrrolo[2,3-c]pyrrol-1-yl)-9H-pyrido[2,3-b]indol-3-yl)-4-oxo-1,8-naphthyridin-1(4H)-yl)acetate (65.3 mg, 55.3% yield) as a yellow solid. MS (ESI): 687.4 ([M+H]⁺).

### Step (d) Preparation of 7-(8-((tert-butoxycarbonyl)(methyl)amino)-5,6-difluoro-4- (cis-5-methyl-2,3,3a,4,6,6a-hexahydropyrrolo[2,3-c]pyrrol-1-yl)-9H-pyrido[2,3-b]indol-3-yl)-9-methyl-4-oxo-4H-quinolizine-3 -carboxylic acid

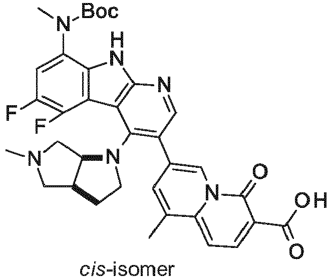

To a solution of ethyl 2-(6-(8-((*tert*-butoxycarbonyl)(methyl)amino)-6-fluoro-4- (*cis-5-*methylhexahydropyrrolo[2,3-c]pyrrol-1(2H)-yl)-9H-pyrido[2,3-b]indol-3-yl)-4-oxo-1,8-naphthyridin-1(4H)-yl)acetate (65.3 mg, 0.095 mmol) in THF (2.0 mL) and H₂O (0.4 mL) was added NaOH (15.2 mg, 0.381 mmol) and the resulting mixture was stirred at 25 °C for 2 h. The reaction mixture was then poured into water (50 mL), adjusted to pH < 7 by aq. HCl solution (0.1 M), and extracted with EtOAc (50 mL). The organic layer was washed with aq. CaCl₂ solution (100 mL, 1 N) two times and brine (100 mL) two times. The organic layer was then dried over anhy. Na₂SO₄, filtered, and concentrated in vacuo to give a crude product of 7-(8-((*tert*-butoxycarbonyl)(methyl)amino)-5,6- difluoro-4-(*cis*-5-methylhexahydropyurolo[2,3-c]pyrrol-1(2H)-yl)-9H-pyrido[2,3-b]indol-3-yl)-9-methyl-4-oxo-4H-quinolizine-3-carboxylic acid (51.3 mg, 82.1% yield) as a yellow solid. MS. (ESI⁺): 659.1 ([M+H]⁺). It was used directly in the next step without further purification.

### Step (e) Preparation of 7-(5,6-difluoro-8-(methylamino)-4-(cis-5-methyl-2,3,3a,4,6,6a-hexahydropyrrolo[2,3-c]pyrrol-1-yl)-9H-pyrido[2,3-b]indol-3-yl)-9-methyl-4-oxo-4H-quinolizine-3-carboxylic acid (Example 2.01)

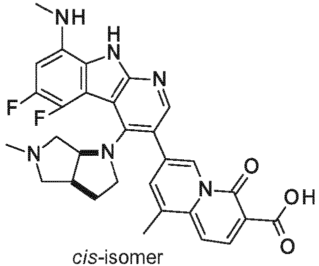

A solution of 7-(8-((*tert*-butoxycarbonyl)(methyl)amino)-5,6-difluoro-4-(*cis*-5-methyl-2,3,3a,4,6,6a-hexahydropyrrolo[2,3-c]pyrrol-1-yl)-9H-pyrido[2,3-b]indol-3-yl)-9-methyl-4-oxo-4H-quinolizine-3-carboxylic acid (51.3 mg, 0.078 mmol) in TFA (0.4 mL) and DCM (2.0 mL) was stirred at 25 °C for 2 h. Then the mixture was concentrated *in vacuo* to give a crude product, which was purified by Prep-HPLC (0.5% TFA as additive) to give 7-(5,6-difluoro-8-(methylamino)-4-(*cis*-5-methylhexahydropyrrolo[2,3-c]pyrrol-1(2H)-yl)-9H-pyrido[2,3-b]indol-3-yl)-9-methyl-4-oxo-4H-quinolizine-3-carboxylic acid (33.5 mg, 77.0% yield) as a yellow solid. MS (ESI): 559.3 ([M+H]⁺). ¹H NMR (400 MHz, MeOD-*d*₄) δ ppm: 11.85 - 12.04 (m, 1 H) 9.60 (br s, 1 H) 9.19 (br d, *J*=12 Hz, 1 H) 8.52 (dd, *J*=9, 2 Hz, 1 H) 8.19 - 8.40 (m, 1 H) 8.03 (br d, *J*=5 Hz, 1 H) 7.39 (dd, *J*=9*,* 3 Hz, 1 H) 6.65 (ddd, *J*=14, 9, 7 Hz, 1 H) 4.32 (br d, *J*=18 Hz, 1 H) 3.52 (br s, 1 H) 3.36 - 3.44 (m, 2 H) 3.25 - 3.35 (m, 1 H) 3.08 - 3.21 (m, 1 H) 2.83 - 2.97 (m, 4 H) 2.62 - 2.75 (m, 6 H) 2.19 (br s, 1 H) 2.05 (br dd, *J*=12, 7 Hz, 1 H) 1.97 - 2.11 (m, 1 H) 1.76 - 1.93 (m, 1 H).

The following examples were prepared in analogy to **Example 2.01,** replacing *ter*t-butyl (3-bromo-4-chloro-5,6-difluoro-9H-pyrido[2,3-b]indol-8-yl)(methyl) carbamate (**Intermediate A2-Br**) with the **"CORE"** in step (a), *cis*-5-methyloctahydropyrrolo[2,3-c]pyrrole with the **"AMINE"** in step (a), THF/H₂O with the **"SOLVENT"** in step (b), Ad₂nBuP Biphenyl with the **"CATALYST"** in step (b), and ethyl 7-bromo-9-methyl- 4-oxo-4H-quinolizine-3-carboxylate (**Intermediate B2**) with "**BROMIDE REAGENT**" in step(c) by the reagents indicated in Table 2.

**Table 2. Compound synthesis and characterization**

| **No.** | **Compound Name and Structure** | **CORE, AMINE, BROMIDE REAGENT, CATALYST and SLOVENT** | **¹H NMR and MS (ESI)** |
|---|---|---|---|
| **2.02** | 7-[4-[*cis*-5-methyl-**2,3,3a,4,6,6a-hexahydropyrrolo[2,3-c]pyrrol-1-yl]-5,6-difluoro-8-(methylamino)-9H-pyrido [2,3-b]indol-3-yl]- 9-fluoro-4-oxo-quinolizine-3-carboxylic acid** | **CORE:** Intermediate A2 | H NMR (400 MHz, DMSO-d6) δ ppm 11.93 - 12.14 (m, 1 H), 9.38 - 10.11 (m, 1 H), 9.06 (d, J=14.43 Hz, 1 H), 8.56 (dd, J=8.56, 3.42 Hz, 1 H), 8.22 - 8.41 (m, 1 H), 8.16 (dd, J=9.66, 5.01 Hz, 1 H), 7.39 (d, J=8.44 Hz, 1 H), 6.64 (ddd, J=13.17, 10.97, 6.42 Hz, 1 H), 4.40 (d, J=6.60 Hz, 1 H), 3.40 - 3.73 (m, 3 H), 2.83 - 3.33 (m, 6 H), 2.53 - 2.77 (m, 4 H), 2.00 - 2.28 (m, 1 H), 1.73 - 1.92 (m, 1H). MS: 563.2 ([M+H]+). |
| | | **AMINE:** *cis*-5-Methyloctahydropyrrolo [ 2,3-c]pyrrole | |
| | | **BROMIDE REAGENT:** | |
| | | Intermediate B4 | |
| | 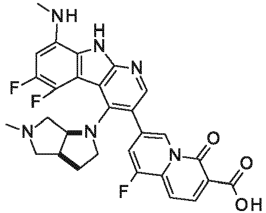 | **CATALYST:** Pd-Ad₂nBuP Biphenyl Precat (CAS#: 1375477-29-4) | |
| | | **SOLVENT:** THF/H₂O | |
| **2.03** | **7-[4-[*cis*-5-methyl-2,3,3a,4,6,6a-** | **CORE:** Intermediate A2 | **¹H NMR** (400 MHz, DMSO-d6) δ ppm: 11.82 (br |
| | **hexahydropyrrolo [2,3-c]pyrrol-1-yl]-5,6-difluoro-8-(methylamino)-9H-pyrido [2,3-b] indol-3-yl]-1-[(dimethylamino)methyl] -4-oxo-quinolizine-3-carboxylic acid** | **AMINE:** *cis-5-*Methyloctahydropyrrolo [ 2,3-c]pyrrole | s, 1 H), 9.39 (s, 1 H), 8.72 (s, 1 H), 8.58 (d, J=9.2 Hz, 1 H), 8.31 (s, 1 H), 8.24 (dd, J=9.1, 1.7 Hz, 1 H), 6.60 ∼ 6.67 (m, 1 H), 4.73 (br s, 2 H), 4.37 (br s, 1 H), 3.60 (br s, 1 H), 2.95 (s, 4 H), 2.86 (s, 8 H), 2.66 ~ 2.74 (m, 3 H), 2.10 (s, 5 H). MS: 602.1 ([M+H]⁺). |
| | | **BROMIDE REAGENT:** | |
| | | Intermediate B5 | |
| | | **CATALYST:** Pd-Ad₂nBuP Biphenyl Precat (CAS#: 1375477-29-4) | |
| | 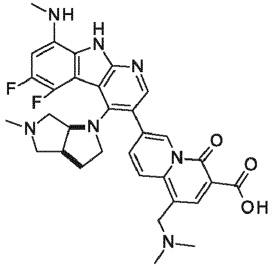 | **SOLVENT:** THF/H2O | |
| **2.04** | 7-[4-[*cis*-5-methyl-**2,3,3a,4,6,6a-hexahydropyrrolo [2,3-c]pyrrol-1-yl]-5,6-difluoro-8-(methylamino)-9H-pyrido [2,3-b] indol-3-yl]-1-methyl-4-oxo-quinolizine-3-carboxylic acid** | **CORE:** Intermediate A2 | **¹H NMR** (400 MHz, DMSO-*d₆*) δ ppm 11.84 (br s, 1 H), 9.33 (d, *J*=1.2 Hz, 1 H), 8.39 (s, 1 H), 8.32 (br s, 1 H), 8.22 ∼ 8.27 (m, 1 H), 8.11 ∼ 8.18 (m, 1 H), 6.62 (dd, *J*=13.4, 6.4 Hz, 1 H), 4.38 (br s, 1 H), 3.08 ~ 3.81 (m, 5 H), 2.95 (s, 4 H), 2.71 (s, 3 H), 2.53 ~ 2.66 (m, 4 H), 2.06 ~ 2.27 (m, 1 H), 1.89 (br s, 1 H). MS (ESI): 559.3 ([M+H]⁺). |
| | | **AMINE:** *cis-5-*Methyloctahydropyrrolo [ 2,3-c]pyrrole | |
| | | **BROMIDE REAGENT:** | |
| | | Intermediate B6 | |
| | | **CATALYST:** Pd-Ad₂nBuP Biphenyl Precat (CAS#: 1375477-29-4) | |
| | | **SOLVENT:** THF/H₂O | |
| | 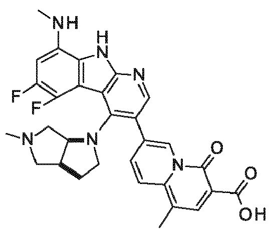 | | |
| **2.05** | **7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-1-cyclopropyl-4-oxo-quinolizine-3-carboxylic acid** | **CORE:** Intermediate A2 | **¹H NMR** (400 MHz, DMSO-*d₆*) δ ppm 11.83 (br s, 1 H), 9.34 (d, *J*=1.0 Hz, 1 H), 8.61 (d, *J*=9.0 Hz, 1 H), 8.18 ∼ 8.38 (m, 3 H), 6.63 (dd, *J*=13.3, 6.2 Hz, 1 H), 4.39 (br s, 1 H), 3.10 ∼ 3.72 (m, 5 H), 2.89 ∼ 3.01 (m, 4 H), 2.71 (s, 3 H), 2.53 ∼ 2.63 (m, 1 H), 2.10 ∼ 2.32 (m, 2 H), 1.89 (br s, 1 H), 1.10 ∼ 1.25 (m, 2 H), 0.69-0.85 (m, 2 H). MS (ESI): 585.3 ([M+H]⁺). |
| | | **AMINE:** *cis*-5-Methyloctahydropyrrolo [ 2,3-c]pyrrole | |
| | | **BROMIDE REAGENT:** | |
| | | Intermediate B7 | |
| | | **CATALYST:** Pd-Ad₂nBuP Biphenyl Precat (CAS#: 1375477-29-4) | |
| | 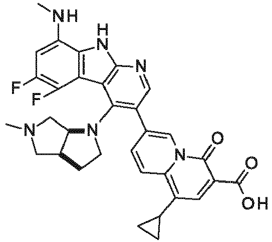 | **SOLVENT:** THF/H₂O | |
| **2.06** | **7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]- 4-oxo-1-(trifluoromethyl)quinoliz ine-3-carboxylic acid** | **CORE:** Intermediate A2 | **¹H NMR** (400 MHz, DMSO-*d₆*) δ ppm 11.84 (br s, 1 H), 9.46 (d, *J*=1.2 Hz, 1 H), 8.66 (s, 1 H), 8.10 ∼ 8.48 (m, 3 H), 6.64 (dd, *J*=13.4, 6.4 Hz, 1 H), 4.41 (br s, 1 H), 3.60 (br s, 1 H), 3.39 ∼ 3.51 (m, 2 H), 2.91 ∼ |
| | | **AMINE:** *cis-*5-Methyloctahydropyrrolo [ 2,3-c]pyrrole | |
| | | **BROMIDE REAGENT:** | |
| | 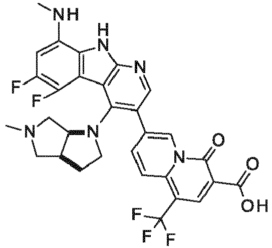 | Intermediate B8 | 3.30 (m, 6 H), 2.72 (s, 3 H), 2.36 ∼ 2.47 (m, 1 H), 2.18 (br s, 1 H), 1 . 89 (br s, 1 H). _{MS} (ESI): 613.3 ([M+H]⁺). |
| | | **CATALYST:** Pd-Ad₂nBuP Biphenyl Precat (CAS#: 1375477-29-4) | |
| | | **SOLVENT:** THF/H₂O | |
| **2.07** | **(*R*)-7-(4-(2-((dimethylamino)methyl) morpholino)-6-fluoro-8-(methylamino)-9H-pyrido [2,3-b] indol-3-yl)-4-oxo-4H-quinolizine-3-carboxylic acid** | **CORE:** Intermediate A1 | 1H NMR (400 MHz, DMSO-d6) δ ppm 11.68 (s, 1 H), 9.44 (s, 1 H), 9.22 (s, 1 H), 8.49 (d, J=8.41 Hz, 1 H), 8.26 (d, J=10.04 Hz, 2 H), 8.13 (d, J=9.16 Hz, 1 H), 7.40 (d, J=8.53 Hz, 1 H), 7.14 (dd, J=9.85, 1.94 Hz, 1 H), 6.49 (dd, J=12.17, 2.01 Hz, 1 H), 4.10 (s, 1 H), 3.98 (s, 3 H), 3.29 (d, J=12.30 Hz, 1 H), 2.99 - 3.16 (m, 4 H), 2.93 (s, 3 H), 2.77 (dd, J=11.86, 4.58 Hz, 6 H). MS: 545.1 ([M+H]⁺). |
| | | **AMINE:** Intermediate C8 | |
| | | **BROMIDE REAGENT:** | |
| | | Intermediate B1 | |
| | 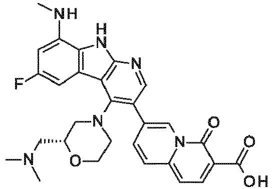 | **CATALYST:** Pd-Ad₂nBuP Biphenyl Precat (CAS#: 1375477-29-4) | |
| | | **SOLVENT:** THF/H₂O | |
| **2.08** | **(*S*)-7-(4-(2-((dimethylamino)methyl) morpholino)-6-fluoro-8-(methylamino)-9H-pyrido [2,3-b] indol-3-yl)-4-oxo-4H-quinolizine-3-carboxylic acid** | **CORE:** Intermediate A1 | ¹H NMR (400 MHz, DMSO-*d₆*) δ ppm 11.71 (s, 1 H) 9.51 (br s, 1 H) 9.23 (s, 1 H) 8.50 (d, *J*=9 Hz, 1 H) 8.20 - 8.33 (m, 2 H) 8.14 (dd, *J*=9, 2 Hz, 1 H) 7.41 (d, *J*=9 Hz, 1 H) 7.34 - 7.49 (m, |
| | | **AMINE:** C9 | |
| | | **BROMIDE REAGENT:** | |
| | | Intermediate B1 | |
| | 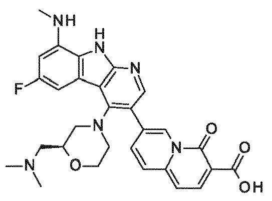 | **CATALYST:** Pd-Ad₂nBuP Biphenyl Precat (CAS#: 1375477-29-4) | 1 H) 7.14 (dd, *J*=10, 2 Hz, 1 H) 6.50 (dd, *J*=12, 2 Hz, 1 H) 3.99 (br s, 3 H) 3.30 (br d, *J*=12 Hz, 1 H) 3.03 -3.20 (m, 4 H) 2.94 (s, 2 H) 2.91 - 2.97 (m, 1 H) 2.78 (dd, *J*=11, 5 Hz, 6 H) 2.60-2.69 (m, 1 H) |
| | | **SOLVENT:** THF/H₂O | |
| | | | MS (ESI): 545.3 ([M+H]⁺). |
| **2.09** | **7-[4-[*cis*-5-methyl-2,3,3a,4,6,6a-hexahydropyrrolo[2,3-c]pyrrol-1-yl]-5,6-difluoro-8-(methylamino)-9H-pyrido [2,3-b] indol-3-yl]-1-(morpholinomethyl)-4-oxo-quinolizine-3-carboxylic acid** | **CORE:** Intermediate A2 | ¹H NMR (400 MHz, DMSO-*d₆*) δ ppm: 11.83 (br s, 1 H), 9.38 (s, 1 H), 8.65 (s, 1 H), 8.56 ~ 8.62 (m, 1 H), 8.31 (s, 1 H), 8.21 (br d, *J*=9.2 Hz, 1 H), 6.64 (dd, *J*=13.3, 6.2 Hz, 1 H), 4.55 (br s, 5 H), 4.38 (br s, 1 H), 3.79 (br s, 4 H), 3.58 ~ 3.68 (m, 1 H), 3.44 (br d, *J*=8.1 Hz, 1 H), 3.01 ~ 3.27 (m, 6 H), 2.95 (s, 3 H), 2.71 (s, 3 H), 2.17 (br s, 1 H), 1.89 (br d, *J*=6.4 Hz, 1 H). |
| | | **AMINE:** *cis*-5-Methyloctahydropyrrolo[ 2,3-c]pyrrole | |
| | | **BROMIDE REAGENT:** | |
| | | Intermediate B9 | |
| | | **CATALYST** : Pd-Ad₂nBuP Biphenyl Precat (CAS#: 1375477-29-4) | |
| | 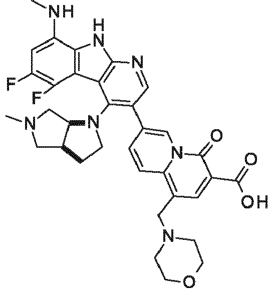 | **SOLVENT:** THF/H₂O | |
| | | | MS: 644.4 ([M+H]⁺). |
| **2.10** | **7-[4-[*cis-*5-methyl-2,3,3a,4,6,6a-hexahydropyrrolo [2,3-c]pyrrol-1-yl]-5,6-** | **CORE:** Intermediate A2 | ¹H NMR (400 MHz, DMSO-*d₆*) δ ppm 11.85 (br s, 1 H): 9.39 (d, *J*=1.6 Hz, 1 H), 8.74 (s, 1 H), 8.61 (s, 1 |
| | | **AMINE:** *cis*-5-Methyloctahydropyrrolo[ 2,3-c]pyrrole | |
| | **difluoro-8-(methylamino)-9H-pyrido [2,3-b] indol-3-yl]-4-oxo-1-(pyrrolidin-1-ylmethyl)quinolizine-3-carboxylic acid** | **BROMIDE REAGENT:** | H), 8.33 (br s, 1 H), 8.23 (dd, *J*=9.2, 1.7 Hz, 1 H), 6.63 (dd, *J*=13.4, 6.4 Hz, 1 H), 4.85 (s, 2 H), 4.41 (br s, 1 H), 3.60 (br s, 1 H), 3.42 - 3.47 (m, 7 H), 2.95 (s, 3 H), 2.69 (s, 3 H), 2.53 (br s, 1 H), 1.88 - 2.24 (m, 7 H), 1.27 (s, 1 H). |
| | | Intermediate B10 | |
| | | **CATALYST:** Pd-Ad₂nBuP Biphenyl Precat (CAS#: 1375477-29-4) | |
| | 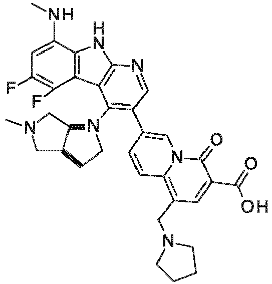 | **SOLVENT:** THF/H₂O | |
| | | | MS: 628.4 ([M+H]⁺). |
| **2.11** | **7-[4-[*cis-*5-methyl-2,3,3a,4,6,6a-hexahydropyrrolo [2,3-c]pyrrol-1-yl]-5,6-difluoro-8-(methylamino)-9H-pyrido [2,3-b] indol-3-yl]-1-chloro-4-oxo-quinolizine-3-carboxylic acid** | **CORE:** Intermediate A2 | 1H NMR (400 MHz, DMSO-d6) δ ppm: 12.02 & 11.94 (each s, 1H), 9.73&9.28 (each br s, 1H), 9.36&9.33 (each s, 1H), 8.50 (s, 1 H), 8.40-8.23 (m, 3H), 6.68-6.60 (m, 1 H), 4.35 (m, 1H), 3.70 (m, 1H), 3.50-3.39 (m, 2H), 3.23-3.10 (m, 1H), 2.90 (s, 3H), 2.86-2.83 (m, 1H), 2.70-2.65 (m, 3H), 2.52 (m, 2H), 2.20-2.01 (m, 1H), 1.88-1.76 (m, 1H). |
| | | **AMINE:** *cis-5-*Methyloctahydropyrrolo [ 2,3-c]pyrrole | |
| | | **BROMIDE REAGENT:** | |
| | | Intermediate B3 | |
| | | **CATALYST** : Pd-Ad₂nBuP Biphenyl Precat (CAS#: 1375477-29-4) | |
| | 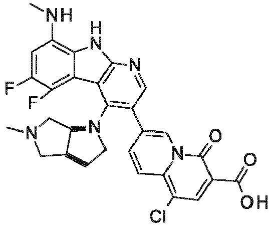 | **SOLVENT:** THF/H₂O | |
| | | | MS (ESI): 579.0. [{35Cl} (M+H)+]. |
| | | | |

### Example 3.01A

### 7- [4- [(3aR,6aR)-1-Methyl-2,3,3a,4,6,6a-hexahydropyrrolo [3,4-b] pyrrol-5-yl] -6-fluoro-8-(methylamino)-9H-pyrido [2,3-b] indol-3-yl] -4-oxo-quinolizine-3-carboxylic acid

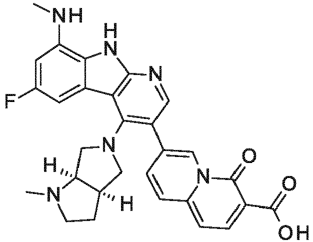

### Step (a) Preparation of cis-tert-butyl N-[4-[1-methyl-2,3,3a,4,6,6a-hexahydropyrrolo [3,4-b]pyrrol-5-yl]-3-bromo-6-fluoro-9H-pyrido[2,3-b]indol-8-yl]-N-methyl- carbamate

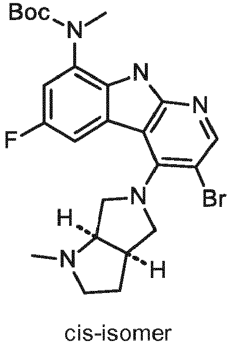

To a mixture of tert-butyl (3-bromo-4-chloro-6-fluoro-9H-pyrido[2,3-b]indol-8-yl) (methyl)carbamate **(Intermediate Al-Br,** 500.0 mg, 1.17 mmol, as the "CORE" in table 3) in DMSO (5 mL) was added N,N-diisopropylethylamine (454 mg, 3.51 mmol) and *cis-1-*methyloctahydropyrrolo[3,4-b]pyrrole (222.0 mg, 1.76 mmol, as the **"AMINE"** in table 3), and the resulting reaction mixture was stirred at 115 °C for 16 hr. After it was cooled back to r.t., the mixture was diluted with EtOAc (200 mL) and washed with brine (50 mL) three times. The organic layer was then dried over anhydrous Na₂SO₄, filtered, and concentrated *in vacuo* to give a crude product, which was triturated with MeCN (5 mL) to give *cis-tert*-butyl N-[4-[1-methyl - 2,3,3a,4,6,6a-hexahydropyrrolo[2,3-c]pyrrol-5-yl]-3-bromo-6-fluoro-9H-pyrido[2,3-b]indol-8-yl]-N-methyl-carbamate (320 mg, 40.22% yield) as a yellow solid. MS (ESI): 518.2 ({⁷⁹Br}[M+H]⁺), 520.2 ({⁸¹Br}[M+H]⁺). It was used directly in the next step without further purification.

### Step (b) Preparation of cis-[4-[1-methyl-2,3,3a,4,6,6a-hexahydropyrrolo[3,4-b]pyrrol-5-yl]-8-[tert-butoxycarbonyl(methyl)amino]-6-fluoro-9H-pyrido [2,3-b]indol-3-yl]boronic acid

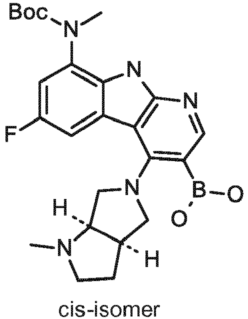

A solution of *cis-tert*-butyl N-[4-[1-methyl-2,3,3a,4,6,6a-hexahydropyrrolo[3,4-b]pyrrol-5-yl]-3-bromo-6-fluoro-9H-pyrido[2,3-b]indol-8-yl]-N-methyl-carbamate (300 mg, 0.58 mmol) in THF (2 mL) was degassed and purged with argon three times before 1,1-dimethylpropyl-magnesium chloride (2.23 mL, 2.89 mmol, 1M in THF) was added slowly and the resulting mixture was stirred at 25 °C for 3 hr. 2-Isopropoxy-4,4,5,5-tetramethyl-1,3,2-dioxaborolane (540 mg, 2.9 mmol) was added and the mixture was stirred at 25 °C for another 1 hr under argon atmosphere. The reaction mixture was quenched with aq. NH₄Cl solution (15 mL) and extracted with EtOAc (50 mL) two times. The combined organic layer was washed with brine (30 mL), dried over anhydrous Na₂SO₄, filtered, and concentrated *in vacuo* to give a crude product, which was triturated with petroleum ether (5 mL) to give *cis*-[4-[1-methyl-2,3,3a,4,6,6a-hexahydropyrrolo[2,3-c]pyrrol-5-yl]-8-[*tert*-butoxycarbonyl(methyl)amino]-6-fluoro-9H-pyrido[2,3-b]indol-3-yl]boronic acid (240 mg, 85.81% yield) as a yellow solid. MS (ESI): 484.2 ([M+H]⁺). It was used directly in the next step without further purification.

### Step (c) Preparation of cis-ethyl 7-[8-[tert-butoxycarbonyl-(methyl)aminol-6-fluoro-4-(1-methyl-2,3,3a,4,6,6a-hexahydropyrrolo[3,4-b]pyrrol-5-yl)-9H-pyrido[2,3-b]indol-3-yl]-4-oxo-quinolizine-3-carboxylate

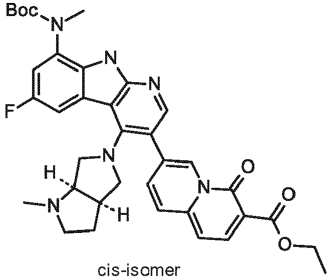

A solution of *cis*-[4-[1-methyl-2,3,3a,4,6,6a-hexahydropyrrolo[3,4-b]pyrrol-5-yl]-8-[*tert-*butoxycarbonyl(methyl)amino]-6-fluoro-9H-pyrido[2,3-b]indol-3-yl]boronic acid (220 mg, 0.46 mmol), ethyl 7-bromo-4-oxo-quinolizine-3-carboxylate (135 mg, 0.46 mmol, as the **"BROMIDE REAGENT"** in table 3), Pd-Ad₂nBuP Biphenyl Precat (122 mg, 0.18 mmol, as the **"CATALYST"** in table 3) and K₃PO₄ (290 mg, 1.37 mmol) in THF/water (2.2 mL v/v=10:1 as the **"SOLVENT"** in table 3) was degassed and purged with argon three times before it was stirred at 60 °C for 16 hr. After it was cooled down to r.t., the mixture was diluted with EtOAc (80 mL), washed with water (30 mL), and brine (30 mL). The organic layer was then dried over anhydrous Na₂SO₄, filtered, and concentrated *in vacuo* to give a crude product, which was purified by Prep-TLC (DCM: MeOH=10:1) to give *cis*-ethyl 7-[8-[*tert*-butoxycarbonyl-(methyl)amino]-6-fluoro-4-(1-methyl-2,3,3a,4,6,6a- hexahydropyrrolo[2,3-c]pyrrol-5-yl)-9H-pyrido[2,3-b]indol-3-yl]-4-oxo-quinolizine- 3-carboxylate (130 mg, 41.88% yield) as a yellow solid. MS (ESI): 655.3 ([M+H]⁺).

### Step (d) Preparation of ethyl 7-[4-[(3aR,6aR)-1-methyl-2,3,3a,4,6,6a-hexahydropynolo[3,4-blpvrrol-5-yl]-8- [tert-butoxycarbonyl(methyl)aminol-6-fluoro-9H-pyrido[2,3-b]indol-3-yl]-4-oxo-quinolizine-3-carboxylate and ethyl 7-[4-[(3aS,6aS)-1-methyl-2,3,3a,4,6,6a-hexahydropyrrolo[3,4-b]pyrrol-5-yl]-8-[tert-butoxycarbonyl(methyl)amino]-6-fluoro-9H-pyrido[2,3-b]indol-3-yl]-4-oxo-quinolizine-3-carboxylate

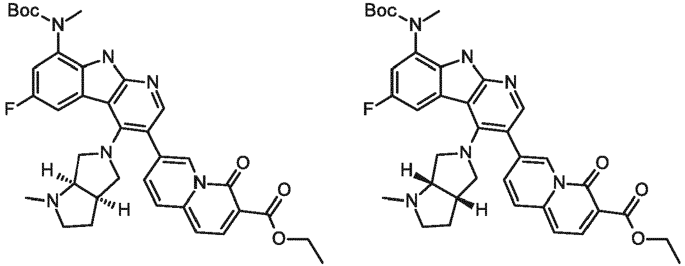

130 mg of *cis*-ethyl 7-[8-[*tert*-butoxycarbonyl- (methyl)amino]-6-fluoro-4-(1-methyl-2,3,3a,4,6,6a- hexahydropyrrolo[3,4-b]pyrrol-5-yl)-9H-pyrido[2,3-b]indol-3-yl]-4-oxo-quinolizine-3-carboxylate was purified by chiral SFC to give ethyl 7-[4-[(3aR,6aR)-1-methyl-2,3,3a,4,6,6a-hexahydropyrrolo[3,4-b]pyrrol-5-yl]-8-[*tert*-butoxycarbonyl(methyl)amino]-6-fluoro-9H-pyrido[2,3-b]indol-3-yl]-4-oxo-quinolizine-3-carboxylate (60 mg, 44.77% yield, **peak 1** Rt = 5.802 min) as a yellow solid and ethyl 7-[4-[(3a*S*,6a*S*)-1-methyl-2,3,3a,4,6,6a-hexahydropyrrolo[3,4-b]pyrrol-5-yl]-8-[*tert*-butoxycarbonyl(methyl)amino]-6-fluoro-9H-pyrido[2,3-b]indol-3-yl]-4-oxo-quinolizine-3-carboxylate (60 mg, 42.46% yield, **peak 2** Rt = 6.945 min) as another yellow solid. SFC Column: OD (250mm × 30mm,10um, Flowing Phase: 40% IPA (0.1%NH₃·H₂O) in CO₂ (as **"Chiral SFC condition"** in table 3).

### Step (c) Preparation of 7-[4-[(3aR,6aR)-1-methyl-2,3,3a,4,6,6a-hexahydropyrrolo[3,4-b]pyrrol-5-yl]-8-[tert-butoxycarbonyl(methyl)amino]-6-fluoro-9H-pyrido[2,3-b]indol-3-yl]-4-oxo-quinolizine-3-carboxylic acid

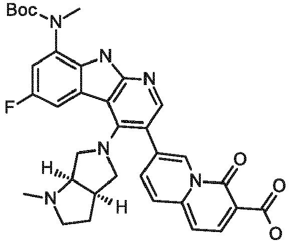

To a solution of ethyl 7-[4-[(3a*R*,6a*R*)-1-methyl-2,3,3a,4,6,6a-hexahydropyrrolo[3,4-b]pyrrol-5-yl]-8-[*tert*-butoxycarbonyl(methyl)amino]-6-fluoro-9H-pyrido[2,3-b]indol-3-yl]-4-oxo-quinolizine-3-carboxylate (60 mg, 0.09 mmol) in ethanol (5 mL) was added NaOH in water (1.5 mL, 1.5 mmol, 1 N) and the resulting mixture was stirred at 25 °C for 1 hr. Afterwards, the reaction mixture was diluted with water (20 mL) and acidified with 1N HCl to pH = 5. The aqueous layer was extracted with EtOAc (30 mL) three times, and then the combined organic layer was washed with brine (20 mL), dried over anhydrous Na₂SO₄, filtered, and concentrated in vacuo to give a crude product of 7-[4-[(3a*R*,6a*R*)-1-methyl- 2,3,3a,4,6,6a-hexahydropyrrolo [3,4-b]pyrrol-5-yl]-8-[tert-butoxycarbonyl(methyl)amino]-6-fluoro-9H-pyrido[2,3-b]indol-3-yl]-4-oxo-quinolizine-3-carboxylic acid (55 mg, 95.77% yield) as a yellow solid. MS (ESI): 626.2 ([M+H]⁺). It was used directly in the next step without further purification.

### Step (f) Preparation of 7-[4-[(3aR,6aR)-1-methyl-2,3,3a,4,6,6a-hexahydropyrrolo[3,4-b]pyrrol-5-yl]-6-fluoro-8-(methylamino)-9H-pyrido[2,3-b]indol-3-yl]-4-oxo-quinolizine-3-carboxylic acid (Example 3.01A)

To a mixture of 7-[4-[(3a*R*,6a*R*)-1-methyl-2,3,3a,4,6,6a-hexahydropyrrolo[3,4-b]pyrrol-5-yl]-8-[*tert*-butoxycarbonyl(methyl)amino]-6-fluoro-9H-pyrido[2,3-b]indol-3-yl]-4-oxo-quinolizine-3-carboxylic acid (55 mg, 0.09 mmol) in DCM (5 mL) was added trifluoroacetic acid (1.5 mL, 19.47 mmol). After the resulting mixture was stirred at 25 °C for 0.5 h, the mixture was concentrated *in vacuo* to give a crude product, which was purified by Prep-HPLC (water-CAN, 0.1%TFA as additive) to give 7-[4-[(3a*R*,6a*R*)-1-methyl-2,3,3a,4,6,6a-hexahydropyrrolo[3,4-b]pyrrol-5-yl]-6-fluoro-8-(methylamino)-9H-pyrido[2,3-b]indol-3-yl]-4-oxo-quinolizine-3-carboxylic acid. (20 mg, 35.36% yield) as a yellow solid. MS (ESI): 527.3 [(M+H)+]. ¹H NMR (400 MHz, DMSO-*d₆*) *δ* ppm: 11.67 (s, 1H), 9.62 (s, 1H), 9.20 (s, 1H), 8.50 (d, 1H), 8.27 (s, 1H), 8.22-8.19 (m, 1H), 8.13-8.11 (m, 1H), 7.42-7.39 (d, 1H), 6.83-6.81 (m, 1H), 6.50-6.47 (m, 1H), 4.17 (s, 1 H), 3.77 (m, 1H), 3.59 (m, 1H), 3.23 (m, 4H), 3.08 (m, 1H), 2.93 (s, 3H), 2.79 (m, 3 H), 2.14-1.82 (m, 2H).

### Example 3.01B

### 7-[4-[(3aS,6aS)-1-Methyl-2,3,3a,4,6,6a-hexahydropyrrolo[3,4-b]pyrrol-5-yl]-6-fluoro-8-(methylamino)-9H-pyrido[2,3-b]indol-3-yl]-4-oxo-quinolizine-3-carboxylic acid

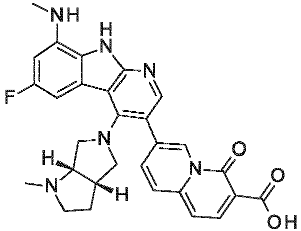

The title compound was prepared in analogy to **Example 3.01A** by replacing ethyl 7-[4-[(3a*R*,6a*R*)-1-methyl-2,3,3a,4,6,6a-hexahydropyrrolo[3,4-b]pyrrol-5-yl]- 8-[tert-butoxycarbonyl(methyl)amino]-6-fluoro-9H-pyrido[2,3-b]indol-3-yl]-4-oxo-quinolizine-3-carboxylate with ethyl 7-[4-[(3a*S*,6a*S*)-1-methyl-2,3,3a,4,6,6a- hexahydropyrrolo[3,4-b]pyrrol-5-yl]-8-[tert-butoxycarbonyl(methyl)amino]-6-fluoro-9H-pyrido[2,3-b]indol-3-yl]-4-oxo-quinolizine-3-carboxylate in step (e). MS (ESI): 527.3 [(M+H)+]. ¹H NMR (400 MHz, DMSO-*d₆*) *δ* ppm: 11.69 (s, 1H), 9.70 (s, 1H), 9.20 (s, 1H), 8.49-8.47 (m, 1H), 8.27 (s, 1H), 8.20-8.18 (m, 1H), 8.12-8.10 (m, 1H), 7.41-7.39 (m, 1H), 6.83-6.80 (m, 1H), 6.49-6.46 (m, 1H), 4.16 (s, 1H), 3.76 (m, 1H), 3.22 (m, 5H), 3.10 (m, 1H), 2.92 (s, 3H), 2.78 (s, 3H), 2.06 (m, 1H), 1.85 (m, 1H).

The following examples were prepared in analogy to **Example 3.01A** and **Example 3.01B,** replacing *tert*-butyl (3-bromo-4-chloro-6-fluoro-9H-pyrido[2,3-b]indol-8-yl) (methyl)carbamate **(Intermediate A1-Br)** with the **"CORE"** in step (a), cis-1-methyloctahydropyrrolo [3,4-b]pyrrole with the **"AMINE"** in step (a), THF/H₂O with the **"SOLVENT"** in step (b), Ad₂nBuP Biphenyl with the **"CATALYST"** in step (c) and ethyl 7-bromo-4-oxo-quinolizine-3-carboxylate with the **"BROMIDE REAGENT"** in step (c) by the reagents indicated in Table 3.

**Table 3. Compound synthesis and characterization**

| **No.** | **Compound Name and Structure** | **CORE, AMINE, BROMIDE REAGENT, CATALYST, SLOVENT and SFC Condition (peak)** | **¹H NMR and MS (ESI)** |
|---|---|---|---|
| **3.02A** | **7-[4-[(3a*S*,6a*S*)-5-methyl-2,3,3a,4,6,6a-hexahydropyrrolo[2,3-c]pyrrol-1-yl]-6-fluoro-8-(methylamino)-9H-pyrido[2,3-b]indol-3-yl]-4-oxo-quinolizine-3-carboxylic acid** | **CORE:** Intermediate A1-Br | **¹H NMR** (400 MHz, DMSO-d₆): *δ* ppm: 11.65 (m, 1H), 9.67-9.41 (m, 1H), 9.22 (s, 1H), 8.49 (m, 1H), 8.28-8.18 (m, 2H), 8.15-8.01 (m, 1H), 7.40 (m, 1H), 6.95-6.84 (m, 1H), 6.49 (m, 1H), 4.73-3.97 (m, 1H), 3.96-3.82 (m, 1H), 3.29-3.17 (m, 3H), 3.07 (m, 1H), 2.93 (s, 3H), 2.77 (m, 1H), 2.73-2.58 (m, 3H), 2.28 (m, 1H), 2.21-2.10 (m, 1H), 2.05-1.78 (m, 1 H). |
| | | **AMINE:** *cis*-5-Methyloctahydropyrrolo [2 ,3-c]pyrrole | |
| | | **BROMIDE REAGENT:** Intermediate B1.4 | |
| | 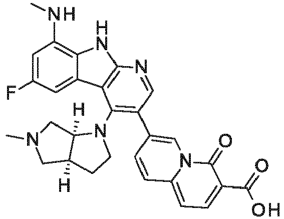 | **CATALYST:** Pd-Ad₂nBuP Biphenyl Precat (CAS#: 1375477-29-4) | |
| | | **SOLVENT:** THF/H₂O | |
| | | **SFC Condition (peak 1,** Rt = 4.773 min): SFC Column: | |
| | | AY(250mm×50mm,10µm ) | MS: 527.3 ([M+H]⁺). |
| | | Mobile Phase: MeOH (0.1%NH₃H₂O) in CO | |
| **3.02B** | **7-[4-[(3a*R*,6a*R*)-5-methyl-2,3,3a,4,6,6a-** | **CORE:** Intermediate A1- | **¹H NMR** (400 MHz, DMSO-d₆): *δ* ppm: 11.65 |
| | **hexahydropyrrolo[2,3-c]pyrrol-1-yl]-6-fluoro-8-(methylamino)-9H-pyrido[2,3-b]indol-3-yl]-4-oxo-quinolizine-3-carboxylic acid** | Br | (s, 1H), 9.64-9.39 (m, 1H), 9.22 (s, 1H), 8.49 (d, 1H), 8.28-8.21(m, 2H), 8.12-8.02 (m, 1H), 7.41 (m, 1H), 6.96-6.84 (m, 1H), 6.49 (m, 1H), 4.33-4.06 (m, 1H), 3.97-3.65 (m, 1H), 3.30-3.19 (m, 3H), 3.12-3.04 (m, 1H), 2.93 (s, 3H), 2.77 (m, 1H), 2.71-2.65 (m, 3 H), 2.28 (m, 1H), 2.13 (m, 1H), 2.02-1.84 (m, 1H). |
| | | **AMINE:** *cis-5-*Methyloctahydropyrrolo[2 ,3-c]pyrrole | |
| | 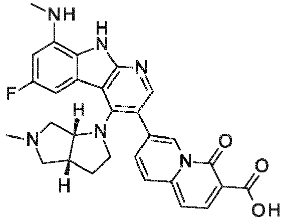 | **BROMIDE REAGENT:** Intermediate B1.4 | |
| | | **CATALYST:** Pd-Ad₂nBuP Biphenyl Precat (CAS#: 1375477-29-4) | |
| | | **SOLVENT:** THF/H₂O | |
| | | **SFC Condition (peak 2,** Rt = 5.382 min): | |
| | | SFC Column: | MS: 527.3 ([M+H]⁺). |
| | | AY(250mm×250mm,10µm ) | |
| | | Mobile Phase: MeOH (0.1%NH₃H₂O) in CO | |
| **3.03A** | **7-[4-[(3aR,6aR)-1-methyl-2,3,3a,4,6,6a-hexahydropyrrolo [3,4-b]pyrrol-5-yl]-5,6-difluoro-8-(methylamino)-9H-pyrido [2,3-b] indol-3-yl] -4-oxo-quinolizine-3-carboxylic acid** | **CORE:** Intermediate A2 | ¹H NMR (400 MHz, METHANOL-d₄) δ ppm 9.34 (br s, 1H), 8.43-8.54 (m, 2H), 8.23-8.43 (m, 1H), 8.04 (br s, 2H), 7.23 (br d, *J*=6.85 Hz, 1H), 6.61 (br dd, *J*=5.81, 13.14 Hz, 1H), 3.79 (br s, 2H), 3.60-3.73 (m, 1H), 3.05-3.27 (m, 3H), 2.91-3.03 (m, 4H), 2.79 (br d, *J*=13.33 Hz, 1H), 2.34-2.54 (m, 3H), 2.15 (br s, 1H), 1.67 (br s, 1H) |
| | | **AMINE:** *cis*-1-methyl-3,3a,4,5,6,6a-hexahydro-2H-pyrrolo[2,3-c]pyrrole | |
| | | **BORONIC REAGENT:** Intermediate B1 | |
| | 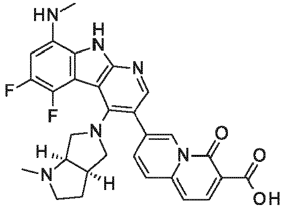 | **CATALYST:** Pd-Ad₂nBuP Biphenyl Precat (CAS#: 1375477-29-4) | MS (ESI): 544.9 ([M+H]⁺). |
| | | **SOLVENT:** dioxane/H₂O | |
| **3.03B** | **7-[4-[(3a*S*,6a*S*)-1-methyl-2,3,3a,4,6,6a-hexahydropyrrolo[3,4-b] pyrrol-5-yl]-5,6-difluoro-8-(methylamino)-9H-pyrido[2,3-b]indol-3-yl]-4-oxo-quinolizine-3-carboxylic acid** | **CORE:** Intermediate A2 | ¹H NMR (400 MHz, METHANOL-d₄) δ ppm 9.34 (br s, 1H), 8.50 (m, 2H), 8.32 (m, 1H), 8.03 (br s, 2H), 7.22 (br s, 1H), 6.51-6.70 (m, 1H), 3.57-3.92 (m, 3H), 3.07-3.27 (m, 3H), 2.86-3.05 (m, 4H), 2.78 (br d, *J*=19.56 Hz, 1H), 2.32-2.53 (m, 3H), 2.14 (br s, 1H), 1.66 (br s, 1H) |
| | | **AMINE:** *cis*-1-methyl-3,3a,4,5,6,6a-hexahydro-2H-pyrrolo[2,3-c]pyrrole | |
| | | **BORONIC REAGENT:** Intermediate B1 | |
| | 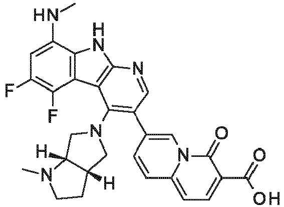 | **CATALYST:** Pd-Ad₂nBuP Biphenyl Precat (CAS#: 1375477-29-4) | |
| | | **SOLVENT:** dioxane/H₂O | MS (ESI): 544.9 ([M+H]⁺). |
| **3.04A** | **7-[4-[(3aR,7aR)-1-methyl-3,3a,4,5,7,7a-hexahydro-2H-pyrrolo[2,3-c]pyridin-6-yl]-5,6-difluoro-8-(methylamino)-9H-pyrido [2,3-b] indol-3-yl] -4-oxo-quinolizine-3-carboxylic acid** | **CORE:** Intermediate A2 | ¹H NMR (400 MHz, DMSO-d₆) δ ppm 14.18 (br s, 1H), 11.86 (s, 1H), 9.31 (s, 1H), 8.45 (d, *J*=8.44 Hz, 1H), 8.38 (s, 1H), 8.15-8.31 (m, 2H), 7.37 (d, *J*=8.44 Hz, 1H), 6.63 (dd, *J*=6.30, 13.51 Hz, 1H), 5.70 (br d, *J*=4.89 Hz, 1H), 3.37-3.48 (m, 2H), 3.20-3.29 (m, 1H), 3.16 (brs, 1H), 2.90 (d, *J*=4.89 Hz, 4H), 2.65-2.81 (m, 1H), 2.07-2.29 (m, 2H), 1.99 (br s, 1H), 1.84 (br s, 1H), 1.67 (br s, 4H), 1.19-1.36 (m, 1H). |
| | | **AMINE:** *cis*-1-methyl-2,3,3a,4,5,6,7,7a-octahydropyrrolo[2,3-c]pyridine | MS (ESI): 559.2 ([M+H]⁺). |
| | | **BORONIC REAGENT:** Intermediate B1 | |
| | 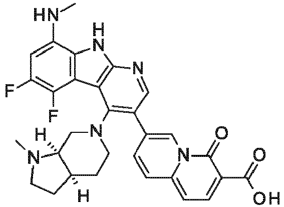 | **CATALYST:** Pd-Ad₂nBuP Biphenyl Precat (CAS#: 1375477-29-4) | |
| | | **SOLVENT:** dioxane/H₂O | |
| **3.04B** | **7-[4-[(3a*S*,7a*S*)-1-methyl-3,3a,4,5,7,7a-hexahydro-2H-pyrrolo [2,3-c]pyridin-6-yl]-5,6-difluoro-8-(methylamino)-9H-pyrido [2,3-b] indol-3-yl] -4-oxo-quinolizine-3-carboxylic acid** | **CORE:** Intermediate A2 | ¹H NMR (400 MHz, DMSO-d₆) δ ppm 14.17 (br s, 1H), 11.87 (br s, 1H), 9.31 (s, 1H), 8.46 (d, *J*=8.44 Hz, 1H), 8.38 (s, 1H), 8.18-8.28 (m, 2H), 7.37 (d, *J*=8.56 Hz, 1H), 6.63 (dd, *J*=6.24, 13.45 Hz, 1H), 5.71 (br d, *J*=4.77 Hz, 1H), 3.38-3.49 (m, 2H), 3.09-3.29 (m, 2H), 2.90 (d, *J*=4.89 Hz, 4H), 2.71 (br s, 1H), 2.12 (br d, *J*=10.27 Hz, 2H), 1.95 (br s, 1H), 1.84 (br s, 1H), 1.50-1.77 (m, 4H), 1.16-1.41 (m, 1H). |
| | | **AMINE:** *cis*-1-methyl-2,3,3a,4,5,6,7,7a-octahydropyrrolo[2,3-c]pyridine | |
| | | **BORONIC REAGENT:** Intermediate B1 | |
| | 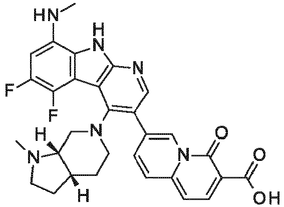 | **CATALYST:** Pd-Ad₂nBuP Biphenyl Precat (CAS#: 1375477-29-4) | |
| | | **SOLVENT:** dioxane/H₂O | |
| | | | MS (ESI): 559.2 ([M+H]⁺). |

### Example 4.01A

### 7-[4-[(1S,3R)-1-amino-5-azaspiro[2.4]heptan-5-yl]-5,6-difluoro-8-(methylamino)-9H-pyrido[2,3-b]indol-3-yl]-4-oxo-quinolizine-3-carboxylic acid

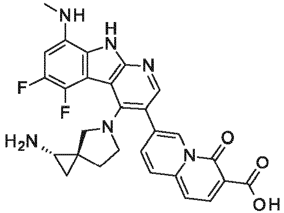

### Step (a) Preparation of tert-butyl (4-(1-((tert-butoxycarbonyl)amino)-5-azaspiro[2.4] heptan-5-yl)-3-chloro-5,6-difluoro-9H-pyrido[2,3-b]indol-8-yl)(methyl)carbamate

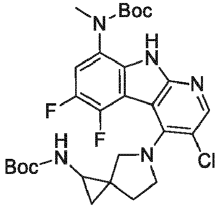

A solution of *tert*-Butyl N-(3,4-dichloro-5,6-difluoro-9H-pyrido[2,3-b]indol-8-yl)-N-methyl- carbamate (400.0 mg, 1 mmol, intermediate A2, as **"CORE"** in table 4), *tert-butyl 5-*azaspiro[2.4]heptan -1-ylcarbamate (390.8 mg, 1.8 mmol, as **"AMINE"** in table 4) and N,N-diisopropylethylamine (0.57 mL, 3.2 mmol) in DMSO (5 mL) was stirred at 110 °C for 12 hr. After it was cooled back to r.t., the mixture was diluted with EtOAc (100mL), poured into water (100 mL), and extracted with EtOAc (100 mL). The organic layer was washed with aq. NaCl solution (100 mL), dried over anhydrous Na₂SO₄, filtered, and concentrated in vacuo to give a crude product, which was purified by Prep-TLC (petroleum ether: EtOAc = 2:1) to give *tert-*butyl N-[4-[2-(*tert*-butoxycarbonylamino)-5-azaspiro[2.4]heptan-5-yl]-3-chloro-5,6-difluoro-9H-pyrido[2,3-b]indol-8-yl]-N-methyl-carbamate (550 mg, 95.68% yield) as a yellow solid. MS (ESI): 770.6 ([{³⁵Cl}M+H]⁺).

### Step (b) Preparation of tert-butyl (4-((1S,3R)-1-((tert-butoxvcarbonvl)amino)- 5-azaspiro[2.4]heptan-5-yl)-3-chloro-5,6-difluoro-9H-pyrido[2,3-b]indol-8-yl)(methyl)carbamate

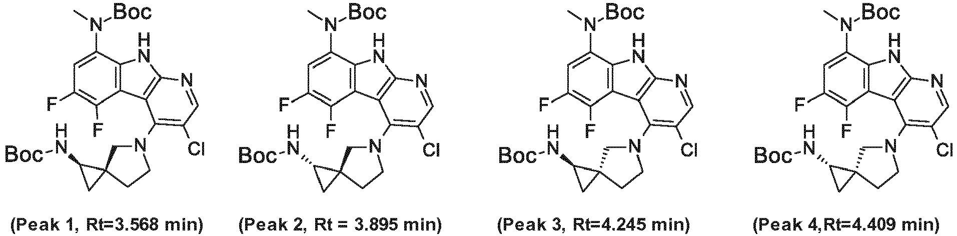

550 mg of *tert*-butyl N-[4-[2-(tert-butoxycarbonylamino)-5-azaspiro[2.4]heptan-5- yl]-3-chloro-5,6-difluoro-9H-pyrido[2,3-b]indol-8-yl]-N-methyl-carbamate was applied to chiral SFC separation to give *tert*-butyl (4-((1*S*,3*R*)-1-((tert-butoxycarbonyl)amino)-5- azaspiro[2.4]heptan-5-yl)-3-chloro-5,6-difluoro-9H-pyrido[2,3-b]indol-8-yl)(methyl)carbamate (110 mg, Peak 2, Rt=3.895min). MS (ESI): 770.6 ([{³⁵Cl}M+H]⁺).
SFC condition: Column: AD, 250×20 mm I.D., 5µm. Mobile phase: A for CO₂ and B for IPA (0.1% NH₃H₂O); Gradient: B 20%. Flow rate: 50 mL/min. Back pressure: 100 bar. Column temperature: 35 °C.

### Step (c) Preparation of ethyl 7-(8-((tert-butoxycarbonyl)(methyl)amino)-4-((1S,3R)- 1-((tert-butoxycarbonyl)amino)-5-azaspiror2.41heptan-5-yl)-5,6-difluoro-9H-pyridor2,3-bTindol-3-yl)-4-oxo-4H-quinolizine-3-carboxylate

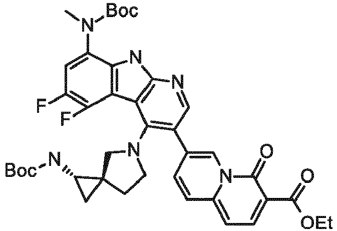

A mixture solution of *tert*-butyl (4-((1*S*,3*R*)-1-((*tert*-butoxycarbonyl)amino)-5-azaspiro [2.4]heptan-5-yl)-3-chloro-5,6-difluoro-9H-pyrido[2,3-b]indol-8-yl)(methyl)carbamate (100.0 mg, 0.2 mmol), ethyl 4-oxo-7-(4,4,5,5-tetramethyl-1,3,2-dioxaborolan-2-yl)- 4H-quinolizine-3-carboxylate (70.0 mg, 0.2 mmol, Intermediate B1, as **"BRONIC REAGENT"** in table 4), and K₂CO₃ (0.05 mL, 0.57 mmol) in THF (5.0 mL) and water (1.0 mL) was degassed with N₂ five times before cataCXium^{®} A Pd G2 (11.2 mg, 0.02 mmol) was added under N₂. The resulting reaction mixture was degassed with N₂ five times and then stirred at 70 °C for 2 hr under N₂. After it was cooled down to r.t., the mixture was diluted with EtOAc (100 mL), poured into water (100 mL), and extracted with EtOAc (100 mL) two times. The organic layer was washed with brine (100 mL), dried over anhydrous Na₂SO₄, filtered, and concentrated to give a crude product, which was purified by Prep-TLC (DCM: MeOH = 20:1) to give ethyl 7-[4-[(2*S*,3*R*)-2-(*tert*-butoxycarbonylamino)-5-azaspiro[2.4]heptan-5-yl]-8-[*tert*-butoxycarbonyl(methyl)amino]-5,6-difluoro-9H-pyrido[2,3-b]indol-3-yl]-4-oxo-quinolizine-3-carboxylate (95 mg, 72.37% yield) as a yellow solid, MS (ESI): 759.4 ([M+H]⁺).

### Step (d) Preparation of 7-(8-((tert-butoxycarbonyl)(methyl)amino)-4-((1S,3R)-1-((tert-butoxycarbonyl)amino)-5-azaspiro[2.4]heptan-5-yl)-5,6-difluoro-9H-pyrido[2,3-b]indol-3-yl)-4-oxo-4H-quinolizine-3-carboxylic acid

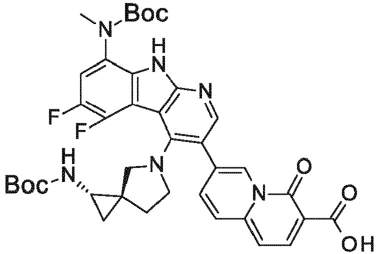

To a solution of ethyl 7-[4-[(2*S,*3*R*)-2-(*tert*-butoxycarbonylamino)-5- azaspiro[2.4]heptan-5-yl]-8-[*tert*-butoxycarbonyl(methyl)amino]-5,6-difluoro-9H-pyrido[2,3-b]indol-3-yl]-4-oxo-quinolizine-3-carboxylate (95.0 mg, 0.13 mmol) in ethanol (5.0 mL) and water (1.0 mL) was added NaOH (50.0 mg, 1.2 mmol), and the resulting reaction mixture was stirred at 20 °C for 2 hr. Afterwards, the reaction mixture was diluted with DCM (50 mL), poured into ice water (20 mL), adjusted to pH = 3 with aq. HCl solution (2M), and extracted with DCM (100 mL). The organic layer was then washed with brine (50 mL),dried over anhydrous Na₂SO₄, filtered, and concentrated in vacuo to give a crude product of 7-[4-[(2*S*,3*R*)-2-(*tert*-butoxycarbonylamino)-5-azaspiro[2.4]heptan-5-yl]-8-[*tert*-butoxycarbonyl(methyl)amino]-5,6-difluoro-9H-pyrido[2,3-b]indol-3-yl]-4-oxo-quinolizine-3-carboxylic acid (90 mg, 98.37% yield) as a yellow solid, MS (ESI): 731.3 ([M+H]⁺). It was used directly in the next step without further purification.

### Step (e) Preparation of 7-(4-((1S,3R)-1-amino-5-azaspiro[2.4]heptan-5-yl)-5,6-difluoro-8-(methylamino)-9H-pyrido[2,3-b]indol-3-yl)-4-oxo-4H-quinolizine-3-carboxylic acid

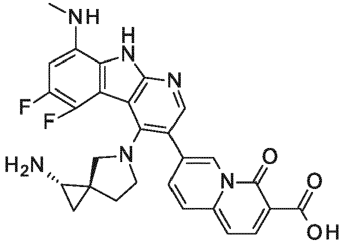

To a solution of 7-[4-[(2*S*,3*R*)-2-(*tert*-butoxycarbonylamino)-5-azaspiro [2.4]heptan-5-yl]-8-[*tert*-butoxycarbonyl(methyl)amino]-5,6-difluoro-9H-pyrido[2,3-b]indol-3-yl]-4-oxo-quinolizine-3-carboxylic acid (90.0 mg, 0.12 mmol) in anhydrous DCM (4.0 mL) was added trifluoroacetic acid (2.0 mL, 25.9 mmol), and the resulting reaction mixture was stirred at 20 °C for 1 hr. Afterwards, the mixture was concentrated *in vauco* to give a crude product, which was purified by Prep-HPLC (0.1% TFA as additive) to give 7-[4-[(1*S*,3*R*)-1-amino-5-azaspiro[2.4]heptan-5-yl]-5,6-difluoro-8- (methylamino)-9H-pyrido[2,3-b]indol-3-yl]-4-oxo-quinolizine-3-carboxylic acid. (37 mg, 46.3% yield) as a yellow solid. MS (ESI): 531.2 ([M+H]⁺).¹H NMR (400 MHz, DMSO-*d₆*) δ ppm 11.99 (br s, 1 H), 9.26 (s, 1 H), 8.48 (d, *J*=8.44 Hz, 1 H), 8.29∼8.35 (m, 1 H), 8.21~8.26 (m, 1 H), 8.08∼8.14 (m, 1 H), 7.98∼8.08 (m, 3 H), 7.41 (s, 1 H), 6.57∼6.66 (m, 1 H), 3.44∼3.53 (m, 2 H), 3.23∼3.33 (m, 2 H), 2.90 (s, 3 H), 2.64∼2.72 (m, 1 H), 1.98∼2.09 (m, 1 H), 1.68∼1.78 (m, 1 H), 0.95∼1.02 (m, 1 H), 0.65-0.73 (m, 1 H).

The following examples were prepared in analogy to **Example 4.01A,** replacing *tert*-butyl (3-bromo-4-chloro-5,6-difluoro-9H-pyrido[2,3-b]indol-8-yl)(methyl) carbamate **(Intermediate A2)** with the **"CORE"** in step (a), N-methylmethanamine hydrochloride with the **"AMINE"** in step (a), THF/H₂O with the **"SOLVENT"** in step (c), Ad₂nBuP Biphenyl with the **"CATALYST"** in step (c) and ethyl 4-oxo-7-(4,4,5,5-tetramethyl-1,3,2-dioxaborolan-2-yl)quinolizine-3-carboxylate with the **"BORONIC REAGENT"** in step (c) by the reagents indicated in Table 4.

**Table 4. Compound synthesis and characterization**

| **No.** | **Compound Name and Structure** | **CORE, AMINE, BROMIDE REAGENT, CATALYST and SLOVENT** | **¹H NMR and MS (ESI)** |
|---|---|---|---|
| 4.01 B | **7-(4-((1*R*,3*R*)-1-amino-5-azaspiro [2.4] heptan-5-yl)-5,6-difluoro-8-(methylamino)-9H-pyrido [2,3-b] indol-3-yl)-4-oxo-4H-quinolizine-3-carboxylic acid** | **CORE:** Intermediate A2 | ¹H NMR (400MHz, DMSO-d₆) δ ppm 11.94 (br s, 1 H) 9.24 (s, 1 H) 8.48 (d, *J*=9 Hz, 1 H) 8.32 (s, 1 H) 8.22 (br d, *J*=8 Hz, 3 H) 8.11 (dd, *J*=9, 1 Hz, 1 H) 7.34 - 7.41 (m, 1 H) 6.61 (dd, *J*=13, 6 Hz, 1 H) 3.45 - 3.59 (m, 2 H) 3.24 (br d, *J*=10 Hz, 1 H) 3.07 (br d, *J*=9 Hz, 1 H) 3.04 - 3.11 (m, 1 H) 2.91 (s, 3 H) 2.54 - 2.63 (m, 1 H) 2.01-2.11 (m, 1 H) 1.95 (dt, *J*=12, 6 Hz, 1 H) 0.77 - 0.92 (m, 2 H) |
| | | **AMINE:** *tert*-butyl 5-azaspiro[2.4]heptan-1-ylcarbamate | |
| | | **BORONIC REAGENT:** Intermediate B1 | |
| | 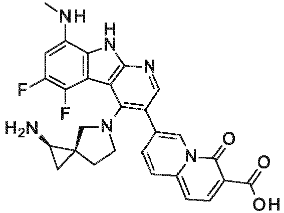 | **CATALYST:** Pd-Ad₂nBuP Biphenyl Precat (CAS#: 1375477-29-4) | |
| | | **SOLVENT:** THF/H₂O | |
| | | | MS (ESI): 531.3 ([M+H]⁺). |
| 4.01 C | **7-(4-((1*R*,3*S*)-1-amino-5-azaspiro [2.4] heptan-5-yl)-5,6-difluoro-8-(methylamino)-9H-pyrido [2,3-b] indol-3-yl)-4-oxo-4H-quinolizine-3-carboxylic acid** | **CORE:** Intermediate A2 | ¹H NMR (400MHz, DMSO-d₆) 11.95 (s, 1 H) 9.27 (s, 1 H) 8.50 (d, *J*=8 Hz, 1 H) 8.32 (s, 1 H) 8.24 (d, *J*=9 Hz, 1 H) 8.12 (dd, *J*=9, 2 Hz, 1 H) 8.03 (br s, 3 H) 7.41 (d, *J*=9 Hz, 1 H) 6.63 |
| | | **AMINE:** *tert*-butyl 5-azaspiro[2.4]heptan-1-ylcarbamate | |
| | | **BORONIC REAGENT:** | |
| | 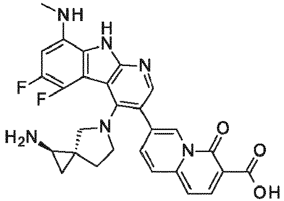 | Intermediate B1 | (dd, *J*=13, 6 Hz, 1 H) 3.49 (br t, *J*=7 Hz, 2 H) 3.27 (q, *J*=10 Hz, 2 H) 2.91 (s, 3 H) 2.69 (br d, *J*=2 Hz, 1 H) 2.00-2.11 (m, 1 H) 1.73 (dt, *J*=12, 6 Hz, 1 H) 1.00 (t, *J*=7 Hz, 1 H) 0.69 (t, *J*=5 Hz, 1 H) |
| | | **CATALYST:** Pd-Ad₂nBuP Biphenyl Precat (CAS#: 1375477-29-4) | |
| | | **SOLVENT:** THF/H₂O | |
| | | | MS (ESI): 531.3 ([M+H]⁺). |
| 4.01 D | **7-(4-((1*S*,3*S*)-1-amino-5-azaspiro [2.4] heptan-5-yl)-5,6-difluoro-8-(methylamino)-9H-pyrido [2,3-b] indol-3-yl)-4-oxo-4H-quinolizine-3-carboxylic acid** | **CORE:** Intermediate A2 | 1H NMR (400 MHz, DMSO-d6) δ ppm: 11.87 (s, 1 H), 9.24 (s, 1 H), 8.48 (d, J=8.6 Hz, 1 H), 8.32 (s, 1 H), 8.22 (d, J=8.8 Hz, 1 H), 8.14 ~ 8.18 (m, 1 H), 8.14 ∼-8.18 (m, 1 H), 8.16 (br d, J=3.8 Hz, 2H), 8.14 ∼ 8.18 (m, 1 H), 8.11 (dd, J=8.9, 1.6 Hz, 1 H), 8.09 ∼ 8.13 (m, 1 H), 7.38 (d, J=8.4 Hz, 1 H), 6.62 (dd, J=13.5, 6.3 Hz, 1 H), 3.47 3.53 (m, 4 H), 3.23 (br d, J=9.4 Hz, 1 H), 3.04 (d, J=9.5 Hz, 1 H), 2.91 (s, 3H), 1.88-2.13 (m, 2 H), 0.79 ∼ 0.91 (m, 2 H). |
| | | **AMINE:** *tert-*butyl 5-azaspiro[2.4]heptan -1-ylcarbamate | |
| | | **BORONIC REAGENT:** Intermediate B1 | |
| | 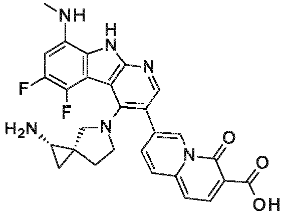 | **CATALYST:** Pd-Ad₂nBuP Biphenyl Precat (CAS#: 1375477-29-4) | |
| | | **SOLVENT:** THF/H2O | |
| | | | MS: 531.3 ([M+H]+), 257.2 ([M+2H]+). |

### Example 5.01A

### 7-(4-((1S,3R)-1-amino-5-azaspiro[2.4]heptan-5-yl)-5-chloro-6-fluoro-8-(methylamino)-9H-pyrido[2,3-b]indol-3-yl)-4-oxo-4H-quinolizine-3-carboxylic acid

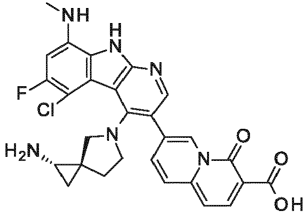

### Step (a) Preparation of cis-tert-butyl (3-bromo-4-(1-((tert-butoxycarbonyl)amino)-5-azaspiro[2.4]heptan-5-yl) -5-chloro-6-fluoro-9H-pyrido[2,3-b]indol-8-yl)(methyl)carbamate and trans-tert-butyl (3-bromo-4-(1-((tert-butoxycarbonyl)amino)-5-azaspiro[2.4]heptan-5-yl)-5-chloro-6-fluoro-9H-pyrido[2,3-b]indol-8-yl)(methyl)carbamate

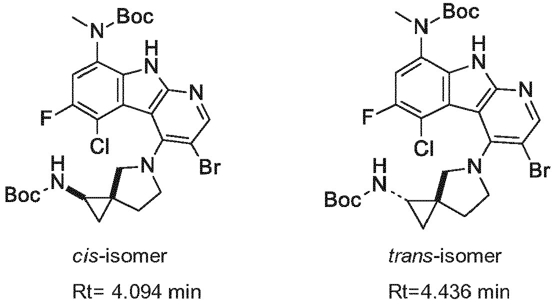

To a solution of tert-butyl (3-bromo-4,5-dichloro-6-fluoro-9H-pyrido[2,3-b]indol-8-yl) (methyl)carbamate (**Intermediate** A3, 900.0 mg, 1.94 mmol as **"CORE"** in table 5), *tert*-butyl 5-azaspiro[2.4]heptan-1-ylcarbamate (500.0 mg, 2.36 mmol) in sulfolane (10.0 mL) was added DIPEA (750.0 mg, 5.80 mmol), then the solution was stirred at 100 °C for 2 h. Then the mixture was poured into water (100 mL), extracted with EtOAc (80 mL) two times. The combined organic layer was dried over anhydrous Na₂SO₄, filtered and concentrated to give a crude material which was purified by prep-HPLC (H₂O (0.225% TFA)-MeCN to give *cis-tert*-butyl *(3-*bromo-4-(1-((tert-butoxycarbonyl)amino)-5-azaspiro[2.4]heptan- 5-yl)-5-chloro-6-fluoro-9H-pyrido[2,3-b]indol-8-yl)(methyl)carbamate (**peak 1**, Rt = 4.094 min, 710 mg, 57.2%) as yellow solid and *trans-tert-*butyl (3-bromo-4-(1-((tert-butoxycarbonyl) amino)-5-azaspiro[2.4]heptan- 5-yl)-5-chloro-6-fluoro-9H-pyrido[2,3-b]indol-8-yl) (methyl)carbamate (**peak 2**, Rt = 4.346 min , 320.0 mg, 25.8%) as yellow solid. MS (ESI): 640.0 [{³⁵Cl+⁸¹Br & ³⁷Cl+⁷⁹Br}(M+H)⁺] (**peak 1**); MS (ESI): 639.9 [{³⁵Cl+⁸¹Br & ³⁷Cl+⁷⁹Br}(M+H)⁺] (**peak 2**).

### Step (b) Preparation of tert-butyl (3-bromo-4-((1S,3R)-1-((tert-butoxycarbonyl) amino)-5-azaspiro[2.4]heptan-5-yl)-5-chloro-6-fluoro-9H-pyrido[2,3-b]indol-8-yl) (methyl)carbamate and tert-butyl (3-bromo-4-((1R,3S)-1-((tert-butoxycarbonyl) amino)-5-azaspiro[2.4]heptan-5-yl)-5-chloro-6-fluoro-9H-pyrido[2,3-b]indol-8-yl) (methyl)carbamate and tert-butyl (3-bromo-4-((1R,3R)-1-((tert-butoxycarbonyl) amino)-5-azaspiro[2.4]heptan-5-yl)-5-chloro-6-fluoro-9H-pyrido[2,3-b]indol-8-yl) (methyl)carbamate and tert-butyl (3-bromo-4-((1S,3S)-1-((tert-butoxycarbonyl) amino)-5-azaspiro[2.4]heptan-5-yl)-5-chloro-6-fluoro-9H-pyrido[2,3-b]indol-8-yl) (methyl)carbamate

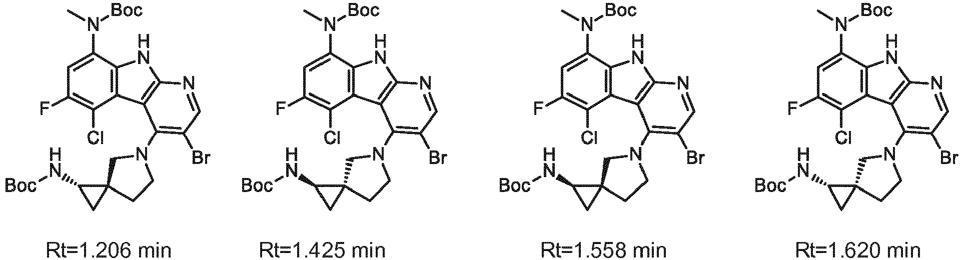

*trans-tert*-butyl (3-bromo-4-(1-((tert-butoxycarbonyl)amino)-5-azaspiro[2.4]heptan- 5-yl)-5-chloro-6-fluoro-9H-pyrido[2,3-b]indol-8-yl)(methyl)carbamate (320.0 mg, 0.500 mmol) was separated by SFC. After SFC separation, the eluent of the first peak was concentrated to give *tert*-butyl (3-bromo-4-((1*S*,3*R*)-1-((*tert*-butoxycarbonyl) amino)-5-azaspiro[2.4]heptan-5-yl)-5-chloro-6-fluoro-9H-pyrido[2,3-b]indol-8-yl) (methyl) carbamate (**peak 1**, Rt=1.206 min, 80.0 mg, Optical Rotation: (-)) as yellow solid and the eluent of the second peak was concentrated to give *tert*-butyl (3-bromo-4-((1*R*,3*S*)-1- *((tert-* butoxycarbonyl)amino)-5-azaspiro[2.4]heptan-5-yl)-5-chloro-6-fluoro-9H- pyrido[2,3-b]indol-8-yl)(methyl)carbamate (**peak 2**, Rt=1.425 min, 78.0 mg, Optical Rotation: (+)) as yellow solid. SFC condition: Column: Daicel Chiralcel AS(250mm × 30mm,10µm); Mobile Phase: 0.1%NHaH₂O MeOH; Flow Rate (mL/min): 70 mL/min.

*cis-tert*-butyl (3-bromo-4-(1-((tert-butoxycarbonyl)amino)-5 -azaspiro [2.4]heptan-5-yl)-5-chloro-6-fluoro-9H-pyrido[2,3-b]indol-8-yl)(methyl)carbamate (710.0 mg, 1.11 mmol) was separated by SFC. After SFC separation, the eluent of the first peak was concentrated to give *tert*-butyl (3-bromo-4-((1*R*,3*R*)-1- ((*tert*-butoxycarbonyl)amino)-5-azaspiro[2.4]heptan-5-yl)-5-chloro-6-fluoro- 9H-pyrido[2,3-b]indol-8-yl)(methyl)carbamate (**peak 3**, Rt=1.558 min, 360.0 mg, Optical Rotation: (-)) as yellow solid and the eluent of the second peak was concentrated to give *tert*-butyl (3-bromo-4-((1*S*,3*S*)-1-((*tert*-butoxycarbonyl)amino)-5-azaspiro[2.4] heptan-5-yl)-5-chloro-6-fluoro-9H-pyrido[2,3-b]indol-8-yl)(methyl)carbamate (**peak 4**, Rt=1.620 min, 300.0 mg, Optical Rotation: (+)) as yellow solid. SFC condition: Column: Daicel Chiralcel OD (250mm × 30mm,10µm); Mobile Phase: 0.1%NH₃H₂O EtOH; Flowrate (mL/min): 50 mL/min.

### Step (c) Preparation of ethyl 7-(8-((tert-butoxycarbonyl)(methyl)amino)-4- ((1S,3R)-1-((tert-butoxycarbonyl)- amino)-5-azaspiror2.41heptan-5-yl)-5-chloro-6- fluoro-9H-pyrido[2,3-b]indol-3-yl)-4-oxo-4H-quinolizine-3-carboxylate

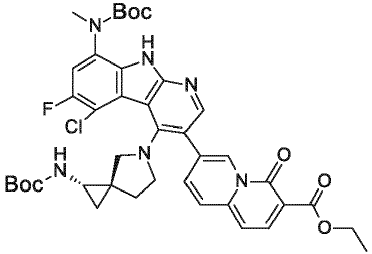

To a solution of compound tert-butyl (3-bromo-4-((1*S*,3*R*)-1-((tert-butoxycarbonyl)amino)-5-azaspiro[2.4]heptan-5-yl)-5- chloro-6-fluoro-9H-pyrido[2,3-b]indol-8-yl)(methyl)carbamate (30.0 mg, 0.047 mmol), ethyl 4-oxo-7-(4,4,5,5-tetramethyl-1,3,2-dioxaborolan-2-yl)-4H-quinolizine -3-carboxylate (38.0 mg, 0.077 mmol, as **"BORONIC REAGENT"** in table 5 ) in THF/H₂O (1.5 mL/0.1 mL, as **"SOLVENT"** in table 5) was added Pd-Ad₂nBuP Biphenyl Precat (5.0 mg, 0.007 mmol, as **"CATALYST"** in table 5) and K₃PO₄ (40.0 mg, 0.212 mmol), then the solution was stirred at 60 °C under Ar for 18 h. The reaction mixture was poured into water (20 mL), extracted with EtOAc (15 mL) two times. The combined organic layer was washed with brine (15 mL) two times, dried over anhydrous Na₂SO₄, filtered and concentrated to give a residual, which was purified by prep-TLC with EtOAc: MeOH=20:1 to give ethyl 7-(8-((*tert*-butoxycarbonyl)(methyl)amino)-4- ((1*S*,3*R*)-1-((*tert*-butoxycarbonyl)-amino)-5-azaspiro[2.4]heptan-5-yl)-5-chloro- 6-fluoro-9H-pyrido[2,3-b]indol-3-yl)-4-oxo-4H-quinolizine-3-carboxylate (25.0 mg, yield: 68.7%) as yellow solid. MS (ESI): 775.2 [{³⁵Cl} (M+H)⁺].

### Step (d) Preparation of 7-(8-((tert-butoxycarbonyl)(methyl)amino)-4-((1S,3R)-1-((tert-butoxycarbonyl)amino)-5-azaspiro[2.4]heptan-5-yl)-5-chloro-6-fluoro-9H-pyrido[2,3-b]indol-3-yl)-4-oxo-4H-quinolizine-3-carboxylic acid

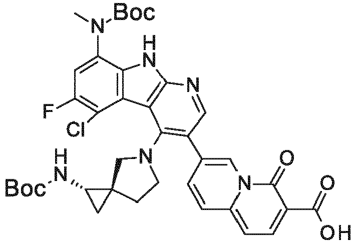

To a solution of 7-(8-((*tert*-butoxycarbonyl)(methyl)amino)-4-((1*S*,3*R*)-1-((*tert-*butoxycarbonyl)- amino)-5-azaspiro[2.4]heptan-5-yl)-5-chloro-6-fluoro-9H- pyrido[2,3-b]indol-3-yl)-4-oxo-4H-quinolizine-3-carboxylate (25.0 mg, 0.032 mmol) in EtOH (5.0 mL) was added NaOH (aq. 1 N, 2.0 mL), then the solution was stirred at 20 °C for 18 h. Then the mixture was concentrated to remove organic solvents and the residual was acidified with 1 N HCl to pH = 5~6, then extracted with EtOAc (30 mL) two times. The combined EtOAc layer was washed with brine (30 mL) three times, dried over anhydrous Na₂SO₄, filtered and concentrated to give 7-(8-((*tert*-butoxycarbonyl) (methyl)amino)- 4-((1*S*,3*R*)-1-((*tert*-butoxycarbonyl)-amino)-5-azaspiro[2.4]heptan- 5-yl)-5-chloro-6- fluoro-9H-pyrido[2,3-b]indol-3-yl)-4-oxo-4H-quinolizine-3- carboxylic acid (25.0 mg, crude) as yellow solid.
MS (ESI): 747.2 [{³⁵Cl} (M+H)⁺].

### Step (e) Preparation of 7-(4-((1S,3R)-1-amino-5-azaspiro[2.4]heptan-5-yl)-5-chloro-6-fluoro-8-(methylamino)-9H-pyrido[2.3-b]indol-3-yl)-4-oxo-4H-quinolizine-3-carboxylic acid (Example 5.01A)

To a solution of 7-(8-((*tert*-butoxycarbonyl)(methyl)amino)-4-((1*S*,3*R*)-1-((tert-butoxycarbonyl)- amino)-5-azaspiro[2.4]heptan-5-yl)-5-chloro-6-fluoro-9H- pyrido[2,3-b]indol-3-yl)-4-oxo-4H-quinolizine-3-carboxylic acid (25.0 mg, 0.032 mmol) in DCM (3.0 mL) was added TFA (0.5 mL). After stirred at 20 °C for 2 h, the reaction mixture was concentrated to give a crude material, which was dissolved in MeCN (3 mL) and purified by prep-HPLC (water(1% TFA)-ACN) to give 7-(4-((1S,3R)-1-amino-5-azaspiro[2.4]heptan-5-yl)-5-chloro-6-fluoro-8-(methyl- amino)-9H-pyrido[2,3-b]indol-3-yl)-4-oxo-4H-quinolizine-3-carboxylic acid (5.2 mg, 21.6%). MS (ESI): 547.1 [{³⁵Cl}(M+H)⁺]. ¹H NMR (400 MHz, DMSO) *δ* ppm: 14.17 (brs, 1H), 11.85 (s, 1H), 9.22 (s, 1H), 8.48 (s, 1H), 8.20-8.06 (m, 6H), 7.38-7.36 (d, 1H), 6.64-6.61 (d, 1 H), 6.06 (brs, 1H), 3.27 (s, 3H), 2.91 (s, 3H), 2.52 (m, 2H), 1.92 (m, 2H), 0.83 (m, 2H).

The following examples were prepared in analogy to **Example 5.01A,** replacing *tert*-butyl (3-bromo-4,5-dichloro-6-fluoro-9H-pyrido[2,3-b]indol-8-yl) (methyl) carbamate (**Intermediate A3**) with the "**CORE**" in step (a), THF/H₂O with the "**SOLVENT**" in step (c), Ad₂nBuP Biphenyl with the "**CATALYST**" in step (c) and ethyl 4-oxo-7-(4,4,5,5-tetramethyl-1,3,2-dioxaborolan-2-yl)quinolizine-3-carboxylate with the "**BORONIC REAGENT**" in step (c) by the reagents indicated in Table 5.

**Table 5. Compound synthesis and characterization**

| **No.** | **Compound Name and Structure** | **CORE, AMINE, BROMIDE REAGENT, CATALYST and SLOVENT** | **¹H NMR and MS (ESI)** |
|---|---|---|---|
| 5.01 B | **7-(4-((1*R*,3*S*)-1-amino-5-azaspiro[2.4]heptan-5-yl)-5-chloro-6-fluoro-8-(methylamino)-9H-pyrido[2,3-b]indol-3-yl)-4-oxo-4H-quinolizine-3-carboxylic acid** | **CORE:** Intermediate A3 | ¹H NMR (400 MHz, DMSO) *δ* ppm: 14.15 (brs, 1H), 11.89 (s, 1H), 9.22 (s, 1H), 8.46 (s, 1H), 8.20-8.06 (m, 6H), 7.38-7.36 (d, 1H), 6.64-6.61 (d, 1 H), 6.03 (brs, 1H), 3.27 (m, 3H), 2.91 (s, 3H), 2.52 (m, 2H), 1.92 (m, 2H), 1.05-0.83 (m, 2H). |
| | | **AMINE:** *tert*-butyl 5-azaspiro[2.4]heptan-1-ylcarbamate | |
| | | **BROMIDE REAGENT:** Intermediate B1 | |
| | 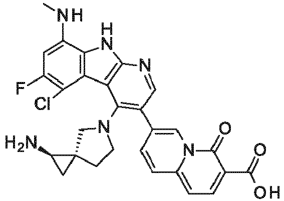 | **CATALYST:** Pd-Ad₂nBuP Biphenyl Precat (CAS#: 1375477-29-4) | |
| | | | MS (ESI): 547.1 |
| | | **SOLVENT:** THF/H₂O | [{³⁵Cl}(M+H)⁺]. |
| 5.01 C | **7-(4-((1*R*,3*R*)-1-amino-5-azaspiro[2.4]heptan-5-yl)-5-chloro-6-fluoro-8-(methylamino)-9H-pyrido[2,3-b]indol-3-yl)-4-oxo-4H-quinolizine-3-carboxylic acid** | **CORE:** Intermediate A3 | ¹H NMR (400 MHz, DMSO-*d*₆) δ ppm: 11.91 (s, 1H), 9.27 (s, 1H), 8.48 (d, 1H), 8.22-8.25 (m, 2H), 8.08 (m, 1H), 7.95 (brs, 2H), 7.39 (d, 1H), 6.64 (d, 1H), 2.92 (s, 3H), 2.62 (m, 2H), 2.52 (m, 5H), 1.70 (m, 1H), 0.98 (m, 1H). |
| | | **AMINE:** *tert*-butyl 5-azaspiro[2.4]heptan-1-ylcarbamate | |
| | | **BROMIDE REAGENT:** Intermediate B1.4 | |
| | 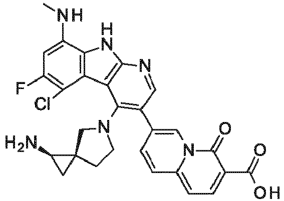 | **CATALYST:** Pd-Ad₂nBuP Biphenyl Precat (CAS#: 1375477-29-4) | |
| | | | MS (ESI): 547.4 |
| | | **SOLVENT:** THF/H₂O | [{³⁵Cl}(M+H)⁺] . |
| 5.01 D | **7-(4-((1*S*,3*S*)-1-amino-5-azaspiro[2.4]heptan-5-yl)-5-chloro-6-fluoro-8**- | **CORE:** Intermediate A3 | ¹H NMR (400 MHz, DMSO-*d*₆) δ ppm 14.09 (s, 1H), 11.91 (s, 1H), 9.26 (s, |
| | | **AMINE:** *tert*-butyl 5-azaspiro[2.4]heptan-1- | |
| | **(methylamino)-9H-pyrido[2,3-b]indol-3-yl)-4-oxo-4H-quinolizine-3-carboxylic acid** | ylcarbamate | 1H), 8.48 (d, 1H), 8.19-8.26 (m, 2H), 8.08 (m, 1H), 7.95 (brs, 3H), 7.39 (d, 1H), 6.64 (d, 1H), 3.06-3.25 (m, 2H), 2.92 (s, 3H), 2.62 (m, 2H), 2.52 (m, 3H), 1.70 (m, 1H), 0.98 (m, 1H). |
| | | **BROMIDE REAGENT:** Intermediate B1.4 | |
| | 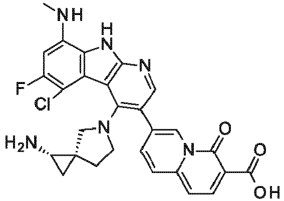 | **CATALYST:** Pd-Ad₂nBuP Biphenyl Precat (CAS#: 1375477-29-4) | |
| | | | MS (ESI): 547.2 |
| | | **SOLVENT:** THF/H₂O | [{³⁵Cl}(M+H)⁺]. |

### BIOLOGICAL EXAMPLES

### Example 6

### 50% Growth Inhibitory Concentration (IC₅₀) Determination Assay:

The in vitro antimicrobial activity of the compounds against *S. aureus* (ATCC29213), *K. pneumoniae* (ATCC10031), and *A. baumannii* (ATCC17978), was determined according to the following procedure:
The assay used a 10-points Iso-Sensitest broth medium to measure quantitatively the in vitro activity of the compounds against *S. aureus* ATCC29213, *K. pneumoniae* ATCC 10031, and *A*. *baumannii* ATCC17978.

Stock compounds in DMSO were serially two-fold diluted (range from 50 to 0.097 µM final concentration) in 384 wells microtiter plates and inoculated with 49 µL the bacterial suspension in Iso-Sensitest broth medium to have a final cell concentration of ~ 5 × 10⁵ CFU/mL in a final volume/well of 50 µL/well. Microtiter plates were incubated at 35 ± 2 °C.

Bacterial cell growth was determined with the measurement of optical density at λ=600nm each 20 minutes over a time course of 16h.

Growth inhibition was calculated during the logarithmic growth of the bacterial cells with determination of the concentration inhibiting 50% (IC₅₀) of the growth.

GP-6 (Example 4.131 disclosed in WO 2012/125746 A1) was used as the reference compound in Table 6-8.

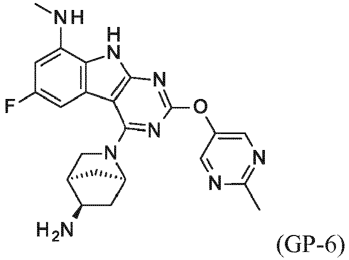

Compounds of the present invention were tested for their concentration inhibiting 50% (IC₅₀). The data of IC₅₀ over *S. aureus* (ATCC29213), *K. pneumoniae* (ATCC10031), and *A. baumannii* (ATCC 17978) are illustrated in Table 6. Particular compounds of the present invention were found to have IC₅₀ ≤ 1 µM.

**Table 6: IC₅₀ values of the compounds of this invention against S. aureus, K. pneumoniae and A. baumannii**

| **Example No.** | **IC50 (µM)** | | |
|---|---|---|---|
| | *S. aureus* ATCC29213 | *K. pneumoniae* ATCC10031 | *A. baumannii* ATCC 17978 |
| **GP-6** | <0.098* | <0.098* | <0.098* |
| **1.01** | <0.098* | <0.098* | <0.098* |
| **1.02** | 0.159 | <0.098* | <0.098* |
| **1.03** | <0.098* | <0.098* | <0.098* |
| **1.04** | <0.098* | <0.098* | <0.098* |
| **1.06** | 0.137* | <0.098* | <0.098* |
| **1.07** | <0.098* | <0.098* | <0.098* |
| **1.08** | <0.098* | <0.098* | <0.098* |
| **1.09** | <0.098* | <0.098* | <0.098* |
| **1.10** | <0.098* | <0.098* | <0.098* |
| **1.11** | <0.098* | <0.098* | <0.098* |
| **1.12** | <0.098* | <0.098* | <0.098* |
| **1.13** | <0.098* | <0.098* | <0.098* |
| **1.14** | <0.098* | <0.098* | <0.098* |
| **1.15** | 0.103 | <0.098* | <0.098* |
| **1.16** | <0.098* | <0.098* | <0.098* |
| **1.17** | 0.139 | <0.098* | <0.098* |
| **1.19** | 0.185 | <0.098* | <0.098* |
| **2.01** | <0.098* | <0.098* | <0.098* |
| **2.02** | 0.108 | <0.098* | <0.098* |
| **2.03** | 0.194 | <0.098* | <0.098* |
| **2.04** | <0.098* | <0.098* | <0.098* |
| **2.05** | <0.098* | <0.098* | <0.098* |
| **2.07** | 0.379 | <0.098* | 0.242 |
| **2.08** | 0.433 | <0.098* | 0.132 |
| **2.09** | 0.174 | <0.098* | <0.098* |
| **2.11** | <0.098* | <0.098* | <0.098* |
| **3.01A** | <0.098* | <0.098* | <0.098* |
| **3.01B** | <0.098* | <0.098* | <0.098* |
| **3.02A** | 0.102 | <0.098* | <0.098* |
| **3.02B** | 1.282 | <0.098* | 0.483 |
| **3.03A** | <0.098* | <0.098* | <0.098* |
| **3.03B** | <0.098* | <0.098* | <0.098* |
| **3.04A** | <0.098* | <0.098* | <0.098* |
| **3.04B** | <0.098* | <0.098* | <0.098* |
| **4.01A** | <0.098* | <0.098* | <0.098* |
| **4.01B** | <0.098* | <0.098* | <0.098* |
| **4.01C** | <0.098* | <0.098* | <0.098* |
| **4.01D** | <0.098* | <0.098* | <0.098* |
| **5.01C** | <0.098* | <0.098* | <0.098* |
| **5.01D** | <0.098* | <0.098* | <0.098* |

| | | | |
|---|---|---|---|
| "*": the detection limit. | | | |

### Example 7

### Lyophilisation Solubility Assay (LYSA):

The solubility of compounds of present invention was assessed by LYSA assay. Samples were prepared in duplicate from 10 mM DMSO stock solutions (20µL) diluted in 30µL pure DMSO. After evaporation of DMSO with a centrifugal vacuum evaporator, the residue was dissolved in 0.05 M phosphate buffer (150µL, pH 6.5), which was stirred for one hour and shook for two hours. After one night, the solution was filtered using a microtiter filter plate. Then the filtrate and its 1/10 dilution were analyzed by HPLC-UV. In addition a four-point calibration curve was prepared from the 10 mM stock solutions and used for the solubility determination of the compounds. The results were in µg/mL and summarized in Table 7. Particular compounds of the present invention were found to have LYSA > 10 µg/mL with much improved solubility compared to GP-6.

**Table 7: Solubility data of Examples**

| **Example No.** | **Lysa (µg/mL)** | **Example No.** | **Lysa (µg/mL)** |
|---|---|---|---|
| **GP-6** | <1.0* | **2.06** | 7 |
| **1.03** | 134 | **2.07** | 48 |
| **1.04** | 460 | **2.08** | 54 |
| **1.10** | 90 | **2.09** | 795 |
| **1.17** | 12 | **2.10** | >940 |
| **1.19** | 29 | **3.01A** | 9 |
| **2.01** | 41 | **3.01B** | 16 |
| **2.02** | 58 | **3.02A** | 559 |
| **2.03** | >1065 | **3.02B** | >683 |

| | | | |
|---|---|---|---|
| "*": the detection limit. | | | |

### Example 8

### Cytotoxicity assay:

The cytotoxicity of compounds of present invention was assessed by HepG2 assay. HepG2 cells (ATCC HB-8065^{™}) were seeded into 96-well plates (1.2 ×10⁴ cells per well) and treated with compounds for 3 days, cell viability was measured at day 3 post compound treatment using the CellTiter-Blue^{®} Cell Viability Assay (Promega, Cat. No. G8082). 20µL of Cell titer blue buffer were added to each well of the cells, which were incubated for 3 hours at 37°C, and the fluorescence value was measured by a plate reader (Molecular Device SpectraMax M2). The CC₅₀ value of each compound is plotted with GraphPad Prism using four parameter logistic equation. Results of cytotoxicity are given in Table 8.

**Table 8: CC₅₀ of Examples**

| **Example No.** | **CC₅₀ (µM)** | **Example No.** | **CC₅₀ (µM)** |
|---|---|---|---|
| **GP-6** | 5.6 | **2.10** | 89.2 |
| **1.02** | 24.1 | **3.02A** | 41.3 |
| **1.03** | 21.9 | **3.04B** | 10.8 |
| **1.04** | 15.0 | **4.01B** | >100* |
| **1.05** | 15.5 | **4.01C** | >100* |
| **1.12** | >100* | **4.01D** | >100* |
| **1.14** | 15.3 | **5.01A** | >100* |
| **1.15** | 75.4 | **5.01B** | >100* |
| **2.03** | 46.4 | **5.01C** | >100* |
| **2.09** | 86.9 | **5.01D** | >100* |

| | | | |
|---|---|---|---|
| "*": the detection limit. | | | |

### Example 9

### Single dose PK study (SDPK) in Female CD-1 mouse:

The single dose PK in female CD-1 mouse was performed to assess compound's pharmacokinetic properties. One group of animals were dosed via bolus intravenous (IV) of the respective compound. Blood samples (approximately 30 µL) were collected via saphenous vein at 5 mins, 15 min, 30 min, 1hr, 2hr, 4hr and 7hr. For last time point (24hr), samples were collected via cardiac puncture while the mouse was under anesthesia. Blood samples were placed into tubes containing EDTA-K2 anticoagulant (2 µL of 0.5M per tube) and centrifuged at 3000 rpm for 10 minutes at 4°C within half an hour to separate plasma from the samples. Following centrifugation, plasma samples were stored in polypropylene tubes, quick frozen over dry ice and kept at -70±10 °C until LC/MS/MS analysis. The pharmacokinetic parameters were calculated using non-compartmental module of WinNonlin^{®} Professional 6.4. Results of SDPK assay are given in Table 9.

**Table 9: SDPK data of Examples**

| **Example No.** | **PK Conditions: MOUSE IV** | **CL total (mL/min/kg)** | **AUC total (h×ng/mL)** |
|---|---|---|---|
| **GP-6** | 1.0mg/kg-Solution | 132.1 | 126.2 |
| **1.03** | 1.0mg/kg-Solution | 39.1 | 374.7 |
| **1.04** | 1.0mg/kg-Solution | 64.1 | 256 |
| **1.23** | 1.0mg/kg-Solution | 82.4 | 204 |
| **1.14** | 1.0mg/kg-Solution | 78.7 | 214 |
| **5.01D** | 1.0mg/kg-Solution | 93.0 | 180 |

### Example 10

### Minimal Inhibitory Concentration (MIC):

The MIC of antibacterial compounds against multiple species and strains was determined using the broth microdilution method according to M07-A10 Clinical and Laboratory Standards Institute (CLSI) guidelines (CLSI (2015) Methods for dilution antimicrobial susceptibility tests for bacteria that grow aerobically; Approved Standard, Tenth Edition. CLSI Document M7-A10 (ISBN 1-56238-987-4). Wayne, PA: Clinical and Laboratory Standards Institute, 19087, USA.). In short, bacterial strain preparation was performed on D1 and D2. The bacterial strains were revived from storage frozen two days before the MIC screening, and streaked onto surface of agar plates, incubated in an ambient-air incubator with humidity for 20-24 hr at 35±2°C. 5-10 well-isolated colonies of similar morphology were selected and re-streaked onto fresh agar plates using sterile loops and incubated for 20-24 hr at 35±2°C.. Bacterial strains were subcultured two times to ensure fresh inoculums for MIC screening. On the day of MIC assay (Day 3), colonies were transferred from fresh culture plates into 5 mL of saline with sterile loops and mixed well, which was measured and adjusted turbidity to 0.5 McFarland barium sulfate standard (1-2×10⁸ Colony Forming Units (CFU)/mL) using a turbidity meter. Alternatively, 1-2 colonies were transferred into 500 µL of saline and OD600 was adjusted to ~0.1 using an OD meter. Bacterial inoculum was diluted by 1:280 for Gram-positive strains and 1:400 for Gram-negative strains into corresponding medium broth (CAMHB, CAMHB+3% lysed horse blood, HTM) (e.g. 35.6 µL of inoculum into 10 mL of CAMHB or 25 µL of inoculum into 10 mL of CAMHB). For compound plate, the compound stock solutions were prepared in 100% DMSO on the day of MIC screening and used immediately. Compound stock concentration = [(highest testing concentration) × 100 µL / 2 µL] (e.g. if the required highest testing concentration is 64 µg/mL in assay plates, stock concentration = 64 µg/mL × 100 µL / 2 µL =3.2 mg/mL). The compound plates were prepared by dispensing 100µL of each stock solution in the first well of a 96 well microtiter plate (MTP) at a concentration 100-fold higher than the final concentration desired in broth. Eleven serial 2-fold dilutions of the highest concentration were made in DMSO for new compounds and for reference compounds (GP-6 and ciprofloxacin). 1µL of each well was transferred in a new MTP, which served as the test plate by subsequent inoculation. The bacterial suspension prepared above is dispensed at 98µL/well within 15 minutes after preparation. Negative controls (lack of bacterial cells) and growth control wells (lack of compound) were included in all plates. MTPs were incubated for 18-24 hours at 35±2°C in ambient air. The MIC was recorded and CFUs were calculated on Day 4. The assay plate was placed on the top of MIC reader, and the magnification mirror was adjusted to read each wells, recording growth status as raw data. Photo images of each assay plates were recorded using Q Count 530(Spiral Biotech).. The MIC value of each compound, expressed as µg/mL, was determined as the lowest concentration required for complete growth inhibition (no visible growth). Results of MIC were summarized in Table 10.

**Table 10. MIC of antibacterial compounds against multiple species**

| **Ex.** | **S1** | **S2** | **S3** | **S4** | **S5** | **S6** |
|---|---|---|---|---|---|---|
| **Cip** | 2 | 16 | 0.5 | <0.062 | 0.5 | >64 |
| **GP-6** | 0.339 | 0.339 | 0.169 | <0.021 | 1.358 | 2.715 |
| **1.01** | 0.057 | 0.450 | 0.225 | 0.057 | 0.450 | 1.805 |
| **1.03** | 0.260 | 0.260 | 0.130 | 0.030 | 4.120 | 2.060 |
| **1.04** | 0.128 | 0.128 | 0.128 | 0.032 | 1.029 | 1.029 |
| **1.07** | 0.066 | 0.066 | 0.263 | 0.033 | 1.053 | 0.527 |
| **1.08** | 0.274 | 0.274 | 0.548 | 0.069 | 4.394 | 2.197 |
| **1.11** | 0.260 | 0.260 | 0.260 | 0.033 | 1.038 | 1.038 |
| **1.12** | 1.082 | 0.541 | 0.541 | 0.068 | 2.168 | 4.335 |
| **1.14** | 0.108 | 0.215 | 0.215 | 0.027 | 1.724 | 0.860 |
| **1.16** | 0.431 | 0.216 | 0.216 | 0.027 | 1.724 | 1.724 |
| **1.17** | 0.882 | 0.882 | 0.882 | 0.028 | 3.535 | 3.535 |
| **1.18** | 0.092 | 0.367 | 0.184 | 0.046 | 0.092 | 0.735 |
| **1.20** | 0.187 | 1.492 | 0.746 | 0.187 | 1.492 | 2.984 |
| **1.21** | 0.603 | 1.208 | 1.208 | 0.038 | 4.829 | 4.829 |
| **2.04** | 0.461 | 0.614 | 0.922 | 0.077 | 4.916 | 4.916 |
| **2.11** | 0.270 | 0.541 | 0.541 | 0.068 | 4.331 | 4.331 |
| **3.03A** | 0.106 | 0.212 | 0.212 | 0.027 | 3.404 | 1.702 |
| **3.04A** | 0.218 | 0.218 | 0.218 | 0.027 | 1.746 | 1.746 |
| **3.04B** | 0.436 | 0.436 | 0.436 | 0.027 | 1.746 | 1.746 |
| **4.01A** | 0.445 | 0.445 | 0.592 | 0.074 | 2.371 | 2.371 |
| **4.01C** | 0.592 | 0.592 | 1.186 | 0.074 | 2.371 | 2.371 |
| **4.01D** | 0.592 | 1.186 | 1.186 | 0.037 | 4.741 | 4.741 |
| **5.01A** | 1.031 | 1.031 | 1.031 | 0.032 | 2.066 | 4.131 |
| **5.01B** | 0.516 | 1.031 | 1.031 | 0.032 | 4.131 | 4.131 |
| **5.01C** | 0.516 | 0.258 | 0.516 | 0.032 | 2.066 | 2.066 |

| **Ex.** | **S7** | **S8** | **S9** | **S10** | **S11** | |
|---|---|---|---|---|---|---|
| **Cip** | 0.5 | 2 | <0.062 | >64 | | |
| **GP-6** | 0.678 | 1.358 | 0.169 | 1.358 | 0.127 | |
| **1.01** | 0.901 | 0.901 | 0.113 | 0.225 | 0.169 | |
| **1.03** | 1.030 | 2.060 | 0.510 | 2.060 | 0.193 | |
| **1.04** | 0.514 | 2.058 | 0.128 | 0.258 | 0.048 | |
| **1.07** | 0.527 | 8.438 | 0.132 | 0.132 | 0.049 | |
| **1.08** | 2.197 | 4.394 | 0.548 | 0.548 | 0.206 | |
| **1.11** | 1.038 | 2.080 | 0.260 | 0.260 | 0.097 | |
| **1.12** | 1.082 | 2.168 | 0.541 | 0.541 | 0.203 | |
| **1.14** | 0.860 | 1.724 | 0.108 | 0.215 | 0.040 | |
| **1.16** | 0.431 | 3.447 | 0.216 | 0.216 | 0.040 | |
| **1.17** | 0.882 | 3.535 | 0.882 | 0.882 | 0.166 | |
| **1.18** | 0.367 | 0.184 | 0.046 | 0.046 | 0.551 | |
| **1.20** | 2.984 | 2.238 | 0.559 | 0.559 | 0.420 | |
| **1.21** | 1.208 | 2.414 | 0.603 | 0.302 | 0.340 | |
| **2.04** | 2.458 | 3.687 | 0.614 | 0.922 | 0.173 | |
| **2.11** | 2.166 | 4.331 | 1.081 | 1.081 | 0.203 | |
| **3.03A** | 0.850 | 3.404 | 0.425 | 0.425 | 0.080 | |
| **3.04A** | 0.436 | 3.491 | 0.218 | 0.218 | 0.082 | |
| **3.04B** | 0.873 | 1.746 | 0.436 | 1.746 | 0.164 | |
| **4.01A** | 0.592 | 1.186 | 0.297 | 0.297 | 0.111 | |
| **4.01C** | 1.186 | 2.371 | 0.592 | 1.186 | 0.222 | |
| **4.01D** | 1.186 | 2.371 | 1.186 | 0.592 | 0.111 | |
| **5.01A** | 2.066 | 2.066 | 0.516 | 0.516 | 0.194 | |
| **5.01B** | 2.066 | 2.066 | 0.516 | 0.516 | 0.097 | |
| **5.01C** | 1.031 | 2.066 | 0.258 | 0.258 | 0.194 | |

| | | | | | | |
|---|---|---|---|---|---|---|
| Ex.: Example Number Cip: Ciprofloxacin S1: *A. baumannii* ATCC 19606 S2: *A. baumannii* ATCC 51432 S3: *A. baumannii* ATCC BAA-747 S4: *K. pneumoniae* ATCC 10031 S5: *K. pneumoniae* ATCC 700603 S6: *K. pneumoniae* ATCC BAA-2146 S7: *P. aeruginosa* ATCC 27853 S8: *P. aeruginosa* ATCC BAA-2113 S9: *E. coli* ATCC 35218 S10: *E. coli* ATCC BAA-2340 S11: *E. coli* ATCC 25922 | | | | | | |

## Claims

1. A compound of formula (I), 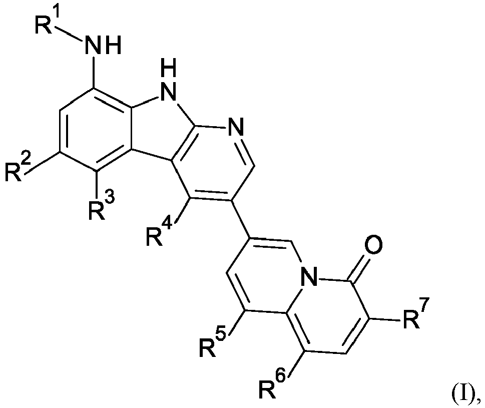 wherein
R¹ is C₁₋₆alkyl;
R² is halogen;
R³ is H, halogen or cyano;
R⁴ is (C₁₋₆alkyl)₂amino or heterocyclyl;
R⁵ is H, halogen or C₁₋₆alkyl;
R⁶ is H, ((C₁₋₆alkyl)zamino)C₁₋₆alkyl, C₁₋₆alkyl, C₃₋₇cycloalkyl, haloC₁₋₆alkyl, halogen, morpholinylC₁₋₆alkyl or pyrrolidinylC₁₋₆alkyl;
R⁷ is carboxy;
or a pharmaceutically acceptable salt, or enantiomer or diastereomer thereof.

2. A compound of formula (I) according to claim 1, wherein
R¹ is C₁₋₆alkyl;
R² is halogen;
R³ is H, halogen or cyano;
R⁴ is (C₁₋₆alkyl)₂amino;
2,3,3a,4,6,6a-hexahydrofuro[2,3-c]pyrrolyl substituted by (C₁₋₆alkyl)₂amino;
2,3,3a,4,6,6a-hexahydropyrrolo[2,3-c]pyrrolyl substituted by C₁₋₆alkyl;
2,3,3a,4,6,6a-hexahydropyrrolo[3,4-b]pyrrolyl substituted by C₁₋₆alkyl;
2,3,4a,5,7,7a-hexahydropyrrolo[3,4-b][1,4]oxazinyl substituted by C₁₋₆alkyl;
3,3a,4,5,6,6a-hexahydro-1H-cyclopenta[c]pyrrolyl substituted by (C₁₋₆alkyl)₂amino;
3,3a,4,5,7,7a-hexahydro-2H-pyrrolo[2,3-c]pyridinyl substituted by C₁₋₆alkyl;
3,4,6,6a-tetrahydro-2H-pyrrolo[2,3-c]pyrrolyl substituted twice by C₁₋₆alkyl;
3-azabicyclo[3.1.0]hexanyl substituted by (C₁₋₆alkyl)₂amino;
5-azaspiro[2.4]heptanyl substituted by amino;
aminoC₃₋₇cycloalkylpyrrolidinyl;
morpholinyl, said morpholinyl being unsubstituted or substituted by ((C₁₋₆alkyl)zamino)C₁₋₆alkyl;
R⁵ is H, halogen or C₁₋₆alkyl;
R⁶ is H, ((C₁₋₆alkyl)zamino)C₁₋₆alkyl, C₁₋₆alkyl, C₃₋₇cycloalkyl, haloC₁₋₆alkyl, halogen, morpholinylC₁₋₆alkyl or pyrrolidinylC₁₋₆alkyl;
R⁷ is carboxy;
or a pharmaceutically acceptable salt, or enantiomer or diastereomer thereof.

3. A compound according to claim 2, wherein
R¹ is methyl;
R² is chloro or fluoro;
R³ is H, chloro, fluoro or cyano;
R⁴ is dimethylamino; dimethylamino-2,3,3a,4,6,6a-hexahydrofuro[2,3-c]pyrrolyl; methyl-2,3,3a,4,6,6a-hexahydropyrrolo[2,3-c]pyrrolyl; methyl-2,3,3a,4,6,6a-hexahydropyrrolo[3,4-b]pyrrolyl; methyl-2,3,4a,5,7,7a-hexahydropyrrolo[3,4-b][1,4]oxazinyl; dimethylamino-3,3a,4,5,6,6a-hexahydro-1H-cyclopenta[c]pyrrolyl; methyl-3,3a,4,5,7,7a-hexahydro-2H-pyrrolo[2,3-c]pyridinyl; dimethyl-3,4,6,6a-tetrahydro-2H-pyrrolo[2,3-c]pyrrolyl; dimethylamino-3-azabicyclo[3.1.0]hexanyl; amino-5-azaspiro[2.4]heptanyl; aminocyclopropylpyrrolidinyl; morpholinyl or ((dimethylamino)methyl)morpholinyl;
R⁵ is H, fluoro or methyl;
R⁶ is H, (dimethylamino)methyl, methyl, cyclopropyl, trifluoromethyl, chloro, morpholinylmethyl or pyrrolidinylmethyl;
R⁷ is carboxy;
or a pharmaceutically acceptable salt, or enantiomer or diastereomer thereof.

4. A compound according to claim 2, wherein R⁴ is 2,3,3a,4,6,6a-hexahydropyrrolo[2,3-c]pyrrolyl substituted by C₁₋₆alkyl; 2,3,3a,4,6,6a-hexahydropyrrolo[3,4-b]pyrrolyl substituted by C₁₋₆alkyl; or 3,3a,4,5,6,6a-hexahydro-1H-cyclopenta[c]pyrrolyl substituted by (C₁₋₆alkyl)₂amino.

5. A compound according to claim 4, wherein R⁴ is methyl-2,3,3a,4,6,6a-hexahydropyrrolo[2,3-c]pyrrolyl; methyl-2,3,3a,4,6,6a-hexahydropyrrolo[3,4-b]pyrrolyl; or dimethylamino-3,3a,4,5,6,6a-hexahydro-1H-cyclopenta[c]pyrrolyl.

6. A compound according to claim 2, wherein
R¹ is C₁₋₆alkyl;
R² is halogen;
R³ is H, halogen or cyano;
R⁴ is 2,3,3a,4,6,6a-hexahydropyrrolo[2,3-c]pyrrolyl substituted by C₁₋₆alkyl;
2,3,3a,4,6,6a-hexahydropyrrolo[3,4-b]pyrrolyl substituted by C₁₋₆alkyl; or
3,3a,4,5,6,6a-hexahydro-1H-cyclopenta[c]pyrrolyl substituted by (C₁₋₆alkyl)₂amino;
R⁵ is H;
R⁶ is H;
R⁷ is carboxy;
or a pharmaceutically acceptable salt, or enantiomer or diastereomer thereof.

7. A compound according to claim 6, wherein
R¹ is methyl;
R² is chloro or fluoro;
R³ is H, chloro, fluoro or cyano;
R⁴ is methyl-2,3,3a,4,6,6a-hexahydropyrrolo[2,3-c]pyrrolyl;
methyl-2,3,3a,4,6,6a-hexahydropyrrolo[3,4-b]pyrrolyl; or
dimethylamino-3,3a,4,5,6,6a-hexahydro-1H-cyclopenta[c]pyrrolyl;
R⁵ is H;
R⁶ is H;
R⁷ is carboxy;
or a pharmaceutically acceptable salt, or enantiomer or diastereomer thereof.

8. A compound according to any one of claims 1 or 2, selected from
7-(4-(dimethylamino)-5,6-difluoro-8-(methylamino)-9H-pyrido[2,3-b]indol-3-yl)-4-oxo-4H-quinolizine-3-carboxylic acid;
7-(6-fluoro-8-(methylamino)-4-morpholino-9H-pyrido[2,3-b]indol-3-yl)-4-oxo-4H-quinolizine-3-carboxylic acid;
7-[5, 6-difluoro-4-(*cis*-5-methyl-2,3,3 a,4,6, 6a-hexahydropyrrolo[2,3 -c]pyrrol-1-yl)-8-(methylamino)-9H-pyrido[2,3-b]indol-3-yl]-4-oxo-quinolizine-3-carboxylic acid;
7-[4-[(3a*S*,6a*S*)-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-4-oxo- quinolizine-3-carboxylic acid;
7-[4-[*cis*-(4R)-4-(dimethylamino)-3,3 a,4, 5,6, 6a-hexahydro-1H-cyclopenta[c]pyrrol-2-yl]-5,6-difluoro-8-(methylamino)-9H-pyrido[2,3-b]indol-3-yl]-4-oxo-quinolizine-3-carboxylic acid;
7-[4-[*cis*-3-(dimethylamino)-2,3,3a,4,6,6a-hexahydrofuro[2,3-c]pyrrol-5-yl]-5,6-difluoro-8-(methylamino)-9H-pyrido[2,3-b]indol-3-yl]-4-oxo-quinolizine-3-carboxylic acid;
7-(5-chloro-6-fluoro-8-(methylamino)-4-((3a*S*,6a*S*)-5-methyl-2,3,3a,4,6,6a-hexahydropyrrolo[2,3-c]pyrrol-1-yl)-9H-pyrido[2,3-b]indol-3-yl)-4-oxo-4H-quinolizine-3-carboxylic acid;
7-[4-[*cis*-(4R)-4-(dimethylamino)-3,3 a,4, 5,6, 6a-hexahydro-1H-cyclopenta[c]pyrrol-2-yl]-5-chloro-6-fluoro-8-(methylamino)-9H-pyrido[2,3-b]indol-3-yl]-4-oxo-quinolizine-3-carboxylic acid;
7-[4-[*cis*-(4*S*)-4-(dimethylamino)-3,3a,4,5,6,6a-hexahydro-1H-cyclopenta[c]pyrrol-2-yl]-5,6-difluoro-8-(methylamino)-9H-pyrido[2,3-b]indol-3-yl]-4-oxo-quinolizine-3-carboxylic acid;
7-[4-(3a,5-dimethyl-3,4,6,6a-tetrahydro-2H-pyrrolo[2,3-c]pyrrol-1-yl)-6-fluoro-8-(methylamino)-9H-pyrido[2,3-b]indol-3-yl]-4-oxo-quinolizine-3-carboxylic acid;
7-[5-cyano-6-fluoro-4-(cis-1-methyl-2,3,3 a,4, 6,6a-hexahydropyrrolo[3,4-b]pyrrol-5-yl)-8-(methylamino)-9H-pyrido[2,3-b]indol-3-yl]-4-oxo-quinolizine-3-carboxylic acid;
7-(5-cyano-4-(*cis*-(4*R*)-4 (dimethylamino)-3,3a,4,5,6,6a-hexahydro-1H-cyclopenta[c]pyrrol-2-yl)-6-fluoro-8-(methylamino)-9H-pyrido[2,3-b]indol-3-yl)-4-oxo-4H-quinolizine-3-carboxylic acid;
7-(4-((1*R*,5*S*,6*S*)-6-(dimethylamino)-3-azabicyclo[3.1.0]hexan-3-yl)-5,6-difluoro-8-(methylamino)-9H-pyrido[2,3-b]indol-3-yl)-4-oxo-4H-quinolizine-3-carboxylic acid;
7-[4-[(3a*S*,6a*S*)-5-methyl-2,3,3a,4,6,6a-hexahydropyrrolo[2,3-c]pyrrol-1-yl]-5-cyano-6-fluoro-8-(methylamino)-9H-pyrido[2,3-b]indol-3-yl]-4-oxo-quinolizine-3-carboxylic acid;
7-[5-cyano-4-[*cis*-3-(dimethylamino)-2,3,3a,4,6,6a-hexahydrofuro[2,3-c]pyrrol-5-yl]-6-fluoro-8-(methylamino)-9H-pyrido[2,3-b]indol-3-yl]-4-oxo-quinolizine-3-carboxylic acid;
7-[4-[*cis*-1-methyl-2,3,3a,4,6,6a-hexahydropyrrolo[3,4-b]pyrrol-5-yl]-5-cyano-6-fluoro-8-(methylamino)-9H-pyrido[2,3-b]indol-3-yl]-4-oxo-quinolizine-3-carboxylic acid;
7-[4-[*cis*-1-methyl-3,3a,4,5,7,7a-hexahydro-2H-pyrrolo[2,3-c]pyridin-6-yl]-5-cyano-6-fluoro-8-(methylamino)-9H-pyrido[2,3-b]indol-3-yl]-4-oxo-quinolizine-3-carboxylic acid;
7-[5-cyano-4-(dimethylamino)-6-fluoro-8-(methylamino)-9H-pyrido[2,3-b]indol-3-yl]-4-oxo-quinolizine-3-carboxylic acid;
7-[6-chloro-4-(*cis*-5-methyl-2,3,3 a,4,6,6a-hexahydropyrrolo[2,3-c]pyrrol-1-yl)-8-(methylamino)-9H-pyrido[2,3-b]indol-3-yl]-4-oxo-quinolizine-3-carboxylic acid;
7-[4-(dimethylamino)-5,6-difluoro-8-(methylamino)-9H-pyrido[2,3-b]indol-3-yl]-9-methyl-4-oxo-quinolizine-3-carboxylic acid;
7-[4-[3-(1-aminocyclopropyl)pyrrolidin-1-yl]-5,6-difluoro-8-(methylamino)-9H-pyrido[2,3-b]indol-3-yl]-4-oxo-quinolizine-3-carboxylic acid;
7-[4-[(4a*S*,7a*R*)-4-methyl-2,3,4a,5,7,7a-hexahydropyrrolo[3,4-b][1,4]oxazin-6-yl]-5,6-difluoro-8-(methylamino)-9H-pyrido[2,3-b]indol-3-yl]-4-oxo-quinolizine-3-carboxylic acid;
7-[4-[(3aS,6aS)-5-methyl-2,3,3a,4,6,6a-hexahydropyrrolo[2,3-c]pyrrol-1-yl]-6-cyano-5-fluoro-8-(methylamino)-9H-pyrido[2,3-b]indol-3-yl]-4-oxo-quinolizine-3-carboxylic acid;
7-(5,6-difluoro-8-(methylamino)-4-(*cis*-5-methyl-2,3,3a,4,6,6a-hexahydropyrrolo[2,3-c]pyrrol-1-yl)-9H-pyrido[2,3-b]indol-3-yl)-9-methyl-4-oxo-4H-quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-9-fluoro-4-oxo-quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-1-[(dimethylamino)methyl]-4-oxo-quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-1-methyl-4-oxo-quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-1-cyclopropyl-4-oxo-quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-4-oxo-1-(trifluoromethyl)quinolizine-3-carboxylic acid;
(*R*)-7-(4-(2-((dimethylamino)methyl)morpholino)-6-fluoro-8-(methylamino)-9H-pyrido[2,3-b]indol-3-yl)-4-oxo-4H-quinolizine-3-carboxylic acid;
(*S*)-7-(4-(2-((dimethylamino)methyl)morpholino)-6-fluoro-8-(methylamino)-9H-pyrido[2,3-b]indol-3-yl)-4-oxo-4H-quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-1-(morpholinomethyl)-4-oxo-quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-4-oxo-1-(pyrrolidin-1-ylmethyl)quinolizine-3-carboxylic acid;
7-[4-[*cis*-5-methyl-2,3,3a,4,6,6a-hexahydropyrrolo[2,3-c]pyrrol-1-yl]-5,6-difluoro-8-(methylamino)-9H-pyrido[2,3-b]indol-3-yl]-1-chloro-4-oxo-quinolizine-3-carboxylic acid;
7-[4-[(3a*R*,6a*R*)-1-methyl-2,3,3a,4,6,6a-hexahydropyrrolo[3,4-b]pyrrol-5-yl]-6-fluoro-8-(methylamino)-9H-pyrido[2,3-b]indol-3-yl]-4-oxo-quinolizine-3-carboxylic acid;
7-[4-[(3a*S*,6a*S*)-1-methyl-2,3,3a,4,6,6a-hexahydropyrrolo[3,4-b]pyrrol-5-yl]-6-fluoro-8-(methylamino)-9H-pyrido[2,3-b]indol-3-yl]-4-oxo-quinolizine-3-carboxylic acid;
7-[4-[(3a*S*,6a*S*)-5-methyl-2,3,3a,4,6,6a-hexahydropyrrolo[2,3-c]pyrrol-1-yl]-6-fluoro-8-(methylamino)-9H-pyrido[2,3-b]indol-3-yl]-4-oxo-quinolizine-3-carboxylic acid;
7-[4-[(3a*R*,6a*R*)-5-methyl-2,3,3a,4,6,6a-hexahydropyrrolo[2,3-c]pyrrol-1-yl]-6-fluoro-8-(methylamino)-9H-pyrido[2,3-b]indol-3-yl]-4-oxo-quinolizine-3-carboxylic acid;
7-[4-[(3a*R*,6a*R*)-1-methyl-2,3,3a,4,6,6a-hexahydropyrrolo[3,4-b]pyrrol-5-yl]-5,6-difluoro-8-(methylamino)-9H-pyrido[2,3-b]indol-3-yl]-4-oxo-quinolizine-3-carboxylic acid;
7-[4-[(3a*S*,6a*S*)-1-methyl-2,3,3a,4,6,6a-hexahydropyrrolo[3,4-b]pyrrol-5-yl]-5,6-difluoro-8-(methylamino)-9H-pyrido[2,3-b]indol-3-yl]-4-oxo-quinolizine-3-carboxylic acid;
7-[4-[(3a*R*,7a*R*)-1-methyl-3,3a,4,5,7,7a-hexahydro-2H-pyrrolo[2,3-c]pyridin-6-yl]-5,6-difluoro-8-(methylamino)-9H-pyrido[2,3-b]indol-3-yl]-4-oxo-quinolizine-3-carboxylic acid;
7-[4-[(3a*S,*7a*S*)-1-methyl-3,3a,4,5,7,7a-hexahydro-2H-pyrrolo[2,3-c]pyridin-6-yl]-5,6-difluoro-8-(methylamino)-9H-pyrido[2,3-b]indol-3-yl]-4-oxo-quinolizine-3-carboxylic acid;
7-[4-[(1*S*,3*R*)-1-amino-5-azaspiro[2.4]heptan-5-yl]-5,6-difluoro-8-(methylamino)-9H-pyrido[2,3-b]indol-3-yl]-4-oxo-quinolizine-3-carboxylic acid;
7-(4-((1*R*,3*R*)-1-amino-5-azaspiro[2.4]heptan-5-yl)-5,6-difluoro-8-(methylamino)-9H-pyrido[2,3-b]indol-3-yl)-4-oxo-4H-quinolizine-3-carboxylic acid;
7-(4-((1*R*,3*S*)-1-amino-5-azaspiro[2.4]heptan-5-yl)-5,6-difluoro-8-(methylamino)-9H-pyrido[2,3-b]indol-3-yl)-4-oxo-4H-quinolizine-3-carboxylic acid;
7-(4-((1*S*,3*S*)-1-amino-5-azaspiro[2.4]heptan-5-yl)-5,6-difluoro-8-(methylamino)-9H-pyrido[2,3-b]indol-3-yl)-4-oxo-4H-quinolizine-3-carboxylic acid;
7-(4-((1*S*,3*R*)-1-amino-5-azaspiro[2.4]heptan-5-yl)-5-chloro-6-fluoro-8-(methylamino)-9H-pyrido[2,3-b]indol-3-yl)-4-oxo-4H-quinolizine-3-carboxylic acid;
7-(4-((1*R*,3*S*)-1-amino-5-azaspiro[2.4]heptan-5-yl)-5-chloro-6-fluoro-8-(methylamino)-9H-pyrido[2,3-b]indol-3-yl)-4-oxo-4H-quinolizine-3-carboxylic acid;
7-(4-((1*R*,3*R*)-1-amino-5-azaspiro[2.4]heptan-5-yl)-5-chloro-6-fluoro-8-(methylamino)-9H-pyrido[2,3-b]indol-3-yl)-4-oxo-4H-quinolizine-3-carboxylic acid; and
7-(4-((1*S*,3*S*)-1-amino-5-azaspiro[2.4]heptan-5-yl)-5-chloro-6-fluoro-8-(methylamino)-9H-pyrido[2,3-b]indol-3-yl)-4-oxo-4H-quinolizine-3-carboxylic acid;
or a pharmaceutically acceptable salt, or enantiomer or diastereomer thereof.

9. A process for the preparation of a compound according to any one of claims 1 to 8 comprising the reaction of compound of formula (Ii), 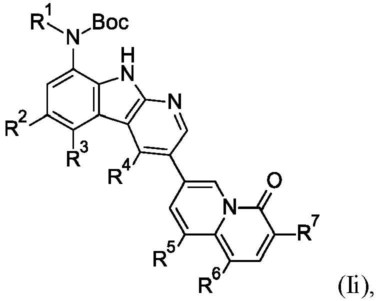 with an acid, wherein R¹ to R⁶ are defined as in any one of claims 1 to 9.

10. A compound according to any one of claims 1 to 8 for use as therapeutically active substance.

11. A pharmaceutical composition comprising a compound in accordance with any one of claims 1 to 8 and a therapeutically inert carrier.

12. A compound according to any one of claims 1 to 8 for the treatment or prophylaxis of bacterial infection.

13. The compound according to claim 12, wherein the bacterial infection is caused by *Streptococcus pneumoniae, Streptococcus pyogenes, Enterococcus faecalis, Enterococcus faecium, Enterobacter spp.* species, *Proteus spp.* species, *Serratia marcescens, Staphylococcus aureus, Coag. Neg. Staphylococci, Haemophilus influenzae, Bacillus anthraces, Mycoplasma pneumoniae, Moraxella catarrhalis, Chlamydophila pneumoniae, Chlamydia trachomatis, Legionella pneumophila, Mycobacterium tuberculosis, Helicobacter pylori, Staphylococcus saprophyticus, Staphylococcus epidermidis, Francisella tularensis, Yersinia pestis, Clostridium difficile, Bacteroides spp.* species *Neisseria gonorrhoeae, Neisseria meningitidis, Burkholderia pseudomallei, Burkholderia mallei, Borrelia burgdorferi, Mycobacterium avium complex, Mycobacterium abscessus, Mycobacterium kansasii, E. coli* or *Mycobacterium ulcerans.*

14. The compound according to claim 12, wherein the bacterial infection is caused by *Acinetobacter baumannii, Klebsiella pneumoniae, Pseudomonas aeruginosa, Staphylococcus aureus* or *E. coli.*

15. A compound according to any one of claims 1 to 8, when manufactured according to a process of claim 9.

## Patentansprüche

1. Verbindung der Formel (I) 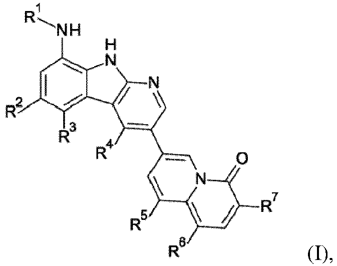 wobei
R¹ C₁₋₆-Alkyl ist;
R² Halogen ist;
R³ H, Halogen oder Cyano ist;
R⁴ (C₁₋₆-Alkyl)₂-amino oder Heterocyclyl ist;
R⁵ H, Halogen oder C₁₋₆-Alkyl ist;
R⁶ H, ((C₁₋₆-Alkyl)₂-amino)-C₁₋₆-alkyl, C₁₋₆-Alkyl, C₃₋₇-Cycloalkyl, Halogen-C₁₋₆-alkyl, Halogen, Morpholinyl-C₁₋₆-alkyl oder Pyrrolidinyl-C₁₋₆-alkyl ist;
R⁷ Carboxy ist;
oder ein pharmazeutisch unbedenkliches Salz oder Enantiomer oder Diastereomer davon.

2. Verbindung der Formel (I) nach Anspruch 1, wobei
R¹ C₁₋₆-Alkyl ist;
R² Halogen ist;
R³ H, Halogen oder Cyano ist;
R⁴ (C₁₋₆-Alkyl)₂-amino;
mit (C₁₋₆-Alkyl)₂-amino substituiertes 2,3,3a,4,6,6a-Hexahydrofuro[2,3-c]pyrrolyl;
mit C₁₋₆-Alkyl substituiertes 2,3,3a,4,6,6a-Hexahydropyrrolo[2,3-c]pyrrolyl;
mit C₁₋₆-Alkyl substituiertes 2,3,3a,4,6,6a-Hexahydropyrrolo[3,4-b]pyrrolyl;
mit C₁₋₆-Alkyl substituiertes 2,3,4a,5,7,7a-Hexahydropyrrolo[3,4-b][1,4]oxazinyl;
mit (C₁₋₆-Alkyl)₂-amino substituiertes 3,3a,4,5,6,6a-Hexahydro-1H-cyclopenta[c]pyrrolyl;
mit C₁₋₆-Alkyl substituiertes 3,3a,4,5,7,7a-Hexahydro-2H-pyrrolo[2,3-c]pyridinyl; zweimal mit C₁₋₆-Alkyl substituiertes 3,4,6,6a-Tetrahydro-2H-pyrrolo[2,3-c]pyrrolyl;
mit (C₁₋₆-Alkyl)₂-amino substituiertes 3-Azabicyclo[3.1.0]hexanyl;
mit Amino substituiertes 5-Azaspiro[2.4]heptanyl; Amino-C₃₋₇-cycloalkylpyrrolidinyl;
Morpholinyl, wobei das Morpholinyl unsubstituiert oder mit ((C₁₋₆-Alkyl)₂-amino)-C₁₋₆-alkyl substituiert ist, ist;
R⁵ H, Halogen oder C₁₋₆-Alkyl ist;
R⁶ H, ((C₁₋₆-Alkyl)₂-amino)-C₁₋₆-alkyl, C₁₋₆-Alkyl, C₃₋₇-Cycloalkyl, Halogen-C₁₋₆-alkyl, Halogen, Morpholinyl-C₁₋₆-alkyl oder Pyrrolidinyl-C₁₋₆-alkyl ist;
R⁷ Carboxy ist;
oder ein pharmazeutisch unbedenkliches Salz oder Enantiomer oder Diastereomer davon.

3. Verbindung nach Anspruch 2, wobei
R¹ Methyl ist;
R² Chlor oder Fluor ist;
R³ H, Chlor, Fluor oder Cyano ist;
R⁴ Dimethylamino; Dimethylamino-2,3,3a,4,6,6a-hexahydrofuro[2,3-c]pyrrolyl; Methyl-2,3,3a,4,6,6a-hexahydropyrrolo[2,3-c]pyrrolyl; Methyl-2,3,3a,4,6,6a-hexahydropyrrolo[3,4-b]pyrrolyl; Methyl-2,3,4a,5,7,7a-hexahydropyrrolo[3,4-b][1,4]oxazinyl; Dimethylamino-3,3a,4,5,6,6a-hexahydro-1H-cyclopenta[c]pyrrolyl; Methyl-3,3a,4,5,7,7a-hexahydro-2H-pyrrolo[2,3-c]pyridinyl; Dimethyl-3,4,6,6a-tetrahydro-2H-pyrrolo[2,3-c]pyrrolyl; Dimethylamino-3-azabicyclo[3.1.0]hexanyl; Amino-5-azaspiro[2.4]heptanyl; Aminocyclopropylpyrrolidinyl; Morpholinyl oder ((Dimethylamino)methyl)morpholinyl ist;
R⁵ H, Fluor oder Methyl ist;
R⁶ H, (Dimethylamino)methyl, Methyl, Cyclopropyl, Trifluormethyl, Chlor, Morpholinylmethyl oder Pyrrolidinylmethyl ist;
R⁷ Carboxy ist;
oder ein pharmazeutisch unbedenkliches Salz oder Enantiomer oder Diastereomer davon.

4. Verbindung nach Anspruch 2, wobei R⁴ mit C₁₋₆-Alkyl substituiertes 2,3,3a,4,6,6a-Hexahydropyrrolo[2,3-c]pyrrolyl; mit C₁₋₆-Alkyl substituiertes 2,3,3a,4,6,6a-Hexahydropyrrolo[3,4-b]pyrrolyl oder mit (C₁₋₆-Alkyl)₂-amino substituiertes 3,3a,4,5,6,6a-Hexahydro-1H-cyclopenta[c]pyrrolyl ist.

5. Verbindung nach Anspruch 4, wobei R⁴ Methyl-2,3,3a,4,6,6a-hexahydropyrrolo[2,3-c]pyrrolyl; Methyl-2,3,3a,4,6,6a-hexahydropyrrolo[3,4-b]pyrrolyl oder Dimethylamino-3,3a,4,5,6,6a-hexahydro-1H-cyclopenta[c]pyrrolyl ist.

6. Verbindung nach Anspruch 2, wobei
R¹ C₁₋₆-Alkyl ist;
R² Halogen ist;
R³ H, Halogen oder Cyano ist;
R⁴ mit C₁₋₆-Alkyl substituiertes 2,3,3a,4,6,6a-Hexahydropyrrolo[2,3-c]pyrrolyl; mit C₁₋₆-Alkyl substituiertes 2,3,3a,4,6,6a-Hexahydropyrrolo[3,4-b]pyrrolyl oder mit (C₁₋₆-Alkyl)₂-amino substituiertes 3,3a,4,5,6,6a-Hexahydro-1H-cyclopenta[c]pyrrolyl ist;
R⁵ H ist;
R⁶ H ist;
R⁷ Carboxy ist;
oder ein pharmazeutisch unbedenkliches Salz oder Enantiomer oder Diastereomer davon.

7. Verbindung nach Anspruch 6, wobei
R¹ Methyl ist;
R² Chlor oder Fluor ist;
R³ H, Chlor, Fluor oder Cyano ist;
R⁴ Methyl-2,3,3a,4,6,6a-hexahydropyrrolo[2,3-c]pyrrolyl; Methyl-2,3,3a,4,6,6a-hexahydropyrrolo[3,4-b]pyrrolyl oder Dimethylamino-3,3a,4,5,6,6a-hexahydro-1H-cyclopenta[c]pyrrolyl ist;
R⁵ H ist;
R⁶ H ist;
R⁷ Carboxy ist;
oder ein pharmazeutisch unbedenkliches Salz oder Enantiomer oder Diastereomer davon.

8. Verbindung nach einem der Ansprüche 1 oder 2, ausgewählt aus
7-(4-(Dimethylamino)-5,6-difluor-8-(methylamino)-9H-pyrido[2,3-b]indol-3-yl)-4-oxo-4H-chinolizin-3-carbonsäure;
7-(6-Fluor-8-(methylamino)-4-morpholino-9H-pyrido[2,3-b]indol-3-yl)-4-oxo-4H-chinolizin-3-carbonsäure;
7-[5,6-Difluor-4-(*cis*-5-methyl-2,3,3a,4,6,6a-hexahydropyrrolo[2,3-c]pyrrol-1-yl)-8-(methylamino)-9H-pyrido[2,3-b]indol-3-yl]-4-oxo-chinolizin-3-carbonsäure;
7-[4-[(3a*S*,6a*S*)-5-Methyl-2,3,3a,4,6,6a-hexahydropyrrolo[2,3-c]pyrrol-1-yl]-5,6-difluor-8-(methylamino)-9H-pyrido[2,3-b]indol-3-yl]-4-oxo-chinolizin-3-carbonsäure;
7-[4-[*cis*-(4*R*)-4-(Dimethylamino)-3,3a,4,5,6,6a-hexahydro-1H-cyclopenta[c]pyrrol-2-yl]-5,6-difluor-8-(methylamino)-9H-pyrido[2,3-b]indol-3-yl]-4-oxo-chinolizin-3-carbonsäure;
7-[4-[*cis*-3-(Dimethylamino)-2,3,3a,4,6,6a-hexahydrofuro[2,3-c]pyrrol-5-yl]-5,6-difluor-8-(methylamino)-9H-pyrido[2,3-b]indol-3-yl]-4-oxo-chinolizin-3-carbonsäure;
7-(5-Chlor-6-fluor-8-(methylamino)-4-((3aS,6aS)-5-methyl-2,3,3a,4,6,6a-hexahydropyrrolo[2,3-c]pyrrol-1-yl)-9H-pyrido[2,3-b]indol-3-yl)-4-oxo-4H-chinolizin-3-carbonsäure;
7-[4-[*cis*-(4R)-4-(Dimethylamino)-3,3a,4,5,6,6a-hexahydro-1H-cyclopenta[c]pyrrol-2-yl]-5-chlor-6-fluor-8-(methylamino)-9H-pyrido[2,3-b]indol-3-yl]-4-oxo-chinolizin-3-carbonsäure;
7-[4-[*cis*-(4S)-4-(Dimethylamino)-3,3a,4,5,6,6a-hexahydro-1H-cyclopenta[c]pyrrol-2-yl]-5,6-difluor-8-(methylamino)-9H-pyrido[2,3-b]indol-3-yl]-4-oxo-chinolizin-3-carbonsäure;
7-[4-(3a,5-Dimethyl-3,4,6,6a-tetrahydro-2H-pyrrolo[2,3-c]pyrrol-1-yl)-6-fluor-8-(methylamino)-9H-pyrido[2,3-b]indol-3-yl]-4-oxo-chinolizin-3-carbonsäure;
7-[5-Cyano-6-fluor-4-(*cis*-1-methyl-2,3,3a,4,6,6a-hexahydropyrrolo[3,4-b]pyrrol-5-yl)-8-(methylamino)-9H-pyrido[2,3-b]indol-3-yl]-4-oxo-chinolizin-3-carbonsäure;
7-(5-Cyano-4-(*cis*-(4R)-4-(dimethylamino)-3,3a,4,5,6,6a-hexahydro-1H-cyclopenta[c]pyrrol-2-yl)-6-fluor-8-(methylamino)-9H-pyrido[2,3-b]indol-3-yl)-4-oxo-4H-chinolizin-3-carbonsäure;
7-(4-((1*R*,5*S*,6*S*)-6-(Dimethylamino)-3-azabicyclo[3.1.0]hexan-3-yl)-5,6-difluor-8-(methylamino)-9H-pyrido[2,3-b]indol-3-yl)-4-oxo-4H-chinolizin-3-carbonsäure;
7-[4-[(3a*S*,6a*S*)-5-Methyl-2,3,3a,4,6,6a-hexahydropyrrolo[2,3-c]pyrrol-1-yl]-5-cyano-6-fluor-8-(methylamino)-9H-pyrido[2,3-b]indol-3-yl]-4-oxo-chinolizin-3-carbonsäure;
7-[5-Cyano-4-[*cis*-3-(dimethylamino)-2,3,3a,4,6,6a-hexahydrofuro[2,3-c]pyrrol-5-yl]-6-fluor-8-(methylamino)-9H-pyrido[2,3-b]indol-3-yl]-4-oxo-chinolizin-3-carbonsäure;
7-[4-[*cis*-1-Methyl-2,3,3a,4,6,6a-hexahydropyrrolo[3,4-b]pyrrol-5-yl]-5-cyano-6-fluor-8-(methylamino)-9H-pyrido[2,3-b]indol-3-yl]-4-oxo-chinolizin-3-carbonsäure;
7-[4-[*cis*-1-Methyl-3,3a,4,5,7,7a-hexahydro-2H-pyrrolo[2,3-c]pyridin-6-yl]-5-cyano-6-fluor-8-(methylamino)-9H-pyrido[2,3-b]indol-3-yl]-4-oxo-chinolizin-3-carbonsäure;
7-[5-Cyano-4-(dimethylamino)-6-fluor-8-(methylamino)-9H-pyrido[2,3-b]indol-3-yl]-4-oxo-chinolizin-3-carbonsäure;
7-[6-Chlor-4-(*cis*-5-methyl-2,3,3a,4,6,6a-hexahydropyrrolo[2,3-c]pyrrol-1-yl)-8-(methylamino)-9H-pyrido[2,3-b]indol-3-yl]-4-oxo-chinolizin-3-carbonsäure;
7-[4-(Dimethylamino)-5,6-difluor-8-(methylamino)-9H-pyrido[2,3-b]indol-3-yl]-9-methyl-4-oxo-chinolizin-3-carbonsäure;
7-[4-[3-(1-Aminocyclopropyl)pyrrolidin-1-yl]-5,6-difluor-8-(methylamino)-9H-pyrido[2,3-b]indol-3-yl]-4-oxo-chinolizin-3-carbonsäure;
7-[4-[(4a*S*,7a*R*)-4-Methyl-2,3,4a,5,7,7a-hexahydropyrrolo[3,4-b][1,4]oxazin-6-yl]-5,6-difluor-8-(methylamino)-9H-pyrido[2,3-b]indol-3-yl]-4-oxo-chinolizin-3-carbonsäure;
7-[4-[(3a*S*,6a*S*)-5-Methyl-2,3,3a,4,6,6a-hexahydropyrrolo[2,3-c]pyrrol-1-yl]-6-cyano-5-fluor-8-(methylamino)-9H-pyrido[2,3-b]indol-3-yl]-4-oxo-chinolizin-3-carbonsäure;
7-(5,6-Difluor-8-(methylamino)-4-(*cis*-5-methyl-2,3,3a,4,6,6a-hexahydropyrrolo[2,3-c]pyrrol-1-yl)-9H-pyrido[2,3-b]indol-3-yl)-9-methyl-4-oxo-4H-chinolizin-3-carbonsäure;
7-[4-[*cis*-5-Methyl-2,3,3a,4,6,6a-hexahydropyrrolo[2,3-c]pyrrol-1-yl]-5,6-difluor-8-(methylamino)-9H-pyrido[2,3-b]indol-3-yl]-9-fluor-4-oxo-chinolizin-3-carbonsäure;
7-[4-[*cis*-5-Methyl-2,3,3a,4,6,6a-hexahydropyrrolo[2,3-c]pyrrol-1-yl]-5,6-difluor-8-(methylamino)-9H-pyrido[2,3-b]indol-3-yl]-1-[(dimethylamino)methyl]-4-oxo-chinolizin-3-carbonsäure;
7-[4-[*cis*-5-Methyl-2,3,3a,4,6,6a-hexahydropyrrolo[2,3-c]pyrrol-1-yl]-5,6-difluor-8-(methylamino)-9H-pyrido[2,3-b]indol-3-yl]-1-methyl-4-oxo-chinolizin-3-carbonsäure;
7-[4-[*cis*-5-Methyl-2,3,3a,4,6,6a-hexahydropyrrolo[2,3-c]pyrrol-1-yl]-5,6-difluor-8-(methylamino)-9H-pyrido[2,3-b]indol-3-yl]-1-cyclopropyl-4-oxo-chinolizin-3-carbonsäure;
7-[4-[*cis*-5-Methyl-2,3,3a,4,6,6a-hexahydropyrrolo[2,3-c]pyrrol-1-yl]-5,6-difluor-8-(methylamino)-9H-pyrido[2,3-b]indol-3-yl]-4-oxo-1-(trifluormethyl)chinolizin-3-carbonsäure;
(*R*)-7-(4-(2-((Dimethylamino)methyl)morpholino)-6-fluor-8-(methylamino)-9H-pyrido[2,3-b]indol-3-yl)-4-oxo-4H-chinolizin-3-carbonsäure;
(*S*)-7-(4-(2-((Dimethylamino)methyl)morpholino)-6-fluor-8-(methylamino)-9H-pyrido[2,3-b]indol-3-yl)-4-oxo-4H-chinolizin-3-carbonsäure;
7-[4-[*cis*-5-Methyl-2,3,3a,4,6,6a-hexahydropyrrolo[2,3-c]pyrrol-1-yl]-5,6-difluor-8-(methylamino)-9H-pyrido[2,3-b]indol-3-yl]-1-(morpholinomethyl)-4-oxo-chinolizin-3-carbonsäure;
7-[4-[*cis*-5-Methyl-2,3,3a,4,6,6a-hexahydropyrrolo[2,3-c]pyrrol-1-yl]-5,6-difluor-8-(methylamino)-9H-pyrido[2,3-b]indol-3-yl]-4-oxo-1-(pyrrolidin-1-ylmethyl)chinolizin-3-carbonsäure;
7-[4-[*cis*-5-Methyl-2,3,3a,4,6,6a-hexahydropyrrolo[2,3-c]pyrrol-1-yl]-5,6-difluor-8-(methylamino)-9H-pyrido[2,3-b]indol-3-yl]-1-chlor-4-oxo-chinolizin-3-carbonsäure;
7-[4-[(3a*R*,6a*R*)-1-Methyl-2,3,3a,4,6,6a-hexahydropyrrolo[3,4-b]pyrrol-5-yl]-6-fluor-8-(methylamino)-9H-pyrido[2,3-b]indol-3-yl]-4-oxo-chinolizin-3-carbonsäure;
7-[4-[(3a*S*,6a*S*)-1-Methyl-2,3,3a,4,6,6a-hexahydropyrrolo[3,4-b]pyrrol-5-yl]-6-fluor-8-(methylamino)-9H-pyrido[2,3-b]indol-3-yl]-4-oxo-chinolizin-3-carbonsäure;
7-[4-[(3a*S*,6a*S*)-5-Methyl-2,3,3a,4,6,6a-hexahydropyrrolo[2,3-c]pyrrol-1-yl]-6-fluor-8-(methylamino)-9H-pyrido[2,3-b]indol-3-yl]-4-oxo-chinolizin-3-carbonsäure;
7-[4-[(3a*R*,6a*R*)-5-Methyl-2,3,3a,4,6,6a-hexahydropyrrolo[2,3-c]pyrrol-1-yl]-6-fluor-8-(methylamino)-9H-pyrido[2,3-b]indol-3-yl]-4-oxo-chinolizin-3-carbonsäure;
7-[4-[(3a*R*,6a*R*)-1-Methyl-2,3,3a,4,6,6a-hexahydropyrrolo[3,4-b]pyrrol-5-yl]-5,6-difluor-8-(methylamino)-9H-pyrido[2,3-b]indol-3-yl]-4-oxo-chinolizin-3-carbonsäure;
7-[4-[(3a*S*,6a*S*)-1-Methyl-2,3,3a,4,6,6a-hexahydropyrrolo[3,4-b]pyrrol-5-yl]-5,6-difluor-8-(methylamino)-9H-pyrido[2,3-b]indol-3-yl]-4-oxo-chinolizin-3-carbonsäure;
7-[4-[(3a*R*,7a*R*)-1-Methyl-3,3a,4,5,7,7a-hexahydro-2H-pyrrolo[2,3-c]pyridin-6-yl]-5,6-difluor-8-(methylamino)-9H-pyrido[2,3-b]indol-3-yl]-4-oxo-chinolizin-3-carbonsäure;
7-[4-[(3aS,7aS)-1-Methyl-3,3a,4,5,7,7a-hexahydro-2H-pyrrolo[2,3-c]pyridin-6-yl]-5,6-difluor-8-(methylamino)-9H-pyrido[2,3-b]indol-3-yl]-4-oxo-chinolizin-3-carbonsäure;
7-[4-[(1*S*,3*R*)-1-Amino-5-azaspiro[2.4]heptan-5-yl]-5,6-difluor-8-(methylamino)-9H-pyrido[2,3-b]indol-3-yl]-4-oxo-chinolizin-3-carbonsäure;
7-(4-((1*R*,3*R*)-1-Amino-5-azaspiro[2.4]heptan-5-yl)-5,6-difluor-8-(methylamino)-9H-pyrido[2,3-b]indol-3-yl)-4-oxo-4H-chinolizin-3-carbonsäure;
7-(4-((1*R*,3*S*)-1-Amino-5-azaspiro[2.4]heptan-5-yl)-5,6-difluor-8-(methylamino)-9H-pyrido[2,3-b]indol-3-yl)-4-oxo-4H-chinolizin-3-carbonsäure;
7-(4-((1*S*,3*S*)-1-Amino-5-azaspiro[2.4]heptan-5-yl)-5,6-difluor-8-(methylamino)-9H-pyrido[2,3-b]indol-3-yl)-4-oxo-4H-chinolizin-3-carbonsäure;
7-(4-((1*S*,3*R*)-1-Amino-5-azaspiro[2.4]heptan-5-yl)-5-chlor-6-fluor-8-(methylamino)-9H-pyrido[2,3-b]indol-3-yl)-4-oxo-4H-chinolizin-3-carbonsäure;
7-(4-((1*R*,3*S*)-1-Amino-5-azaspiro[2.4]heptan-5-yl)-5-chlor-6-fluor-8-(methylamino)-9H-pyrido[2,3-b]indol-3-yl)-4-oxo-4H-chinolizin-3-carbonsäure;
7-(4-((1*R*,3*R*)-1-Amino-5-azaspiro[2.4]heptan-5-yl)-5-chlor-6-fluor-8-(methylamino)-9H-pyrido[2,3-b]indol-3-yl)-4-oxo-4H-chinolizin-3-carbonsäure und
7-(4-((1*S*,3*S*)-1-Amino-5-azaspiro[2.4]heptan-5-yl)-5-chlor-6-fluor-8-(methylamino)-9H-pyrido[2,3-b]indol-3-yl)-4-oxo-4H-chinolizin-3-carbonsäure;
oder ein pharmazeutisch unbedenkliches Salz oder Enantiomer oder Diastereomer davon.

9. Verfahren zur Herstellung einer Verbindung nach einem der Ansprüche 1 bis 8, umfassend die Reaktion einer Verbindung der Formel (Ii), 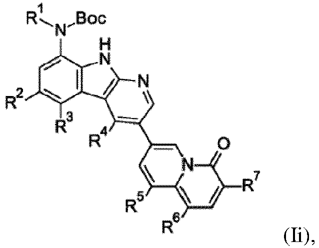 mit einer Säure, wobei R¹ bis R⁶ wie in einem der Ansprüche 1 bis 9 definiert sind.

10. Verbindung nach einem der Ansprüche 1 bis 8 zur Verwendung als therapeutisch wirksame Substanz.

11. Pharmazeutische Zusammensetzung, umfassend eine Verbindung nach einem der Ansprüche 1 bis 8 und einen therapeutisch inerten Träger.

12. Verbindung nach einem der Ansprüche 1 bis 8 zur Behandlung oder Prophylaxe einer bakteriellen Infektion.

13. Verbindung nach Anspruch 12, wobei die bakterielle Infektion von *Streptococcus pneumoniae, Streptococcus pyogenes, Enterococcus faecalis, Enterococcus faecium, Enterobacter spp*.-Spezies, *Proteus spp*. -Spezies, *Serratia marcescens, Staphylococcus aureus, Coag. Neg. Staphylococci, Haemophilus influenzae, Bacillus anthraces, Mycoplasma pneumoniae, Moraxella catarrhalis, Chlamydophila pneumoniae, Chlamydia trachomatis, Legionella pneumophila, Mycobacterium tuberculosis, Helicobacter pylori, Staphylococcus saprophyticus, Staphylococcus epidermidis, Francisella tularensis, Yersinia pestis, Clostridium difficile, Bacteroides* spp.-Spezies, *Neisseria gonorrhoeae, Neisseria meningitidis, Burkholderia pseudomallei, Burkholderia mallei, Borrelia burgdorferi, Mycobacterium avium-Komplex, Mycobacterium abscessus, Mycobacterium kansasii, E. coli* oder *Mycobacterium ulcerans* verursacht wird.

14. Verbindung nach Anspruch 12, wobei die bakterielle Infektion von *Acinetobacter baumannii, Klebsiella pneumoniae, Pseudomonas aeruginosa, Staphylococcus aureus* oder *E*. *coli* verursacht wird.

15. Verbindung nach einem der Ansprüche 1 bis 8, hergestellt gemäß einem Verfahren von Anspruch 9.

## Revendications

1. Composé de formule (I), 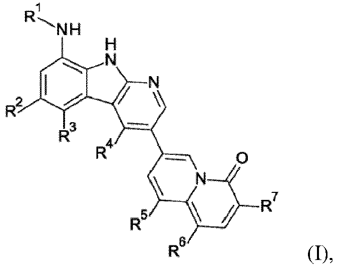 dans lequel
R¹ représente un alkyle en C₁₋₆ ;
R² représente un halogène ;
R³ représente H, un halogène ou un cyano ;
R⁴ représente un (alkyle en C₁₋₆)₂amino ou un hétérocyclyle ;
R⁵ représente H, un halogène ou un alkyle en C₁₋₆ ;
R⁶ représente H, un ((alkyle en C₁₋₆)₂amino)alkyle en C₁₋₆, un alkyle en C₁₋₆, un cycloalkyle en C₃₋₇, un halogénoalkyle en C₁₋₆, un halogène, un morpholinylalkyle en C₁₋₆ ou un pyrrolidinylalkyle en C₁₋₆ ;
R⁷ représente un carboxy ;
ou un sel pharmaceutiquement acceptable, ou un énantiomère ou un diastéréoisomère de celui-ci.

2. Composé de formule (I) selon la revendication 1, dans lequel
R¹ représente un alkyle en C₁₋₆ ;
R² représente un halogène ;
R³ représente H, un halogène ou un cyano ;
R⁴ représente un (alkyle en C₁₋₆)₂amino ;
un 2,3,3a,4,6,6a-hexahydrofuro[2,3-c]pyrrolyle substitué par un (alkyle en C₁₋₆)₂amino ;
un 2,3,3a,4,6,6a-hexahydropyrrolo[2,3-c]pyrrolyle substitué par un alkyle en C₁₋₆ ;
un 2,3,3a,4,6,6a-hexahydropyrrolo[3,4-b]pyrrolyle substitué par un alkyle en C₁₋₆ ;
un 2,3,4a,5,7,7a-hexahydropyrrolo[3,4-b][1,4]oxazinyle substitué par un alkyle en C₁₋₆ ;
un 3,3a,4,5,6,6a-hexahydro-1H-cyclopenta[c]pyrrolyle substitué par un (alkyle en C₁₋₆)₂amino ;
un 3,3a,4,5,7,7a-hexahydro-2H-pyrrolo[2,3-c]pyridinyle substitué par un alkyle en C₁₋₆ ;
un 3,4,6,6a-tétrahydro-2H-pyrrolo[2,3-c]pyrrolyle substitué deux fois par un alkyle en C₁₋₆ ;
un 3-azabicyclo[3.1.0]hexanyle substitué par un (alkyle en C₁₋₆)₂amino ;
un 5-azaspiro[2.4]heptanyle substitué par un amino ;
un aminocycloalkyle en C₃₋₇-pyrrolidinyle ;
un morpholinyle, ledit morpholinyle étant non substitué ou substitué par un ((alkyle en C₁₋₆)₂amino)alkyle en C₁₋₆ ;
R⁵ représente H, un halogène ou un alkyle en C₁₋₆ ;
R⁶ représente H, un ((alkyle en C₁₋₆)₂amino)alkyle en C₁₋₆, un alkyle en C₁₋₆, un cycloalkyle en C₃₋₇, un halogénoalkyle en C₁₋₆, un halogène, un morpholinylalkyle en C₁₋₆ ou un pyrrolidinylalkyle en C₁₋₆ ;
R⁷ représente un carboxy ;
ou un sel pharmaceutiquement acceptable, ou un énantiomère ou un diastéréoisomère de celui-ci.

3. Composé selon la revendication 2, dans lequel
R¹ représente un méthyle ;
R² représente un chlore ou un fluor ;
R³ représente H, un chlore, un fluor ou un cyano ;
R⁴ représente un diméthylamino ; un diméthylamino-2,3,3a,4,6,6a-hexahydrofuro[2,3-c]pyrrolyle ; un méthyl-2,3,3a,4,6,6a-hexahydropyrrolo[2,3-c]pyrrolyle ; un méthyl-2,3,3a,4,6,6a-hexahydropyrrolo[3,4-b]pyrrolyle ; un méthyl-2,3,4a,5,7,7a-hexahydropyrrolo[3,4-b][1,4]oxazinyle ; un diméthylamino-3,3a,4,5,6,6a-hexahydro-1H-cyclopenta[c]pyrrolyle ; un méthyl-3,3a,4,5,7,7a-hexahydro-2H-pyrrolo[2,3-c]pyridinyle ; un diméthyl-3,4,6,6a-tétrahydro-2H-pyrrolo[2,3-c]pyrrolyle ; un diméthylamino-3-azabicyclo[3.1.0]hexanyle ; un amino-5-azaspiro[2.4]heptanyle ; un aminocyclopropylpyrrolidinyle ; un morpholinyle ou un ((diméthylamino)méthyl)morpholinyle ;
R⁵ représente H, un fluor ou un méthyle ;
R⁶ représente H, un (diméthylamino)méthyle, un méthyle, un cyclopropyle, un trifluorométhyle, un chlore, un morpholinylméthyle ou un pyrrolidinylméthyle ;
R⁷ représente un carboxy ;
ou un sel pharmaceutiquement acceptable, ou un énantiomère ou un diastéréoisomère de celui-ci.

4. Composé selon la revendication 2, dans lequel R⁴ représente un 2,3,3a,4,6,6a-hexahydropyrrolo[2,3-c]pyrrolyle substitué par un alkyle en C₁₋₆ ; un 2,3,3a,4,6,6a-hexahydropyrrolo[3,4-b]pyrrolyle substitué par un alkyle en C₁₋₆ ; ou un 3,3a,4,5,6,6a-hexahydro-1H-cyclopenta[c]pyrrolyle substitué par un (alkyle en C₁₋₆)₂amino.

5. Composé selon la revendication 4, dans lequel R⁴ représente un méthyl-2,3,3a,4,6,6a-hexahydropyrrolo[2,3-c]pyrrolyle ; un méthyl-2,3,3a,4,6,6a-hexahydropyrrolo[3,4-b]pyrrolyle ; ou un diméthylamino-3,3a,4,5,6,6a-hexahydro-1H-cyclopenta[c]pyrrolyle.

6. Composé selon la revendication 2, dans lequel
R¹ représente un alkyle en C₁₋₆ ;
R² représente un halogène ;
R³ représente H, un halogène ou un cyano ;
R⁴ représente un 2,3,3a,4,6,6a-hexahydropyrrolo[2,3-c]pyrrolyle substitué par un alkyle en C₁₋₆ ; un 2,3,3a,4,6,6a-hexahydropyrrolo[3,4-b]pyrrolyle substitué par un alkyle en C₁₋₆ ; ou un 3,3a,4,5,6,6a-hexahydro-1H-cyclopenta[c]pyrrolyle substitué par un (alkyle en C₁₋₆)₂amino ;
R⁵ représente H ;
R⁶ représente H ;
R⁷ représente un carboxy ;
ou un sel pharmaceutiquement acceptable, ou un énantiomère ou un diastéréoisomère de celui-ci.

7. Composé selon la revendication 6, dans lequel
R¹ représente un méthyle ;
R² représente un chlore ou un fluor ;
R³ représente H, un chlore, un fluor ou un cyano ;
R⁴ représente un méthyl-2,3,3a,4,6,6a-hexahydropyrrolo[2,3-c]pyrrolyle ; un méthyl-2,3,3a,4,6,6a-hexahydropyrrolo[3,4-b]pyrrolyle ; ou un diméthylamino-3,3a,4,5,6,6a-hexahydro-1H-cyclopenta[c]pyrrolyle ;
R⁵ représente H ;
R⁶ représente H ;
R⁷ représente un carboxy ;
ou un sel pharmaceutiquement acceptable, ou un énantiomère ou un diastéréoisomère de celui-ci.

8. Composé selon l'une quelconque des revendications 1 ou 2, choisi parmi
l'acide 7-(4-(diméthylamino)-5,6-difluoro-8-(méthylamino)-9H-pyrido[2,3-b]indol-3-yl)-4-oxo-4H-quinolizine-3-carboxylique ;
l'acide 7-(6-fluoro-8-(méthylamino)-4-morpholino-9H-pyrido[2,3-b]indol-3-yl)-4-oxo-4H-quinolizine-3-carboxylique ;
l'acide 7-[5,6-difluoro-4-(cis-5-méthyl-2,3,3a,4,6,6a-hexahydropyrrolo[2,3-c]pyrrol-1-yl)-8-(méthylamino)-9H-pyrido[2,3-b]indol-3-yl]-4-oxo-quinolizine-3-carboxylique ;
l'acide 7-[4-[(3aS,6aS)-5-méthyl-2,3,3a,4,6,6a-hexahydropyrrolo[2,3-c]pyrrol-1-yl]-5,6-difluoro-8-(méthylamino)-9H-pyrido[2,3-b]indol-3-yl]-4-oxo-quinolizine-3-carboxylique ;
l'acide 7-[4-[*cis*-(4*R*)-4-(diméthylamino)-3,3a,4,5,6,6a-hexahydro-1H-cyclopenta[c]pyrrol-2-yl]-5,6-difluoro-8-(méthylamino)-9H-pyrido[2,3-b]indol-3-yl]-4-oxo-quinolizine-3-carboxylique ;
l'acide 7-[4-[*cis*-3-(diméthylamino)-2,3,3a,4,6,6a-hexahydrofuro[2,3-c]pyrrol-5-yl]-5,6-difluoro-8-(méthylamino)-9H-pyrido[2,3-b]indol-3-yl]-4-oxo-quinolizine-3-carboxylique ;
l'acide 7-(5-chloro-6-fluoro-8-(méthylamino)-4-((3a*S*,6a*S*)-5-méthyl-2,3,3a,4,6,6a-hexahydropyrrolo[2,3-c]pyrrol-1-yl)-9H-pyrido[2,3-b]indol-3-yl)-4-oxo-4H-quinolizine-3-carboxylique ;
l'acide 7-[4-[*cis*-(4R)-4-(diméthylamino)-3,3a,4,5,6,6a-hexahydro-1H-cyclopenta[c]pyrrol-2-yl]-5-chloro-6-fluoro-8-(méthylamino)-9H-pyrido[2,3-b]indol-3-yl] -4-oxo-quinolizine-3 -carboxylique ;
l'acide 7-[4-[*cis*-(4S)-4-(diméthylamino)-3,3a,4,5,6,6a-hexahydro-1H-cyclopenta[c]pyrrol-2-yl]-5,6-difluoro-8-(méthylamino)-9H-pyrido[2,3-b]indol-3-yl]-4-oxo-quinolizine-3-carboxylique ;
l'acide 7-[4-(3a,5-diméthyl-3,4,6,6a-tétrahydro-2H-pyrrolo[2,3-c]pyrrol-1-yl)-6-fluoro-8-(méthylamino)-9H-pyrido[2,3-b]indol-3-yl]-4-oxo-quinolizine-3-carboxylique ;
l'acide 7-[5-cyano-6-fluoro-4-(*cis*-1-méthyl-2,3,3a,4,6,6a-hexahydropyrrolo[3,4-b]pyrrol-5-yl)-8-(méthylamino)-9H-pyrido[2,3-b]indol-3-yl]-4-oxo-quinolizine-3-carboxylique ;
l'acide 7-(5-cyano-4-(*cis*-(4*R*)-4(diméthylamino)-3,3a,4,5,6,6a-hexahydro-1H-cyclopenta[c]pyrrol-2-yl)-6-fluoro-8-(méthylamino)-9H-pyrido[2,3-b]indol-3-yl)-4-oxo-4H-quinolizine-3-carboxylique ;
l'acide 7-(4-((1*R*,5*S*,6*S*)-6-(diméthylamino)-3-azabicyclo[3.1.0]hexan-3-yl)-5,6-difluoro-8-(méthylamino)-9H-pyrido[2,3-b]indol-3-yl)-4-oxo-4H-quinolizine-3-carboxylique ;
l'acide 7-[4-[(3a*S*,6a*S*)-5-méthyl-2,3,3a,4,6,6a-hexahydropyrrolo[2,3-c]pyrrol-1-yl]-5-cyano-6-fluoro-8-(méthylamino)-9H-pyrido[2,3-b]indol-3-yl]-4-oxo-quinolizine-3-carboxylique ;
l'acide 7-[5-cyano-4-[*cis*-3-(diméthylamino)-2,3,3a,4,6,6a-hexahydrofuro[2,3-c]pyrrol-5-yl]-6-fluoro-8-(méthylamino)-9H-pyrido[2,3-b]indol-3-yl]-4-oxo-quinolizine-3-carboxylique ;
l'acide 7-[4-[*cis*-1-méthyl-2,3,3a,4,6,6a-hexahydropyrrolo[3,4-b]pyrrol-5-yl]-5-cyano-6-fluoro-8-(méthylamino)-9H-pyrido[2,3-b]indol-3-yl]-4-oxo-quinolizine-3-carboxylique ;
l'acide 7-[4-[*cis*-1-méthyl-3,3a,4,5,7,7a-hexahydro-2H-pyrrolo[2,3-c]pyridin-6-yl]-5-cyano-6-fluoro-8-(méthylamino)-9H-pyrido[2,3-b]indol-3-yl]-4-oxo-quinolizine-3-carboxylique ;
l'acide 7-[5-cyano-4-(diméthylamino)-6-fluoro-8-(méthylamino)-9H-pyrido[2,3-b]indol-3-yl]-4-oxo-quinolizine-3-carboxylique ;
l'acide 7-[6-chloro-4-(*cis*-5-méthyl-2,3,3a,4,6,6a-hexahydropyrrolo[2,3-c]pyrrol-1-yl)-8-(méthylamino)-9H-pyrido[2,3-b]indol-3-yl]-4-oxo-quinolizine-3-carboxylique ;
l'acide 7-[4-(diméthylamino)-5,6-difluoro-8-(méthylamino)-9H-pyrido[2,3-b]indol-3-yl]-9-méthyl-4-oxo-quinolizine-3-carboxylique ;
l'acide 7-[4-[3-(1-aminocyclopropyl)pyrrolidin-1-yl]-5,6-difluoro-8-(méthylamino)-9H-pyrido[2,3-b]indol-3-yl]-4-oxo-quinolizine-3-carboxylique ;
l'acide 7-[4-[(4a*S*,7a*R*)-4-méthyl-2,3,4a,5,7,7a-hexahydropyrrolo[3,4-b][1,4]oxazin-6-yl]-5,6-difluoro-8-(méthylamino)-9H-pyrido[2,3-b]indol-3-yl]-4-oxo-quinolizine-3-carboxylique ;
l'acide 7-[4-[(3a*S*,6a*S*)-5-méthyl-2,3,3a,4,6,6a-hexahydropyrrolo[2,3-c]pyrrol-1-yl]-6-cyano-5-fluoro-8-(méthylamino)-9H-pyrido[2,3-b]indol-3-yl]-4-oxo-quinolizine-3-carboxylique ;
l'acide 7-(5,6-difluoro-8-(méthylamino)-4-(*cis*-5-méthyl-2,3,3a,4,6,6a-hexahydropyrrolo[2,3-c]pyrrol-1-yl)-9H-pyrido[2,3-b]indol-3-yl)-9-méthyl-4-oxo-4H-quinolizine-3-carboxylique ;
l'acide 7-[4-[*cis*-5-méthyl-2,3,3a,4,6,6a-hexahydropyrrolo[2,3-c]pyrrol-1-yl]-5,6-difluoro-8-(méthylamino)-9H-pyrido[2,3-b]indol-3-yl]-9-fluoro-4-oxo-quinolizine-3-carboxylique ;
l'acide 7-[4-[*cis*-5-méthyl-2,3,3a,4,6,6a-hexahydropyrrolo[2,3-c]pyrrol-1-yl]-5,6-difluoro-8-(méthylamino)-9H-pyrido[2,3-b]indol-3-yl]-1-[(diméthylamino)méthyl]-4-oxo-quinolizine-3-carboxylique ;
l'acide 7-[4-[*cis*-5-méthyl-2,3,3a,4,6,6a-hexahydropyrrolo[2,3-c]pyrrol-1-yl]-5,6-difluoro-8-(méthylamino)-9H-pyrido[2,3-b]indol-3-yl]-1-méthyl-4-oxo-quinolizine-3-carboxylique ;
l'acide 7-[4-[*cis*-5-méthyl-2,3,3a,4,6,6a-hexahydropyrrolo[2,3-c]pyrrol-1-yl]-5,6-difluoro-8-(méthylamino)-9H-pyrido[2,3-b]indol-3-yl]-1-cyclopropyl-4-oxo-quinolizine-3-carboxylique ;
l'acide 7-[4-[*cis*-5-méthyl-2,3,3a,4,6,6a-hexahydropyrrolo[2,3-c]pyrrol-1-yl]-5,6-difluoro-8-(méthylamino)-9H-pyrido[2,3-b]indol-3-yl]-4-oxo-1-(trifluorométhyl)quinolizine-3-carboxylique ;
l'acide (*R*)-7-(4-(2-((diméthylamino)méthyl)morpholino)-6-fluoro-8-(méthylamino)-9H-pyrido[2,3-b]indol-3-yl)-4-oxo-4H-quinolizine-3-carboxylique ;
l'acide (*S*)-7-(4-(2-((diméthylamino)méthyl)morpholino)-6-fluoro-8-(méthylamino)-9H-pyrido[2,3-b]indol-3-yl)-4-oxo-4H-quinolizine-3-carboxylique ;
l'acide 7-[4-[*cis*-5-méthyl-2,3,3a,4,6,6a-hexahydropyrrolo[2,3-c]pyrrol-1-yl]-5,6-difluoro-8-(méthylamino)-9H-pyrido[2,3-b]indol-3-yl]-1-(morpholinométhyl)-4-oxo-quinolizine-3-carboxylique ;
l'acide 7-[4-[*cis*-5-méthyl-2,3,3a,4,6,6a-hexahydropyrrolo[2,3-c]pyrrol-1-yl]-5,6-difluoro-8-(méthylamino)-9H-pyrido[2,3-b]indol-3-yl]-4-oxo-1-(pyrrolidin-1-ylméthyl)quinolizine-3-carboxylique ;
l'acide 7-[4-[*cis*-5-méthyl-2,3,3a,4,6,6a-hexahydropyrrolo[2,3-c]pyrrol-1-yl]-5,6-difluoro-8-(méthylamino)-9H-pyrido[2,3-b]indol-3-yl]-1-chloro-4-oxo-quinolizine-3-carboxylique ;
l'acide 7-[4-[(3a*R*,6a*R*)-1-méthyl-2,3,3a,4,6,6a-hexahydropyrrolo[3,4-b]pyrrol-5-yl]-6-fluoro-8-(méthylamino)-9H-pyrido[2,3-b]indol-3-yl]-4-oxo-quinolizine-3-carboxylique ;
l'acide 7-[4-[(3a*S*,6a*S*)-1-méthyl-2,3,3a,4,6,6a-hexahydropyrrolo[3,4-b]pyrrol-5-yl]-6-fluoro-8-(méthylamino)-9H-pyrido[2,3-b]indol-3-yl]-4-oxo-quinolizine-3-carboxylique ;
l'acide 7-[4-[(3a*S*,6a*S*)-5-méthyl-2,3,3a,4,6,6a-hexahydropyrrolo[2,3-c]pyrrol-1-yl]-6-fluoro-8-(méthylamino)-9H-pyrido[2,3-b]indol-3-yl]-4-oxo-quinolizine-3-carboxylique ;
l'acide 7-[4-[(3a*R*,6a*R*)-5-méthyl-2,3,3a,4,6,6a-hexahydropyrrolo[2,3-c]pyrrol-1-yl]-6-fluoro-8-(méthylamino)-9H-pyrido[2,3-b]indol-3-yl]-4-oxo-quinolizine-3-carboxylique ;
l'acide 7-[4-[(3a*R*,6a*R*)-1-méthyl-2,3,3a,4,6,6a-hexahydropyrrolo[3,4-b]pyrrol-5-yl]-5,6-difluoro-8-(méthylamino)-9H-pyrido[2,3-b]indol-3-yl]-4-oxo-quinolizine-3-carboxylique ;
l'acide 7-[4-[(3a*S*,6a*S*)-1-méthyl-2,3,3a,4,6,6a-hexahydropyrrolo[3,4-b]pyrrol-5-yl]-5,6-difluoro-8-(méthylamino)-9H-pyrido[2,3-b]indol-3-yl]-4-oxo-quinolizine-3-carboxylique ;
l'acide 7-[4-[(3a*R*,7a*R*)-1-méthyl-3,3a,4,5,7,7a-hexahydro-2H-pyrrolo[2,3-c]pyridin-6-yl]-5,6-difluoro-8-(méthylamino)-9H-pyrido[2,3-b]indol-3-yl]-4-oxo-quinolizine-3-carboxylique ;
l'acide 7-[4-[(3a*S*,7a*S*)-1-méthyl-3,3a,4,5,7,7a-hexahydro-2H-pyrrolo[2,3-c]pyridin-6-yl]-5,6-difluoro-8-(méthylamino)-9H-pyrido[2,3-b]indol-3-yl]-4-oxo-quinolizine-3-carboxylique ;
l'acide 7-[4-[(1*S*,3*R*)-1-amino-5-azaspiro[2.4]heptan-5-yl]-5,6-difluoro-8-(méthylamino)-9H-pyrido[2,3-b]indol-3-yl]-4-oxo-quinolizine-3-carboxylique ;
l'acide 7-(4-((1*R*,3*R*)-1-amino-5-azaspiro[2.4]heptan-5-yl)-5,6-difluoro-8-(méthylamino)-9H-pyrido[2,3-b]indol-3-yl)-4-oxo-4H-quinolizine-3-carboxylique ;
l'acide 7-(4-((1*R*,3*S*)-1-amino-5-azaspiro[2.4]heptan-5-yl)-5,6-difluoro-8-(méthylamino)-9H-pyrido[2,3-b]indol-3-yl)-4-oxo-4H-quinolizine-3-carboxylique ;
l'acide 7-(4-((1*S*,3*S*)-1-amino-5-azaspiro[2.4]heptan-5-yl)-5,6-difluoro-8-(méthylamino)-9H-pyrido[2,3-b]indol-3-yl)-4-oxo-4H-quinolizine-3-carboxylique ;
l'acide 7-(4-((1*S*,3*R*)-1-amino-5-azaspiro[2.4]heptan-5-yl)-5-chloro-6-fluoro-8-(méthylamino)-9H-pyrido[2,3-b]indol-3-yl)-4-oxo-4H-quinolizine-3-carboxylique ;
l'acide 7-(4-((1*R*,3*S*)-1-amino-5-azaspiro[2.4]heptan-5-yl)-5-chloro-6-fluoro-8-(méthylamino)-9H-pyrido[2,3-b]indol-3-yl)-4-oxo-4H-quinolizine-3-carboxylique ;
l'acide 7-(4-((1*R*,3*R*)-1-amino-5-azaspiro[2.4]heptan-5-yl)-5-chloro-6-fluoro-8-(méthylamino)-9H-pyrido[2,3-b]indol-3-yl)-4-oxo-4H-quinolizine-3-carboxylique ; et
l'acide 7-(4-((1*S*,3*S*)-1-amino-5-azaspiro[2.4]heptan-5-yl)-5-chloro-6-fluoro-8-(méthylamino)-9H-pyrido[2,3-b]indol-3-yl)-4-oxo-4H-quinolizine-3-carboxylique ;
ou un sel pharmaceutiquement acceptable, ou un énantiomère ou un diastéréoisomère de ceux-ci.

9. Procédé de préparation d'un composé selon l'une quelconque des revendications 1 à 8 comprenant la réaction d'un composé de formule (Ii), 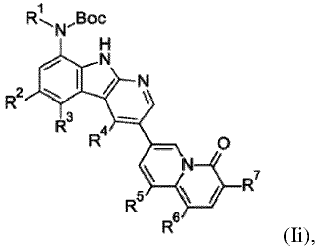 avec un acide, dans lequel R¹ à R⁶ sont tels que définis dans l'une quelconque des revendications 1 à 9.

10. Composé selon l'une quelconque des revendications 1 à 8 pour une utilisation comme substance thérapeutiquement active.

11. Composition pharmaceutique comprenant un composé selon l'une quelconque des revendications 1 à 8 et un véhicule thérapeutiquement inerte.

12. Composé selon l'une quelconque des revendications 1 à 8 pour le traitement ou la prophylaxie d'une infection bactérienne.

13. Composé selon la revendication 12, dans lequel l'infection bactérienne est causée par *Streptococcus pneumoniae, Streptococcus pyogenes, Enterococcus faecalis, Enterococcus faecium,* les espèces *Enterobacter spp.,* les espèces *Proteus spp., Serratia marcescens, Staphylococcus aureus, Staphylococci à coagulase négative, Haemophilus influenzae, Bacillus anthraces, Mycoplasma pneumoniae, Moraxella catarrhalis, Chlamydophila pneumoniae, Chlamydia trachomatis, Legionella pneumophila, Mycobacterium tuberculosis, Helicobacter pylori, Staphylococcus saprophyticus, Staphylococcus epidermidis, Francisella tularensis, Yersinia pestis, Clostridium difficile,* les espèces *Bacteroides spp., Neisseria gonorrhoeae, Neisseria meningitidis, Burkholderia pseudomallei, Burkholderia mallei, Borrelia burgdorferi, Mycobacterium avium complex, Mycobacterium abscessus, Mycobacterium kansasii, E. coli* ou *Mycobacterium ulcerans.*

14. Composé selon la revendication 12, dans lequel l'infection bactérienne est causée par *Acinetobacter baumannii, Klebsiella pneumoniae, Pseudomonas aeruginosa, Staphylococcus aureus* ou *E. coli.*

15. Composé selon l'une quelconque des revendications 1 à 8, lorsqu'il est fabriqué selon un procédé selon la revendication 9.
